# EUROPEAN PATENT SPECIFICATION

(11) **EP 3 744 810 B1**
(45) Date of publication and mention of the grant of the patent: **01.06.2022**
(21) Application number: 17935203.4
(22) Date of filing: 21.12.2017
(51) Int. Cl.: C09K 11/06, C07D 403/08, C07D 413/08, C07D 417/08, G01N 21/64, C09B 57/00

(54) **FLUORESCENT DYE, PREPARATION METHOD THEREFOR AND USE THEREOF**
FLUORESZENTER FARBSTOFF, HERSTELLUNGSVERFAHREN DAFÜR UND VERWENDUNG DAVON
COLORANT FLUORESCENT, PROCÉDÉ DE PRÉPARATION ASSOCIÉ ET UTILISATION CORRESPONDANTE

(43) Date of publication of application: 02.12.2020
(73) Proprietor: Shenzhen University, Shenzhen, Guangdong 518060 (CN)
(72) Inventor: YANG, Zhigang, Shenzhen, Guangdong 518060 (CN); QU, Junle, Shenzhen, Guangdong 518060 (CN)
(74) Representative: Puchberger & Partner Patentanwälte
(86) International application number: PCT/CN2017/117597
(87) International publication number: WO 2019/119331

(56) References cited:
- CN-A- 104 774 606
- DE-A1- 19 815 659
- US-A1- 2003 235 846
- US-A1- 2005 202 565
- US-A1- 2012 276 642
- VOLKOVA, K. D . et al.: "Aza-substituted squaraines for the fluorescent detection of albumins", Dyes and Pigments, vol. 90, no. 1, 18 November 2010 (2010-11-18), pages 41-47, XP028138265,
- VOLKOVA, K. D. et al.: "Spectroscopic study of squaraines as protein-sensi- tive fluorescent dyes", Dyes and Pigments, vol. 72, no. 3, 16 November 2005 (2005-11-16), pages 285-292, XP005499297,

## Description

### TECHNICAL FIELD

The present application relates to the technical field of organic synthesis, and more particularly to a fluorescent dye and a preparation method therefore and use thereof.

### BACKGROUND OF THE INVENTION

Recently, with the rapid development in life science, particularly in cell biology, molecular fluorescence imaging technology has also achieved a great progress. For example, various apparatuses and instruments such as confocal fluorescence imaging microscope and fluorescence spectrum/lifetime instrument based on the fluorescence imaging analysis technology have been developed; especially super-resolution imaging methods recently developed for the research of intracellular microstructures, such as stimulated emission depletion super-resolution imaging (STED) and polarization super-resolution imaging, etc., can break through the limitation of optical diffraction, and provide high-resolution imaging for the microstructure of less than 200 nm. As a key part in the fluorescence imaging and the super-resolution fluorescence imaging, the development of fluorescence dye greatly affects the development of imaging technology and the improvement of bioimaging analysis. However, since the STED super-resolution imaging is a method to improve the resolution based on the principle of stimulated radiation in physics, and the used erase light is of higher power, which has a high requirement for the photostability of the fluorescent material, and a large damage to biological tissues, making it difficult to apply the STED super-resolution imaging in the analysis of living cells. Since the super-resolution imaging for living cells is of greater significance for the investigation of actual biological structures and processes, the research and development a more efficient fluorescent material for the super-resolution imaging of living cell microstructures is of great significance.

Commonly-used fluorescent imaging materials mainly include organic single molecule fluorescent dyes, organic polymer fluorescent nanoparticles and inorganic nano-luminescent materials, etc.. Compared to the organic polymer nanoparticles and inorganic nano-luminescent materials, the organic single molecule fluorescent dyes have attracted much attention due to obvious advantages, and thus are widely applied. There are a variety of dyes, and the commonly-used organic fluorescent dyes can be divided into coumarins, naphthalimides, BODIPYs, fluoresceins, rhodamines, cyanine fluorescent dyes according to the luminescence wavelength. Due to the advantages of appropriate absorption and emission properties and low cytotoxicity, such dyes have been widely used in the life science analysis. Among these dyes, cyanine fluorescent dyes have various excellent characteristics, such as adjustable absorption and emission wavelengths in 400-800 nm, large molar absorptivity (×10⁵), adequate fluorescence quantum yield and low cytotoxicity, facilitating its widely application in various researches such as intracellular single molecule level, living small animals, etc..

Cyanine fluorescent dyes include polymethine cyanine dyes, squaraine dyes, and croconate dyes, wherein the polymethine cyanine dyes are most applied and reported in the technical field of life science due to the presence of one unit of positive charge and modifiable structure. Compared to the methine cyanine dyes, the squaraine dyes, pertaining to inner salt fluorescent dyes due to the simultaneous presence of positive and negative charges, have smaller water-solubility and lower selectivity in intracellular staining, limiting the application in the field of life science. However, due to the presence of an electron-withdrawing four-membered squaric acid ring at the conjugate portion, the squaraine dyes exhibit relatively good photostability and high fluorescence quantum yield, and they are thus suitable as a sensitizing dye in the field of solar cells.

Currently, method for synthesizing and preparing squaraine dyes is mature, and the squaraine dyes are generally prepared by refluxing various quaternary ammonium salts and a squaric acid in an alcohol solvent. However, the asymmetric squaraine dyes often involve complicated preparation, have difficulty in purification and low yield, resulting in great difficulty in the activation and labeling application of the dyes.

US 2005/202565 A1 discloses reporter compounds based on squaric, croconic, and/or rhodizonic acid, among others, reactive intermediates used to synthesize the reporter compounds, and methods of synthesizing and using the reporter compounds, among others. The reporter compounds relate generally to the following structure:

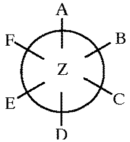

Here, Z is a four, five, or six-member aromatic ring, and A, B, C, D, E, and F are substituents of Z, Where F is absent when z is a five-member ring, and where E and F are absent when Z is a four-member ring. Generally, A, B, C, D, E, and F may be present in any order, although the order may be limited in certain embodiments. The dyes of US 2005/202565 A1 exhibit high intrinsic polarization in the absence of rotational motion, making them useful as tracers in fluorescence polarization (FP) assays. Fluorescence polarization immunoassays (FPI) are widely applied to quantify low molecular weight antigens. The assays are based on polarization measurements of antigens labeled with fluorescent probes. The requirement for polarization probes used in FPIs is that emission from the unbound labeled antigen be depolarized and increase upon binding to the antibody. Low molecular weight species labeled with the compounds of US 2005/202565 A1 can be used in such binding assays, and the unknown analyte concentration can determined by the change in polarized emission from the fluorescent tracer molecule. The fact that the fluorescence lifetime of compounds 15 and 16 is in the order of 1 to 2 ns makes these labels particularly useful as labels in polarization measurements of small molecular-weight antigens.

### SUMMARY OF THE INVENTION

An object of this application is to provide a fluorescent dye and a preparation method therefore and use thereof to overcome the above defects in the prior art, and to solve the problems of poor selectivity and complicated preparation of existing squaraine fluorescent dyes.

To achieve the above purpose, the technical solutions of the present invention are described as follows.

In a first aspect, the present invention provides a fluorescent dye according to claim 1.

In a second aspect, the present invention provides a method for preparing the fluorescent dye according to claim 2.

In a third aspect, the invention provides use of the fluorescent dye in the fluorescence imaging of living cells, the fluorescent labeling of intracellular microstructure and the STED super-resolution fluorescence imaging.

The preparation of the fluorescent dye provided herein is started by modifying one of the oxygen atoms of the squaric acid ring in the squaraine dye, and the specific process is described as follows. Lawesson's reagent, as a commonly-used chemical reagent to convert oxygen into sulfur, is used in the synthesis and modification of squaraine cyanine dyes to substitute an oxygen atom on the squaric acid ring with a sulfur atom. The resulting product is reacted with an alkylating agent to form a stable product, which is subsequently used to prepare the desired fluorescent dye of formula (I). The method introduces a substituent to the squaric acid ring in the middle of the squaraine dye, which can not only change the charge structure of the squaraine dye, but also improve the synthesis and application of the dye. In addition, this method can improve the stability of the groups such as alkyl attachéd on the oxygen atom of the squaric acid ring, which are unstable and easy to leave and decompose, and the resulting fluorescent dye is capable of permeating the membrane of living cells, and thus it is suitable for the fluorescence imaging of living cell microstructures, STED super-resolution fluorescence imaging and laser scanning confocal imaging of living cells.

### BRIEF DESCRIPTION OF THE DRAWINGS

Fig. 1 shows the laser confocal imaging of living cells in the use of ten representative dyes according to Example 11 of the present invention.
Fig. 2 shows the STOM super-resolution fluorescence imaging of macrophages in the use of two representative dyes according to Example 11 of the present invention.

### DETAILED DESCRIPTION OF EMBODIMENTS

The present invention will be further described below with reference to the examples to make the technical problems to be solved, technical solutions and beneficial effects of the invention clearer. It should be understood that the examples described herein are merely illustrative of the present invention and are not intended to limit the present invention.

In a first aspect, the invention provides a fluorescent dye of formula (I):

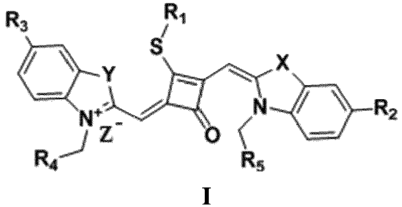

The preparation of the fluorescent dye provided in this example is started by modifying one of the oxygen atoms of the squaric acid ring in the squaraine dye. Lawesson's reagent, as a commonly-used chemical reagent to convert oxygen into sulfur, is used in the synthesis and modification of squaraine cyanine dyes to substitute an oxygen atom on the squaric acid ring with a sulfur atom. The resulting product is reacted with an alkylating agent react to form a stable product, which is subsequently used to prepare the desired fluorescent dye of formula (I). The method introduces a substituent to the squaric acid ring in the middle of the squaraine dye, which can not only change the charge structure of the squaraine dye, but also improve the synthesis and application of the dye. In addition, this method can improve the stability of the groups such as alkyl attached on the oxygen atom of the squaric acid ring, which are unstable and easy to leave and decompose, and the resulting fluorescent dye is capable of permeating the membrane of living cells, and thus it is suitable for the fluorescence imaging of living cell microstructures, STED super-resolution fluorescence imaging and laser scanning confocal imaging of living cells.

In the fluorescent dye represented by the above formula I, Z⁻ is a halide anion or OTs⁻.

In the fluorescent dye represented by the above formula I,
R₁ is (CH₂)ₙ₋₁CH₃, (CH₂)ₙR₇, (CH₂)ₘC₆H₅ or (CH₂)ₘC₆H₄R₇;
R₂ and R₃ are same or different and selected from H, F, Cl, Br, I, (CH₂)ₙ₋₁CH₃, (CH₂)ₙR₈, CH₂C₆H₅, CH₂C₆H₄R₈, O(CH2)ₙ₋₁CH₃, O(CH₂)ₙR₈, OCH₂C₆H₅, OCH₂C₆H₄R₈ or CN;
R₄, R₅ and R₆ are same or different and selected from (CH₂)ₙ₋₁CH₃, (CH₂)ₙR₈, CH₂C₆H₅ and CH₂C₆H₄R₈;
wherein R₇ and R₈ are functional groups, and n and m are integers respectively selected from 1-18 and
Further, R₇ is C₆H₅, C₆H₄R₉, SO₃R₁₀ or COOR₁₁; R₈ is SO₃R₁₀ or COOR₁₁;
Further, in the fluorescent dye, R₉ is arsonic acid, boric acid, carboxylic acid, sulfonic acid, amino, hydroxyl, sulfhydryl or a C₁-C₁₈ alkyl;
R₁₀ is N(R₁₂R₁₃R₁₄R₁₅);
R₁₁ is a C₁-C₁₈ alkyl;
wherein R₁₂, R₁₃, R₁₄ and R₁₅ are same or different and selected from H, C₁-C₁₈ alkyl and (CH₂)ₚOH, n is an integer and p is an integer selected from 0-18.

For X and Y in the formula (I), different groups can be selected respectively, preferably, X and Y are independently selected from the following molecular structures: C(CH₃)₂, O, S, NCH₃ and NCH₂CH₃, specifically, representative molecular structure of more preferred subclasses are listed below (Iₙa to Xₙa, and n is an integer):

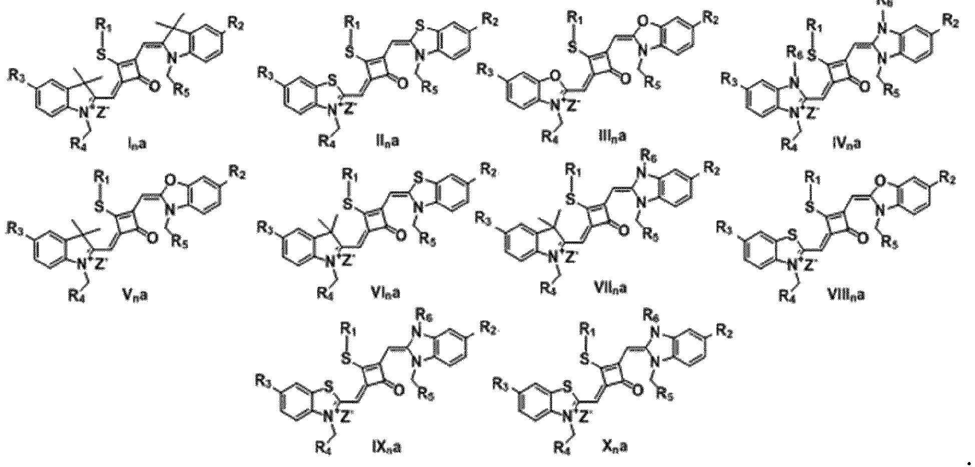

In a second aspect, the present invention provides a method for preparing the above fluorescent dye, comprising:
S01: preparing a compound of formula (III) and Lawesson's reagent;
S02: dissolving the compound of formula (III) and the Lawesson's reagent in a first solvent; and subjecting the reaction mixture to substitution reaction under heating in an inert gas to produce a compound of formula (II); and
S03: subjecting the compound of formula (II) and a nucleophilic reagent of R₁Z to addition reaction to produce the fluorescent dye of formula (I);

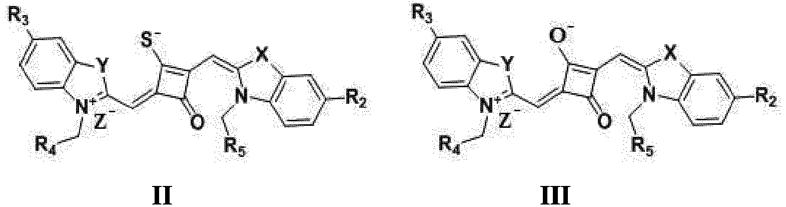

The key technology of the preparation method is to substitute the oxygen atom on the ring at the middle of the squaraine dye with a sulfur atom, wherein a key reagent used is Lawesson's reagent. Then the sulfur-containing universal intermediate (sulfur-substituted squaraine dye) is used for the subsequent preparation of squaraine dyes with different substituents.

Further, in step S02, the first solvent is preferably a mixed solution of dichloromethane and anhydrous tetrahydrofuran; a temperature of the substitution reaction is preferably in a range of 40°C to 50°C; and a molar ratio of the compound of formula III to the Lawesson's reagent is preferably 1:1.

Further, in step S03, a temperature of the addition reaction is preferably in a range of 25°C to 60°C; and a molar ratio of the compound of formula II to the nucleophilic reagent R₁Z is preferably 1: 2 to 5.

Further, in the above preparation method, the compound of formula (III) is prepared by the following steps:
E01: preparing a compound of formula (VI) and a compound of formula (VII);
E02: subjecting the compound of formula (VI) and a nucleophilic reagent R₄Z to addition reaction to produce a compound of formula (V);
E03: subjecting the compound of formula (VII) and a nucleophilic reagent R₅Z to addition reaction to produce a compound of formula (IV);
E04: mixing the compound of formula (V) and the compound of formula (IV) with a squaric acid solution, and carrying out a reaction in the presence of a catalyst to obtain a compound of formula (III);

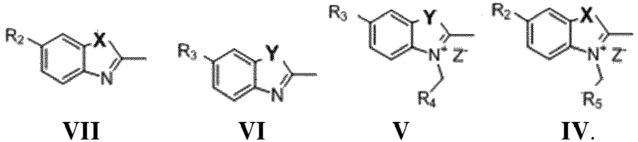

Further, in step E04, a solvent in the squaric acid solution is preferably ethanol; the catalyst is preferably triethyl orthoformate; and a molar ratio of the compound of formula (IV) to the compound of formula (V) to squaric acid in the squaric solution is preferably 1: 0.8 to 1.2: 0.8 to 1.2.

Specifically, the fluorescent dye of this example is prepared as follows.
(1) Compound Iₙe such as 2,3,3-trimethyl-5-R₂(R₃)-3H-indoline and benzoindoline carrying R₄ or R₅, and related substituted benzothiazole, benzoxazole and benzimidazole (is reacted with R₄(R₅)CH₂Z to obtain a corresponding quaternary ammonium salt Iₙd-Xₙd, wherein Z⁻ is a halide anion or OTs⁻; a reaction temperature is in a range of 80°C to 148°C; a reaction time is in a range of 6 h to 36 h; and a reaction solvent is selected from toluene, o-dichlorobenzene, ethanol or acetonitrile; and a molar ratio of the compound Iₙe to R₄(R₅)CH₂Z is in a range of 1:1 to 1:4. In the case that Z is chlorine or bromine, a small amount of KI may be added as a catalyst in the reaction to accelerate the reaction, and to shorten the response time.
(2) Synthesis of corresponding squaraine dyes
   a. Synthesis of symmetrical squaraine dyes (X=Y, Iₙc-IVₙc)
      1 mol of 1,2-dihydroxy-3,4-cyclobutenedione is dissolved in a certain amount of absolute ethanol, to which the catalytic amount of triethyl orthoformate is added. The reaction mixture is refluxed under nitrogen until the solids are completely dissolved. Then 2.2 mol to 2.5 mol of indoline quaternary ammonium salt is added, and the reaction mixture is refluxed sequentially until it is turned into dark blue. Then the refluxing is stopped, and the reaction mixture is cooled to room temperature, dried under vacuum and purified to give a corresponding blue solid.
   b. Synthesis of asymmetrical squaraine dyes (X≠Y, Vₙc-Xₙc)
      1 mol of 1,2-dihydroxy-3,4-cyclobutenedione is dissolved in a certain amount of absolute ethyl alcohol, to which catalytic amount of triethyl orthoformate is added. The reaction mixture is refluxed under nitrogen until the solids are completely dissolved. 1 mol of quaternary ammonium salt A is added, and the reaction mixture is refluxed until the reaction is completed. Then 1 mol to 1.2 mol of quaternary ammonium salt B is added, and the reaction mixture is continuously heated until the raw materials are completely consumed. The reaction mixture is cooled to room temperature, dried under vacuum and purified by column chromatography to give a corresponding blue solid.
   c. Sulfur-substitution of the oxygen atom on the squaric acid in the middle of a squaraine dye (Iₙb-Xₙb)
      1 mol of dye Iₙc-Xₙc is added to a 100 mL round-bottomed flask, to which 30 mL of a mixed solvent of anhydrous dichloromethane and anhydrous tetrahydrofuran is added. After the solid dye is completely dissolved, 1 mol of a sulfurizing reagent capable of substituting an oxygen atom with a sulfur atom is added, and the reaction mixture is heated at 40°C under argon protection for 2 h to 5 h. Then heating is stopped, and the reaction mixture is dried under vacuum, it should be noted that some protective measures should be adopted to prevent odor from spreading. Then the resulting residue is washed with a mixed solution of anhydrous diethyl ether and n-hexane, dissolved and purified by column chromatography to give a corresponding blue solid (Iₙb-Xₙb).
   d. Preparation of the target dyes
      1 mol of sulfur-substituted squaraine dye (Iₙb-Xₙb) is added to 20 mL of anhydrous acetonitrile, to which 2 mol to 5 mol of a corresponding electrophilic reagent (R₁X) is added. Then the reaction mixture is heated at a temperature from room temperature to 60°C for different times. When the reaction is determined to be completed by TLC, the reaction mixture is dried under vacuum and purified by silica gel column chromatography to give a corresponding target dye (Iₙa-Xₙa) with a yield of 30% to 80%.

In a third aspect, this example provides use of the above fluorescent dye in the fluorescence imaging of living cells and the fluorescent labeling of intracellular microstructure and the STED super-resolution fluorescence imaging. Due to the ability to permeate living cell membranes, the above fluorescent dye can be used in the fluorescence imaging of living cell microstructures, and in the STED super-resolution fluorescence imaging and laser scanning confocal imaging of live cells.

The present invention has been tested for many times, and a part of the test results are now given as a reference to further describe the invention in detail. The present invention will be described in detail below with reference to preferred examples.

### Example 1

In this example, in formula (I), both X and Y were C(CH₃)₂, based on which, structures of ten representative dyes respectively varying in R₁ to R₅ were shown as follows, wherein formulae I₂a, I₄a, I₅a, I₆a, I₇a, I₈a, I₉a, I₁₀a are non-inventive embodiments, not falling under the scope of the claims. 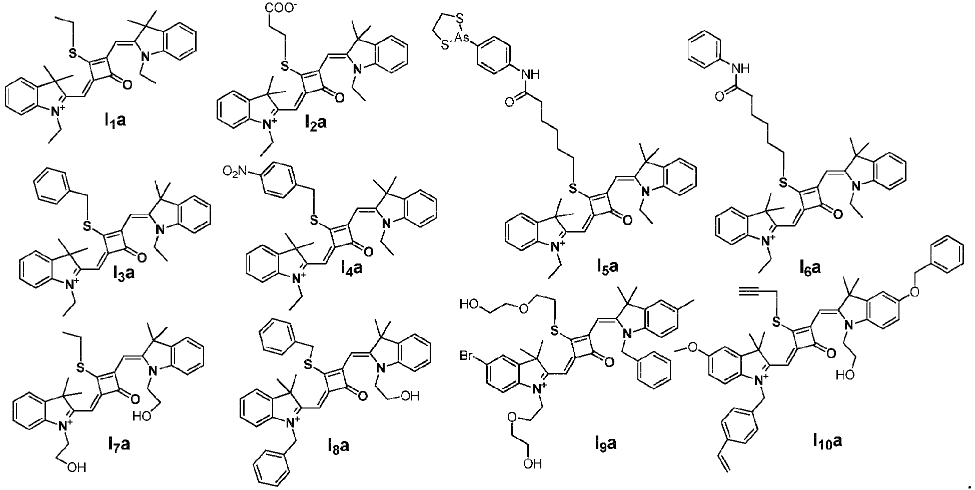
1. Synthetic routes of dyes I₁a to I₆a were shown as follows: 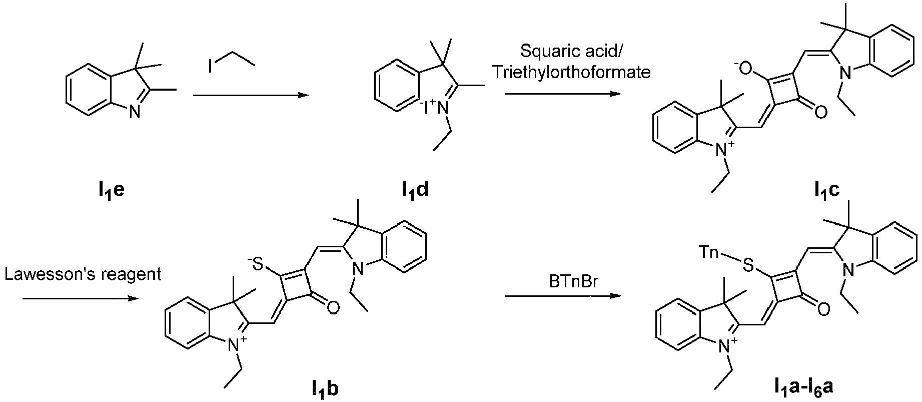 wherein, the structural formulas of Tₙ (n=1 to 6) were respectively shown as follows:

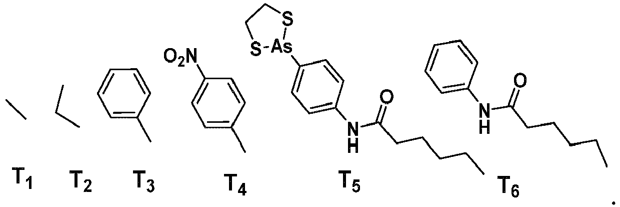

(1) 1 mol of 2,3,3-trimethyl-3H-indoline and 2 mol of iodoethane were added to 20 mL of dry toluene. The reaction mixture was heated under nitrogen protection for 5 h, and then cooled to room temperature. The reaction mixture was washed with anhydrous diethyl ether and recrystallized with propanone to give a white to pink quaternary ammonium salt solid (I₁d), which was dried and then stored for use.
(2) 1 mol of squaric acid was added to 50 mL of absolute ethanol, to which triethyl orthoformate was added as a catalyst. The reaction mixture was heated and refluxed under nitrogen protection until the squaric acid was completely dissolved. Then 2.2 mol of the quaternary ammonium salt (I₁d) was added, and the reaction mixture was continuously refluxed. After the reaction was confirmed to be completed by TLC, the reaction mixture was cooled, dried under reduced pressure to remove the solvent and purified by column chromatography to give a blue solid dye (I₁c).
(3) 1 mol of the dye (I₁c) was added to 25 mL of a mixed solvent of dry dichloromethane and tetrahydrofuran, to which 2 mol of Lawesson's reagent was added. The reaction mixture was stirred at 40°C for 3 h, and then the reaction was stopped. The reaction mixture was dried under vacuum and purified by column chromatography to give a bluish green centrally sulfur-substituted squaraine dye (I₁b).
(4) 1 mol of the sulfur-substituted squaraine dye (I₁b) was added to dry acetonitrile, to which a bromo-substituted intermediate TₙBr (Tₙ was selected from T₁ to T₆) was added. The reaction mixture was stirred at a temperature from room temperature to 60°C for different times, dried under vacuum and purified by column chromatography to give a corresponding target dye (**I₁a** to **I₆a**).

The compounds **I₁a** to **I₆a** were characterized as follows.
**I₁a**: ¹H-NMR (400 MHz, CDCl₃): 1.38 (t, 3H, CH₃, *J*=8.0 Hz), 1.41 (t, 6H, CH₃, *J*=8.0 Hz), 1.83 (s, 12H, CH₃), 3.17 (q, 2H, CH₂, *J*=8.0 Hz), 4.29 (q, 4H, CH₂, *J*=8.0 Hz), 6.48 (s, 2H, CH), 7.07 (d, 2H, ArH, *J*=8.0 Hz), 7.18 (t, 2H, ArH, *J*=8.0 Hz), 7.35 (q, 4H, ArH, *J*=8.0 Hz);
¹³C-NMR (100 MHz, CDCl₃): 12.26, 13.31, 27.71, 31.04, 40.40, 50.14, 87.64, 110.40, 122.61, 124.61, 128.12, 142.02, 142.61, 173.45, 186.68, 204.15.
HRMS-ESI: *m*/*z* calcd M⁺ for C₃₂H₃₇N₂OS⁺, 497.2621; found, 497.2625.
**I₂a**: HRMS-ESI: m/z calcd [M+H]⁺ for C₃₃H₃₇N₂O₃S⁺, 541.2447; found, 541.2452.
**I₃a**: ¹H-NMR (400 MHz, CDCl₃): 1.41 (t, 6H, CH₃, *J*=8.0 Hz), 1.83 (s, 12H, CH₃), 3.70 (s, 2H, CH₂), 4.29 (q, 4H, CH₂, *J*=8.0 Hz), 6.48 (s, 2H, CH), 7.07 (m, 4H, ArH), 7.18 (t, 2H, ArH, *J*=8.0 Hz), 7.35 (q, 4H, ArH, *J*=8.0 Hz), 7.42 (m, 3H, ArH);
¹³C-NMR (100 MHz, CDCl₃): 12.26, 27.71, 28.61, 40.40, 50.14, 87.64, 110.40, 122.61, 124.61, 126.65, 127.71, 128.12, 128.29, 140.83, 142.02, 142.61, 173.45, 186.68, 204.15.
HRMS-ESI: *m*/*z* calcd M⁺ for C₃₇H₃₉N₂OS⁺, 559.2778; found, 559.2781.
**I₄a**: HRMS-ESI: *m*/*z* calcd M⁺ for C₃₇H₃₈N₃O₃S⁺, 604.2628; found, 604.2633.
**I₅a**: ¹H-NMR (400 MHz, CDCl₃): 1.43 (t, 6H, CH₃, *J*=8.0 Hz), 1.70 (m, 14H, CH₃*&* CH₂), 1.85 (m, 2H, CH₂), 2.01 (m, 2H, CH₂), 2.72 (t, 2H, CH₂, *J*=8.0 Hz), 3.11 (m, 2H, CH₂), 3.28 (m, 2H, CH₂), 3.63 (t, 2H, CH₂, *J*=8.0 Hz), 4.22 (q, 4H, CH₂, *J*=8.0 Hz), 5.72 (s, 2H, CH), 7.17 (d, 2H, ArH, *J*=8.0 Hz), 7.29 (m, 2H, ArH), 7.39 (m, 4H, ArH), 7.46 (m, 2H, ArH), 7.96 (d, 2H, ArH, *J*=8.0 Hz), 9.69 (s, 1H, NH);
¹³C-NMR (100 MHz, CDCl₃): 12.57, 25.19, 26.17, 27.59, 30.00, 32.27, 37.24, 40.74, 41.80, 50.64, 88.32, 111.49, 120.01, 122.58, 126.41, 128.87, 131.19, 137.11, 140.59, 141.01, 142.53, 142.61, 171.82, 171.96, 172.01, 173.08, 173.43, 174.12, 175.65.
HRMS-ESI: *m*/*z* calcd M⁺ for C₄₄H₅₁AsN₃O₂S₃⁺, 824.2354; found, 824.2358.
**I₆a**: HRMS-ESI: *m*/*z* calcd M⁺ for C₄₂H₄₈N₃O₂S⁺, 658.3462; found, 658.3466.

2. The synthetic route of dye I₇a was shown as follows: 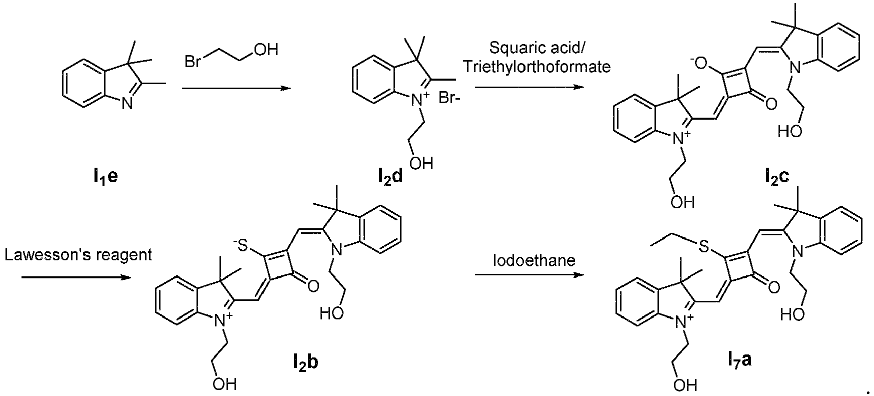
(1) 1 mol of 2,3,3-trimethyl-3H-indoline and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (I₂d), which was dried and then stored for use.
(2) 1 mol of squaric acid was added to 50 mL of absolute ethanol, to which triethyl orthoformate was added as a catalyst. The reaction mixture was heated and refluxed under nitrogen protection until the squaric acid was completely dissolved, and then 2.2 mol of the quaternary ammonium salt (I₂d) was added. The reaction mixture was continuously heated and refluxed until the reaction was confirmed to be completed by TLC. The reaction mixture was cooled, dried under reduced pressure to remove the solvent and purified by column chromatography to give a blue solid dye (I₂c).
(3) 1 mol of the dye (I₂c) was added to 25 mL of a mixed solvent of dry dichloromethane and tetrahydrofuran, to which 2 mol of Lawesson's reagent was added. After the reaction mixture was stirred at 40°C for 3 h, the reaction was stopped. The reaction mixture was dried under vacuum and purified by column chromatography to give a bluish green centrally sulfur-substituted squaraine dye (I₂b).
(4) 1 mol of the sulfur-substituted squaraine dye (I₂b) was added to a dry acetonitrile solvent, to which 2.5 mol of iodoethane was added. The reaction mixture was thermally stirred at room temperature for 2 h. After cooled to room temperature, the reaction mixture was dried under vacuum and purified by column chromatography to give a target dye (**I₇a**).

The compound **I₇a** was characterized as follows:
¹H-NMR (400 MHz, CDCl₃): 1.39 (t, 3H, CH₃, *J*=8.0 Hz), 1.83 (s, 12H, CH₃), 3.19 (q, 2H, CH₂, *J*=8.0 Hz), 3.58 (m, 6H, CH₂*&*OH), 3.98 (m, 4H, CH₂), 6.48 (s, 2H, CH), 7.07 (d, 2H, ArH, *J*=8.0 Hz), 7.18 (t, 2H, ArH, *J*=8.0 Hz), 7.35 (q, 4H, ArH, *J*=8.0 Hz);
¹³C-NMR (100 MHz, CDCl₃): 13.31, 27.71, 31.04, 40.40, 53.14, 59.81, 87.64, 110.40, 122.61, 124.56, 128.12, 142.02, 142.63, 173.45, 186.68,204.10.
HRMS-ESI: *m*/*z* calcd M⁺ for C₃₂H₃₇N₂O₃S⁺, 529.2519; found, 529.2523.

3. The synthetic route of dye **I₈a** was shown as follows: 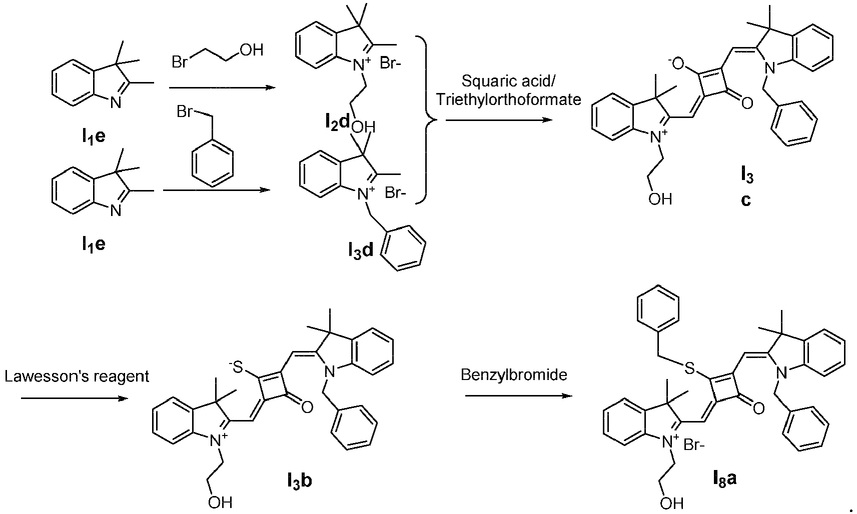
(1) 1 mol of 2,3,3-trimethyl-3H-indoline and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (I₂d), which was dried and stored for use.
(2) 1 mol of 2,3,3-trimethyl-3H-indoline and 3 mol of benzyl bromide were added to 20 mL of dry toluene. The reaction mixture was heated under nitrogen protection for 24 h. After cooled to room temperature, the reaction mixture was washed with anhydrous diethyl ether and crystallized with propanone to give a white to pink quaternary ammonium salt solid (I₃d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (I₂d) and 1.2 mol of the quaternary ammonium salt (I₃d) were added. The reaction mixture was continuously heated and refluxed until the reaction was confirmed to be completed by TLC. The reaction mixture was cooled, dried under reduced pressure to remove the solvent and purified by column chromatography to give a blue solid dye (I₃c).
(4) 1 mol of the dye (I₃c) was added to 25 mL of a mixed solvent of dry dichloromethane and tetrahydrofuran, to which 2 mol of Lawesson's reagent was added. After the reaction mixture was stirred at 40°C for 3 h, the reaction was stopped. Then the reaction mixture was dried under vacuum, and purified by column chromatography to give a bluish green centrally sulfur-substituted squaraine dye (I₃b).
(5) 1 mol of the sulfur-substituted squaraine dye (I₃b) was added to a dry acetonitrile solvent, to which 2.5 mol of benzyl bromide was added. After thermally stirred at room temperature for 2 h, the reaction was cooled to room temperature, dried under vacuum and purified by column chromatography to give a target dye (**I₈a**).

The compound (**I₈a**) was characterized as follows: HRMS-ESI: m/z calcd M⁺ for C₄₂H₄₁N₂O₂S⁺, 637.2883; found, 637.2886.

4. The synthetic route of dye I₉a was shoen as follows: 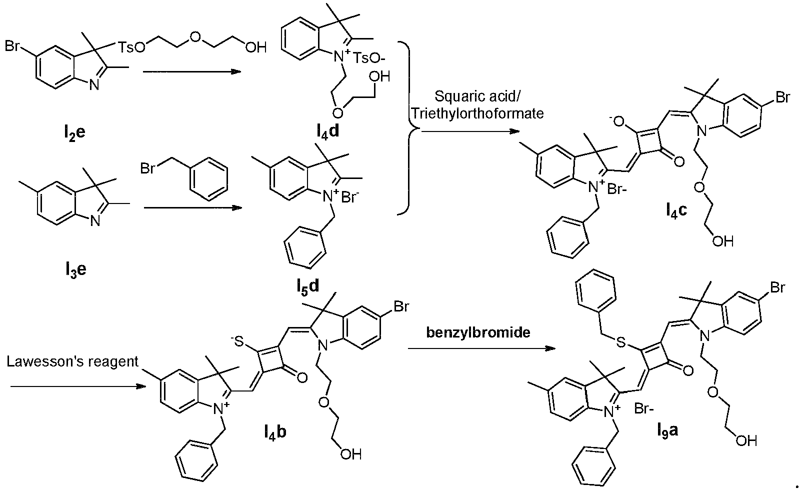
(1) 1 mol of 2,3,3-trimethyl-5-bromo-3H-indoline and 3 mol of ethylene glycol tosylate were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (I₄d), which was dried and stored for use.
(2) 1 mol of 2,3,3-trimethyl-5-methyl-3H-indoline and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (I₅d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (I₄d) and 1.2 mol of the quaternary ammonium salt (I₅d) were added. The reaction mixture was continuously heated and refluxed until the reaction was confirmed to be completed by TLC. The reaction mixture was cooled, dried under reduced pressure to remove the solvent and purified by column chromatography to give a blue solid dye (I₄c).
(4) 1 mol of the dye (I₄c) was added to 25 mL of a mixed solvent of dry dichloromethane and tetrahydrofuran, to which 2 mol of Lawesson's reagent was added. After the reaction mixture was stirred at 40°C for 3 h, the reaction was stopped. The reaction mixture was dried under vacuum, and purified by column chromatography to give a bluish green centrally sulfur-substituted squaraine dye (I₄b).
(5) 1 mol of the sulfur-substituted squaraine dye (I₄b) was added to a dry acetonitrile solvent, to which 2.5 mol of benzyl bromide was added. The reaction mixture was thermally stirred for 2 h at room temperature. After cooled to room temperature, the reaction mixture was dried under vacuum and purified by column chromatography to give a target dye (I₉a).

The compound **I₉a** was characterized as follows:
HRMS-ESI: m/z calcd M⁺ for C₄₂H₄₈BrN₂O₅S⁺, 771.2462; found, 771.2484.

5. The synthetic route of dye I₉a was shown as follows: 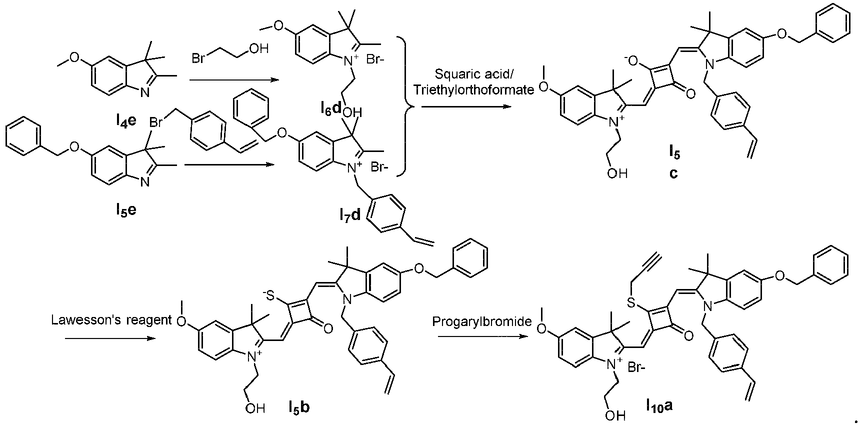
(1) 1 mol of 2,3,3-trimethyl-5-methoxy-3H-indoline (I₄e) and 3 mol of p-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (I₆d) which was dried and stored for use.
(2) 1 mol of 2,3,3-trimethyl-5-benzyloxy-3H-indoline (I₅e) and 3 mol of 4-vinyl benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (I₇d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completed dissolved. Then 0.8 mol of the quaternary ammonium salt (I₆d) and 1.2 mol of quaternary ammonium salt (I₇d) were added. The reaction mixture was continuously heated and refluxed until the reaction was confirmed to be completed by TLC. The reaction mixture was cooled, dried under reduced pressure to remove the solvent and purified by column chromatography to give a blue solid dye (I₅c).
(4) 1 mol of the dye (I₅c) was added to 25 mL of a mixed solvent of dry dichloromethane and tetrahydrofuran to which 2 mol of Lawesson's reagent was added. After the reaction mixture was stirred at 40°C for 3 h, the reaction was stopped. The reaction mixture was dried under vacuum, and purified by column chromatography to give a bluish green centrally sulfur-substituted squaraine dye (I₅b).
(5) 1 mol of the sulfur-substituted squaraine dye (I₅b) was added to a dry acetonitrile solvent to which 2.5 mol of benzyl bromide was added. The reaction mixture was thermally stirred at room temperature for 2 h. After cooled to room temperature, the reaction mixture was dried under vacuum and purified by column chromatography to give a target dye (**I₁₀a**).

The compound **I₁₀a** was characterized as follows:
¹H-NMR (400 MHz, CDCl₃): 1.83 (s, 12H, CH₃), 2.86 (t, 1H, CH, *J*=8.0 Hz),3.56 (t, 2H, CH₂, *J*=8.0 Hz), 3.65 (s, 1H, OH), 3.83 (s, 3H, CH₃), 3.98 (t, 2H, CH₂, *J*= 8.0 Hz), 4.33 (s, 2H, CH₂), 4.87 (s, 2H, CH₂), 5.16 (s, 2H, CH₂), 5.23 (m, 1H, =CH₂), 5.74 (m, 1H, =CH₂), 5.82 (s, 1H, CH), 5.92 (s, 1H, CH), 6.69 (m, 1H, =CH), 7.19 (m, 4H, ArH), 7.38 (m, 4H, ArH), 7.54 (m, 2H, ArH), 7.86 (m, 3H, ArH), 8.10 (m, 2H, ArH);
¹³C-NMR (100 MHz, CDCl₃): 22.93, 24.70, 27.71, 49.60, 53.14, 55.83, 58.20, 59.81, 70.79, 74.62, 94.64, 107.80, 110.41, 111.52, 114.31, 124.61, 126.80, 127.11, 128.12, 129.73, 131.80, 133.72, 134.30, 135.91, 142.02, 142.61, 148.70, 149.11, 152.93, 160.30, 163.42, 173.45, 186.68.
HRMS-ESI: *m*/*z* calcd M⁺ for C₄₈H₄₇N₂O₄S⁺, 747.3251; found, 747.3256.

### Example 2

Listed herein were merely partial representative structures, in the formula I, both X and Y were O, based on which, structures of ten representative dyes respectively varying in R₁ to R₅ were shown below, wherein formulae II₂a, II₃a, II₄a, II₅a, II₆a, II₇a, II₈a, II₉a, II₁₀a are non-inventive embodiments, not falling under the scope of the claims. 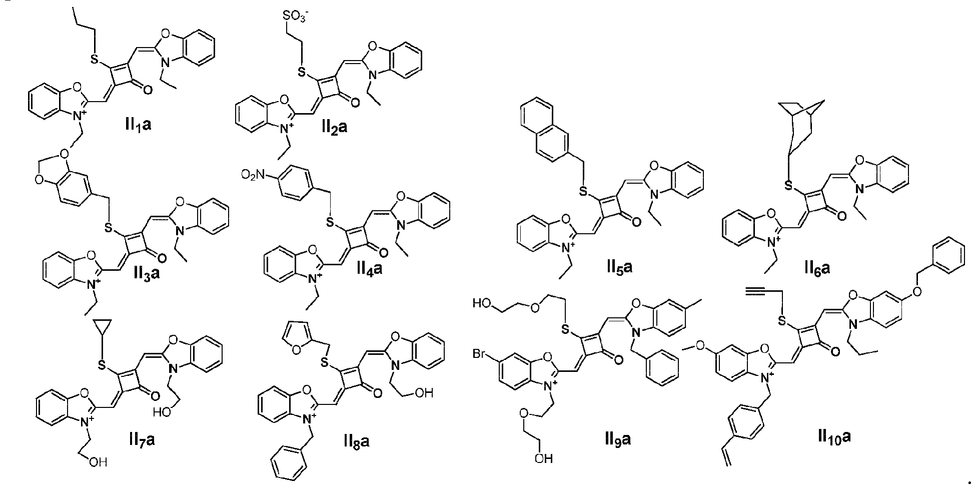
1. Synthetic routes of dyes II₁a to II₆a were shown as follows: 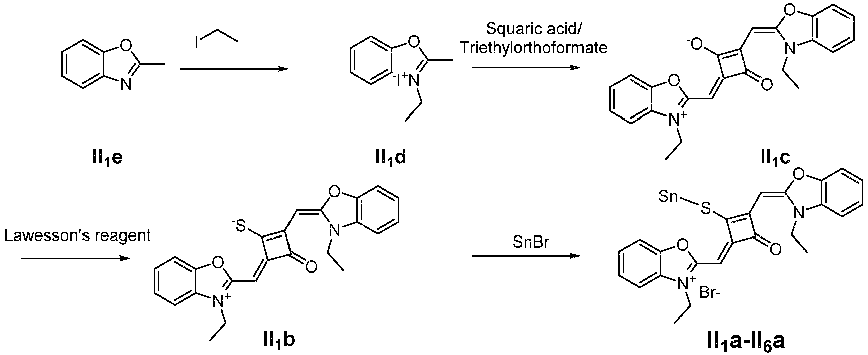 wherein, the structural formulas of Sₙ (n=1 to 6) were respectively shown as follows: 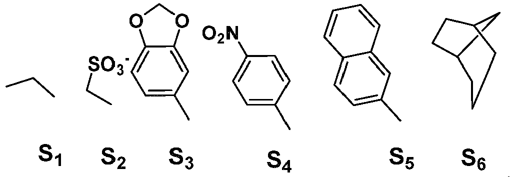
   (1) 1mol of 2-methylbenzoxazole and 2 mol of iodoethane were added to 20 mL of dry 1,2-dichlorobenzene. The reaction mixture was heated under nitrogen protection for 5 h, and then cooled to room temperature. The reaction mixture was washed with anhydrous diethyl ether and recrystallized with propanone to give a white to pink quaternary ammonium salt solid (II₁d) which was dried and then stored for use.
   (2) 1 mol of squaric acid was added to 50 mL of absolute ethanol, to which triethyl orthoformate was added as a catalyst. The reaction mixture was heated and refluxed under nitrogen protection until the squaric acid was completely dissolved. Then 2.2 mol of the quaternary ammonium salt (II₁d) was added, and the reaction mixture was continuously heated and refluxed. After the reaction was confirmed to be completed by TLC, the reaction mixture was cooled, dried under reduced pressure to remove the solvent, and purified by column chromatography to give a blue solid dye (II₁c).
   (3) 1 mol of the dye (II₁c) was added to 25 mL of mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (II₁b).
   (4) 1 mol of the sulfur-substituted squaraine dye (II₁b) was added to dry acetonitrile, to which a corresponding bromo intermediate SₙBr (Sₙ was selected from S₁ to S₆) was added. The reaction mixture was stirred at a temperature from room temperature to 60°C, dried under vacuum, and purified by column chromatography to give a corresponding target dye (**II₁a to II₆a**).

The compounds **II₁a to II₆a** were characterized as follows:
**II₁a**: ¹H-NMR (400 MHz, CDCl₃): 1.01 (t, 3H, CH₃, *J*=8.0 Hz), 1.42 (t, 6H, CH₃, *J*=8.0 Hz), 1.69 (m, 2H, CH₂), 3.24 (t, 2H, CH₂, *J*=8.0 Hz), 4.37 (q, 4H, CH2, *J*=8.0 Hz), 6.69 (s, 2H, CH), 7.09 (d, 2H, ArH, *J*=8.0 Hz), 7.26 (t, 2H, ArH, *J*=8.0 Hz), 7.38 (q, 4H, ArH,*J*=8.0 Hz);
¹³C-NMR (100 MHz, CDCl₃): 12.11, 14.20, 25.51, 34.04, 40.40, 87.64, 110.40, 122.61, 124.61, 128.12, 142.02, 142.61, 173.45, 186.68, 204.15.
HRMS-ESI: *m*/*z* calcd M⁺ for C₂₇H₂₇N₂O₃S⁺, 459.1737; found, 459.1741.
**II₂a**: HRMS-ESI: *m*/*z* calcd [M⁺H]⁺ for C₂₆H₂₅N₂O₆S₂, 525.1076; found, 525.1081.
**II₃a**: ¹H-NMR (400 MHz, CDCl₃): 1.39 (t, 6H, CH₃, *J*= 8.0 Hz), 4.41 (q, 4H, CH₂, *J*=8.0 Hz), 4.62 (s, 2H, CH₂), 6.17 (s, 2H, CH₂), 6.57 (s, 2H, CH), 7.01 (m, 4H, ArH), 7.30 (m, 3H, ArH), 7.38 (m, 4H, ArH);
¹³C-NMR (100 MHz, CDCl₃): 14.27, 43.20, 46.01, 82.53, 101.20, 108.79, 112.40, 122.61, 124.61, 128.12, 133.10, 142.02, 142.61, 148.73, 156.31, 173.45, 186.68, 202.09.
HRMS-ESI: *m*/*z* calcd M⁺ for C₃₂H₂₇N₂O₅S⁺, 551.1635; found, 551.1638.
**II₄a**: HRMS-ESI: *m*/*z* calcd M⁺ for C₃₁H₂₆N₃O₅S⁺, 552.1588; found, 552.1592.
**II₅a**: ¹H-NMR (400 MHz, CDCl₃): 1.40 (t, 6H, CH₃, *J*=8.0 Hz), 4.43 (q, 4H, CH₂, *J*=8.0 Hz), 4.72 (s, 2H, CH₂), 6.63 (s, 2H, CH), 7.10 (m, 3H, ArH), 7.43 (m, 6H, ArH), 7.62 (m, 4H, ArH), 7.91 (m, 2H, ArH);
¹³C-NMR (100 MHz, CDCl₃): 13.98, 42.40, 46.13, 83.35, 101.12, 107.63, 112.27, 123.52, 125.10, 128.12, 134.03, 142.71, 144.15, 148.10, 155.30, 174.69, 186.04, 204.11.
HRMS-ESI: m/z calcd M⁺ for C₃₅H₂₉N₂O₃S⁺, 557.1893; found, 557.1897.
**II₆a:** HRMS-ESI: *m*/*z* calcd M⁺ for C₃₂H₃₃N₂O₃S⁺, 525.2206; found, 525.2210.

2. The synthetic route of dye II₇a was shown as follows: 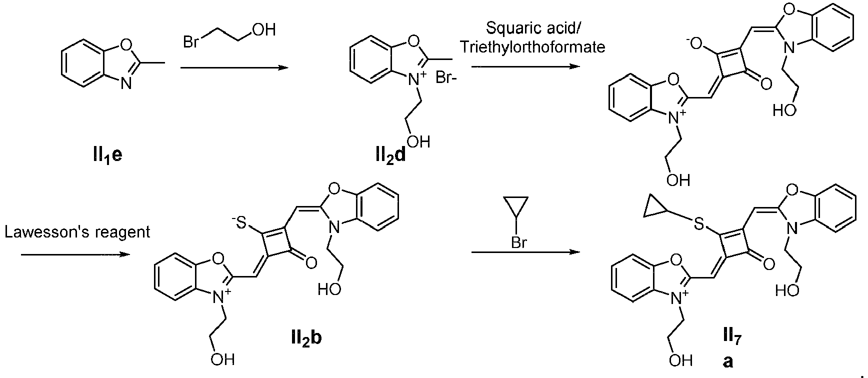
(1) 1mol of 2-methylbenzoxazole and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (II₂d), which was dried and stored for use.
(2) 1 mol of squaric acid was added to 50 mL of absolute ethanol, to which triethyl orthoformate was added as a catalyst. The reaction mixture was heated and refluxed under nitrogen protection until the squaric acid was completely dissolved, then 2.2 mol of the quaternary ammonium salt (II₂d) was added. The reaction mixture was continuously heated and refluxed until the reaction was confirmed to be completed by TLC. The reaction mixture was cooled, dried under reduced pressure to remove the solvent, and purified by column chromatography to give a blue solid dye (II₂c).
(3) 1 mol of the dye (II₂c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (II₂b).
(4) 1 mol of the sulfur-substituted squaraine dye (II₂b) was added to a dry acetonitrile solvent, to which 2.5 mol of cyclopropyl bromide was added. The reaction mixture was thermally stirred at room temperature for 2 h. After cooled to room temperature, the reaction mixture was dried under vacuum and purified by column chromatography to give a target dye (**II₇a**).

The compound **II₇a** was characterized as follows:
¹H-NMR (400 MHz, CDCl₃): 0.48 (m, 2H, CH₂), 0.76 (m, 2H, CH₂), 1.36 (m, 1H, CH), 3.56 (s, 2H, OH), 3.63 (q, 4H, CH₂, *J*=8.0 Hz), 3.98 (q, 4H, CH₂, J = 8.0 Hz), 6.34 (s, 2H, CH), 7.02 (d, 2H, ArH, *J*=8.0 Hz), 7.21 (t, 2H, ArH, *J*=8.0 Hz), 7.38 (q, 4H, ArH, J = 8.0 Hz);
¹³C-NMR (100 MHz, CDCl₃): 6.89, 10.23, 42.38, 58.71, 86.67, 109.30, 122.11, 125.53, 127.91, 142.17, 143.28, 174.30, 187.10, 203.93.
HRMS-ESI: m/z calcd M⁺ for C₂₇H₂₅N₂O₅S₊, 489.1479; found, 489.1482.

3. The synthetic route of dye II₈a was shown as follows: 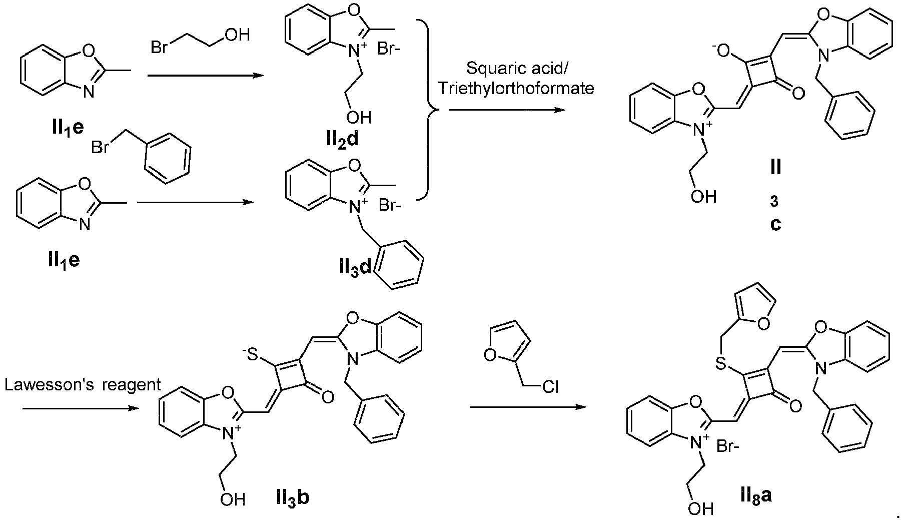
(1) 1mol of 2-methylbenzoxazole and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (I₂d), which was dried and stored for use.
(2) 1 mol of 2-methylbenzoxazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₃d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (II₂d) and 1.2 mol of the quaternary ammonium salt (II₃d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (II₃c).
(4) 1 mol of the dye (II₃c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (II₃b).
(5) 1 mol of the sulfur-substituted squaraine dye (II₃b) was added to a dry acetonitrile solvent, to which 2.5 mol of 2-furan methyl chloride was added. After thermally stirred at room temperature for 2 h, the reaction mixture was cooled to room temperature, dried under vacuum, and purified by column chromatography to give a target dye (**II₈a**).

The compound **II₈a** was characterized as follows: HRMS-ESI: m/z calcd M⁺ for C₃₄H₂₇N₂O₅S⁺, 575.1635; found, 575.1639.

4. The synthetic route of dye II₉a was shown as follows: 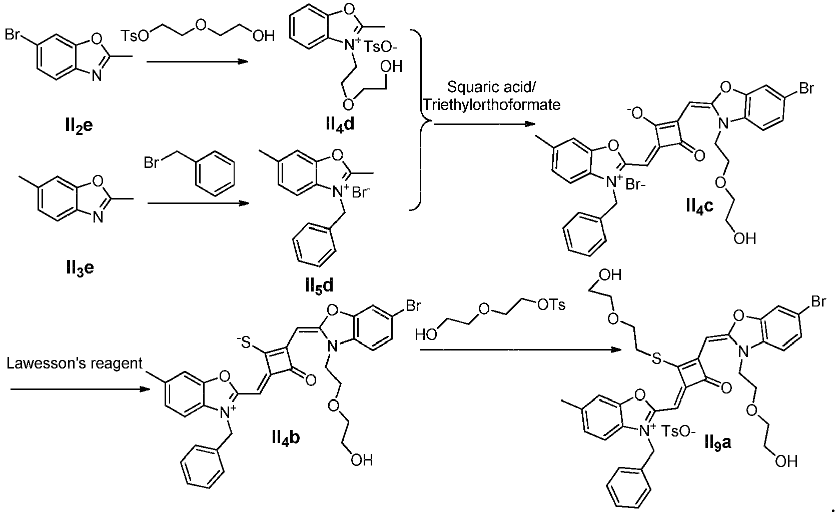
(1) 1 mol of 2-methyl-5-bromobenzoxazole and 3 mol of ethylene glycol tosylate were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (II₄d) which was dried and stored for use.
(2) 1 mol of 2-methyl-5-methylbenzoxazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₅d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (II₄d) and 1.2 mol of the quaternary ammonium salt (II₅d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (II₄c).
(4) 1 mol of the dye (II₄c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (II₄b).
(5) 1 mol of the sulfur-substituted squaraine dye (II₄b) was added to a dry acetonitrile solvent, to which 2.5 mol of 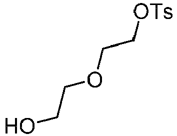 was added. The reaction mixture was thermally stirred at room temperature for 2 h.

After cooled to room temperature, the reaction mixture was dried under vacuum, and purified by column chromatography to give a target dye (**II₉a**).

The compound **II₉a** was characterized as follows:
¹H-NMR (400 MHz, CDCl₃): 2.35 (s, 3H, CH₃), 3.06 (t, 2H, CH₂, *J*= 8.0 Hz), 3.41 (m, 6H, CH₂), 3.58 (s, 2H, OH), 3.67 (m, 4H, CH₂), 4.06 (m, 4H, CH₂), 5.41 (s, 2H, CH₂), 5.93(s, 1H, CH), 6.15 (s, 1H, CH), 7.13 (m, 3H, ArH), 7.29 (m, 5H, ArH), 7.46 (m, 3H, ArH);
¹³C-NMR (100 MHz, CDCl₃): 21.61, 33.90, 44.38, 45.29, 60.31, 66.12, 69.40, 73.14, 84.33, 108.07, 116.80,122.11, 125.53, 131.07, 143.26, 146.82, 159.42, 174.30.
HRMS-ESI: *m*/*z* calcd M⁺ for C₃₆H₃₆BrN₂O₇S⁺, 719.1421; found, 719.1425.

5. The synthetic route of dye II₁₀a was shown as follows: 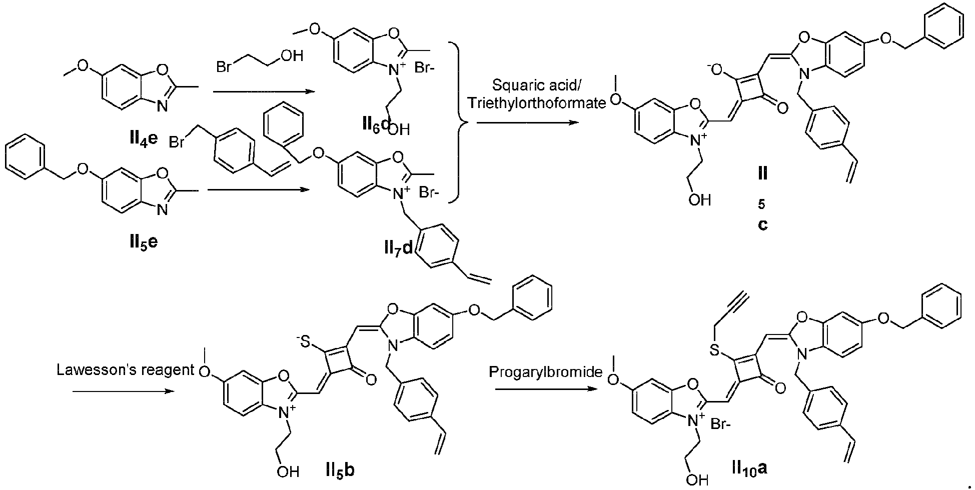
(1) 1 mol of 2-methyl-5-methoxybenzoxazole (II₄e) and 3 mol of p-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (II₆d) which was dried and stored for use.
(2) 1 mol of 2-methyl-5-benzyloxybenzoxazole (II₅e) and 3 mol of 4-vinylbenzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₇d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved. Then 0.8 mol of the quaternary ammonium salt (II₆d) and 1.2 mol of quaternary ammonium salt (II₇d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (II₅c).
(4) 1 mol of the dye (II₅c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (II₅b).
(5) 1 mol of the sulfur-substituted squaraine dye (II₅b) was added to a dry acetonitrile solvent, to which 2.5 mol of propargyl bromide was added. The reaction mixture was thermally stirred at room temperature for 2 h.

After cooled to room temperature, the reaction mixture was dried under vacuum, and purified by column chromatography to give a target dye (**II₁₀a**).

The compound **II₁₀a** was characterized as follows: HRMS-ESI: m/z calcd M⁺ for C₄₃H₃₇N₂O₅S⁺, 693.2418; found, 693.2423.

### Example 3

Listed herein were only partially representative structures, in the formula I, X=Y=S, based on which, structures of ten representative dyes respectively varying in R₁ to R₅ were shown as follows, wherein formulae III₁a, III₂a, III₃a, III₄a, III₅a, III₆a, III₇a, III₈a, III₉a, III₁₀a are non-inventive embodiments, not falling under the scope of the claims : 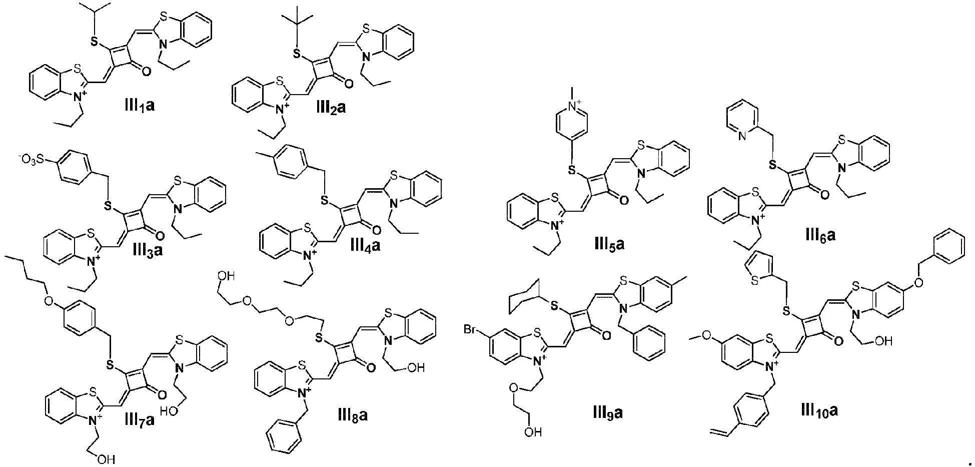
1. Synthetic routes of dyes III₁a to III₆a were shown as follows: 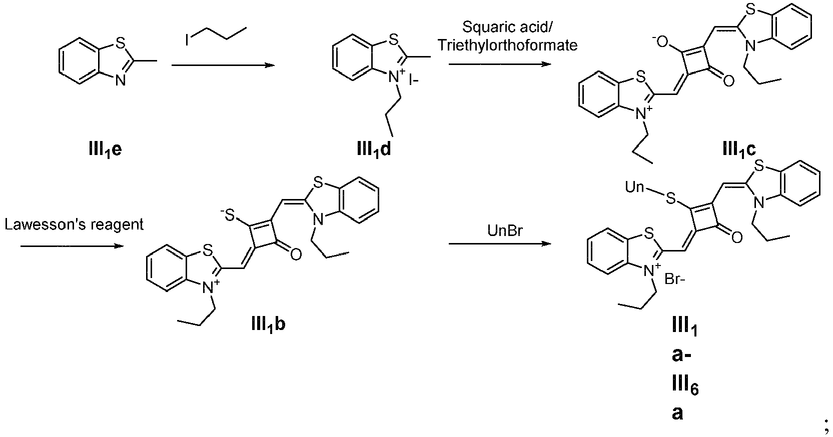 wherein, the structural formulas of Uₙ (n=1 to 6) were respectively shown as follows: 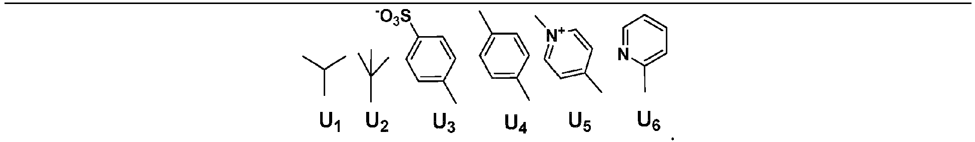
   (1) 1 mol of 2-methylbenzothiazole and 2 mol of iodopropane were added to 20 mL of dry 1,2-dichlorobenzene. The reaction mixture was heated under nitrogen protection for 5 h, and then cooled to room temperature. The reaction mixture was washed with anhydrous diethyl ether and recrystallized with propanone to give a white to pink quaternary ammonium salt solid (III₁d), which was dried and then stored for use.
   (2) 1 mol of squaric acid was added to 50 mL of absolute ethanol, to which triethyl orthoformate was added as a catalyst. The reaction mixture was heated and refluxed under nitrogen protection until the squaric acid was completely dissolved. Then 2.2 mol of the quaternary ammonium salt (III₁d) was added, and the reaction mixture was continuously heated and refluxed. After the reaction was confirmed to be completed by TLC, the reaction mixture was cooled, dried under reduced pressure to remove the solvent, and purified by column chromatography to give a blue solid dye (III₁c).
   (3) 1 mol of the dye (III₁c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (III₁b).
   (4) 1 mol of the sulfur-substituted squaraine dye (III₁b) was added to a dry acetonitrile solvent, to which a bromo-substituted intermediate UₙBr (Uₙ was selected from U₁ to U₆) was added. The reaction mixture was stirred at a temperature from room temperature to 60°C for different times, dried under vacuum, and purified by column chromatography to give a corresponding target dye (**III₁a to III₆a**).

The compounds **III₁a to III₆a** were characterized as follows:
**III₁a:** HRMS-ESI: m/z calcd M⁺ for C₂₉H₃₁N₂OS₃⁺, 519.1593; found, 519.1598.
**III₂a**: ¹H-NMR (400 MHz, CDCl₃): 1.04 (t, 6H, CH₃, J = 8.0 Hz), 1.39 (m, 4H, CH₂), 1.43 (s, 9H, CH₃), 4.30 (t, 4H, CH₂, *J*=8.0 Hz), 6.31 (s, 2H, CH), 7.17 (d, 2H, ArH, *J*=8.0 Hz), 7.39 (t, 2H, ArH, *J*=8.0 Hz), 7.58 (q, 4H, ArH, *J*=8.0 Hz);
¹³C-NMR (100 MHz, CDCl₃): 11.40, 22.71, 32.83, 46.02, 52.64, 89.50, 109.81, 122.47, 125.10, 127.65, 142.11, 144.36, 176.22, 185.04, 202.90.
HRMS-ESI: *m*/*z* calcd M⁺ for C₃₀H₃₃N₂OS₃⁺, 533.1750; found, 533.1754.
**III₃a:** HRMS-ESI: *m*/*z* calcd M⁺ for C₃₃H₃₀N₂O₄S₄, 646.1088; found, 647.1093.
**III₄a**:¹H-NMR (400 MHz, CDCl₃): 1.03 (t, 6H, CH₃, *J*=8.0 Hz), 1.37 (m, 4H, CH₂), 2.43 (s, 3H, CH₃), 4.27 (t, 4H, CH₂, *J*= 8.0 Hz), 4.59 (s, 2H, CH₂), 6.23 (s, 2H, CH), 7.10 (m, 3H, ArH), 7.17 (m, 3H, ArH), 7.39 (t, 2H, ArH, *J*=8.0 Hz), 7. 58 (q, 4H, ArH, *J*=8.0 Hz);
¹³C-NMR (100 MHz, CDCl₃): 10.79, 21.33, 21.71, 43.09, 53.10, 91.06, 108.98, 122.11, 124.83, 128.07, 138.10, 141.80, 143.01, 145.24, 177.35, 184.20, 203.81.
HRMS-ESI: *m*/*z* calcd M⁺ for C₃₄H₃₃N₂OS₃⁺, 581.1750; found, 581.1754.
**III₅a**: HRMS-ESI: *m*/*z* calcd [M⁺Cl]⁺ for C₃₂H₃₁ClN₃OS₃⁺, 604.1312; found, 604.1316.
**III₆a**: ¹H-NMR (400 MHz, CDCl₃): 1.01 (t, 6H, CH₃, *J*=8.0 Hz), 1.41 (m, 4H, CH₂), 4.31 (t, 4H, CH2, *J*=8.0 Hz), 5.16 (s, 2H, CH₂), 6.33 (s, 2H, CH), 7.13 (m, 5H, ArH), 7.38 (m, 4H, ArH), 7.61 (m, 3H, ArH);
¹³C-NMR (100 MHz, CDCl₃): 10.90, 22.70, 36.62, 54.04, 90.63, 109.30, 122.56, 123.90, 127.61, 138.40, 142.07, 144.12, 146.37, 178.51, 187.40.
HRMS-ESI: *m*/*z* calcd M⁺ for C₃₂H₃₀N₃OS₃⁺, 568.1546; found, 568.1551.

2. The synthetic route of dye III₇a was shown as follows: 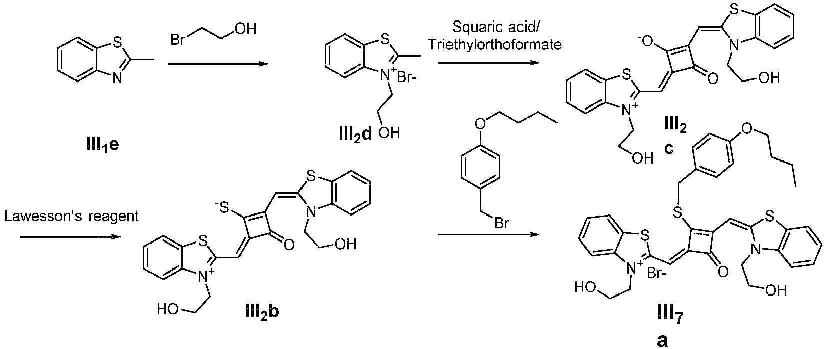
(1) 1 mol of 2-methylbenzothiazole and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (III₂d) which was dried and then stored for use.
(2) 1 mol of squaric acid was added to 50 mL of absolute ethanol, to which triethyl orthoformate was added as a catalyst. The reaction mixture was heated and refluxed under nitrogen protection until the squaric acid was completely dissolved, and then 2.2 mol of the quaternary ammonium salt (III₂d) was added. The reaction mixture was continuously heated and refluxed until the reaction was confirmed to be completed by TLC. The reaction mixture was cooled, dried under reduced pressure to remove the solvent, and purified by column chromatography to give a blue solid dye (III₂c).
(3) 1 mol of the dye (III₂c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (III₂b).
(4) 1 mol of the sulfur-substituted squaraine dye (III₂b) was added to a dry acetonitrile solvent, to which 2.5 mol of 4-butoxy benzyl bromide was added. The reaction mixture was stirred at 60°C for 2 h. After cooled to room temperature, the reaction mixture was dried under vacuum, and purified by column chromatography to give a target dye (**III₇a**).

The compound **III₇a** was characterized as follows:
HRMS-ESI: m/z calcd M⁺ for C₃₅H₃₅N₂O₄S₃⁺, 643.1753; found, 643.1757.

3. The synthetic route of dye III₈a was shown as follows: 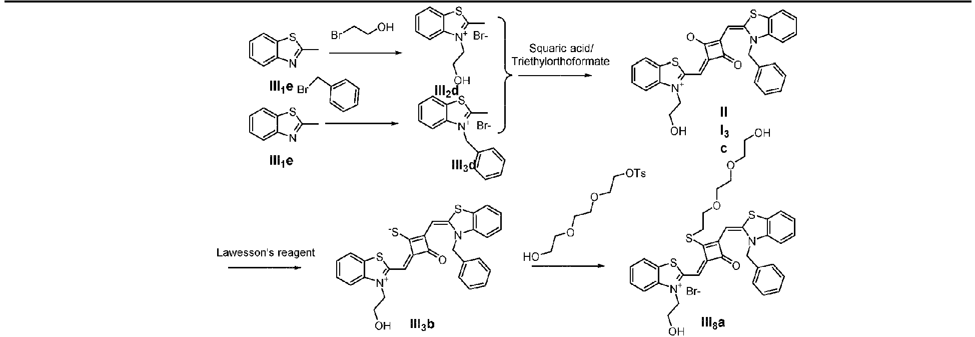
(1) 1mol of 2-methylbenzothiazole and 3 mol of bromoethanol were added to 20 mL of dry toluene. The reaction mixture was heated under nitrogen protection for 12 h and cooled to room temperature. Then the reaction mixture was washed with anhydrous diethyl ether and recrystallized with propanone to give a white to pink quaternary ammonium salt solid (III₂d), which was dried and stored for use.
(2) 1mol of 2-methylbenzothiazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (III₃d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (III₂d) and 1.2 mol of the quaternary ammonium salt (III₃d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (III₃c).
(4) 1 mol of the dye (III₃c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (III₃b).
(5) 1 mol of the sulfur-substituted squaraine dye (III₃b) was added to a dry acetonitrile solvent, to which 2.5 mol of triethylene glycol p-toluenesulfonate was added. After stirred at 50°C for 2 h, the reaction mixture was cooled to room temperature, dried under vacuum, and purified by column chromatography to give a target dye (**III₈a**).

The compound **III₈a** was shown as follows:
¹H-NMR (400 MHz, CDCl₃): 2.98 (t, 2H, CH₂, *J*=8.0 Hz), 3.58 (m, 12H, CH₂), 4.01 (t, 2H, CH₂, *J*=8.0 Hz), 5.42 (s, 2H, CH₂), 5.94(s, 1H, CH), 6.13 (s, 1H, CH), 7.11 (m, 4H, ArH), 7.32 (m, 4H, ArH), 7.48 (m, 2H, ArH), 7.59 (m, 3H, ArH);
¹³C-NMR (100 MHz, CDCl₃): 32.81, 54.55, 55.60, 61.31, 63.07, 69.73, 70.34, 73.48, 89.50, 109.13, 117.60, 122.74, 124.16, 126.53, 130.90, 143.11, 147.75, 158.30, 173.64.
HRMS-ESI: *m*/*z* calcd M⁺ for C₃₅H₃₅N₂O₅S₃⁺, 659.1703; found, 659.1709.

4. The synthetic route of dye III₉a was shown as follows: 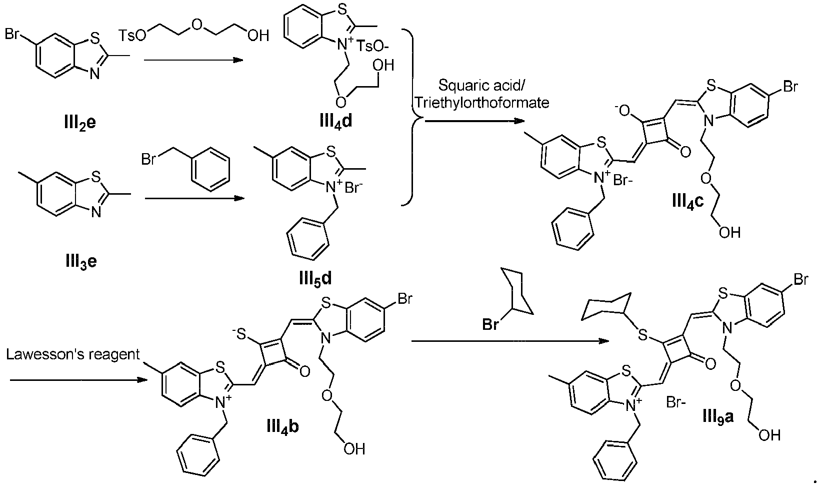
(1) 1 mol of 2-methyl-bromobenzothiazole and 3 mol of diethylene glycol tosylate were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (III₄d) which was dried and stored for use.
(2) 1 mol of 2-methyl-methylbenzothiazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink solid quaternary ammonium salt (III₅d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (III₄d) and 1.2 mol of the quaternary ammonium salt (III₅d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (III₄c).
(4) 1 mol of the dye (III₄c) was added to 25 mL of a mixed solvent of dry dichloromethane and tetrahydrofuran, to which 2 mol of Lawesson's reagent was added. The reaction mixture was stirred at 40°C for 3 h, dried under vacuum, and purified by column chromatography to give a bluish green centrally sulfur-substituted squaraine dye (III₄b).
(5) 1 mol of the sulfur-substituted squaraine dye (III₄b) was added to a dry acetonitrile solvent, to which 2.5 mol of cyclohexyl bromide was added. The reaction mixture was thermally stirred at room temperature for 2 h. After cooled to room temperature, the reaction mixture was dried under vacuum, and purified by column chromatography to give a target dye (**III₉a**).

The compound **III₉a** was characterized as follows:
HRMS-ESI: m/z calcd M⁺ for C₃₈H₃₈BrN₂O₃S₃⁺, 745.1222; found, 745.1225.

5. The synthetic route of dye III₁₀a was shown as follows: 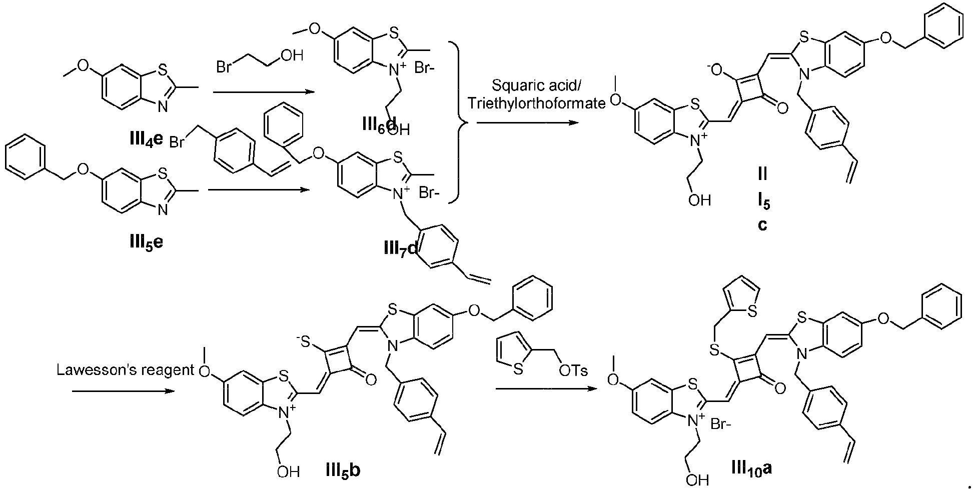
(1) 1 mol of 2-methyl-5-methoxybenzothiazole (III₄e) and 3 mol of p-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (III₆d) which was dried and stored for use.
(2) 2-methyl-5-benzyloxybenzothiazole and 3 mol of 4-vinylbenzyl bromide were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (III₇d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (III₆d) and 1.2 mol of quaternary ammonium salt (III₇d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (III₅c).
(4) 1 mol of the dye (III₅c) was added to 25 mL of a mixed solvent of dry dichloromethane and tetrahydrofuran to produce a solution, to which 2 mol of Lawesson's reagent was added. After stirred at 40°C for 3 h, the reaction mixture was dried under vacuum, and purified by column chromatography to give a bluish green centrally sulfur-substituted squaraine dye (III₅b).
(5) 1 mol of the sulfur-substituted squaraine dye (III₅b) was added to a dry acetonitrile solvent, to which 2.5 mol of alkyne 2-thienylmethyl *p*-toluenesulfonate was added. The reaction mixture was stirred at 35°C for 2 h. After cooled to room temperature, the reaction mixture was dried under vacuum, and purified by column chromatography to give a target dye (**III₁₀a**).

The compound **III₁₀a** was characterized as follows:
¹H-NMR (400 MHz, CDCl₃): 3.67 (s, 1H, OH), 3.83 (m, 5H, CH₂*&*CH₃), 4.01 (t, 2H, CH₂, *J*=8.0 Hz), 4.13 (s, 2H, CH₂), 5.18 (m, 3H, CH₂*&*=CH₂), 5.42 (s, 2H, CH₂), 5.66 (m, 1H, =CH₂), 5.97(s, 1H, CH), 6.16 (s, 1H, CH), 6.63 (m, 1H,=CH), 7.02 (m, 6H, ArH), 7.33 (m, 2H, ArH), 7.45 (m, 8H, ArH), 7.67 (m, 2H, ArH);
¹³C-NMR (100 MHz, CDCl₃): 31.30, 52.91, 54.82, 55.80, 63.15, 70.88, 90.61, 111.03, 114.32, 116.49, 118.34, 122.74, 124.16, 125.18, 126.53, 128.50, 130.90, 133.65, 143.11, 147.44, 158.30, 173.64.
HRMS-ESI: m/z calcd M⁺ for C₄₄H₃₇N₂O₄S₄⁺, 785.1631; found, 785.1635.

### Example 4

Listed herein were only partially representative structures, in formula I, X=Y=NCH₂CH₃, based on which, structures of ten representative dyes respectively varying in R₁ to R₅ were shown below, wherein formulae IV₁a, IV₂a, IV₃a, IV₄a, IV₅a, IV₆a, IV₇a, IV₈a, IV₉a, IV₁₀a are non-inventive embodiments, not falling under the scope of the claims: 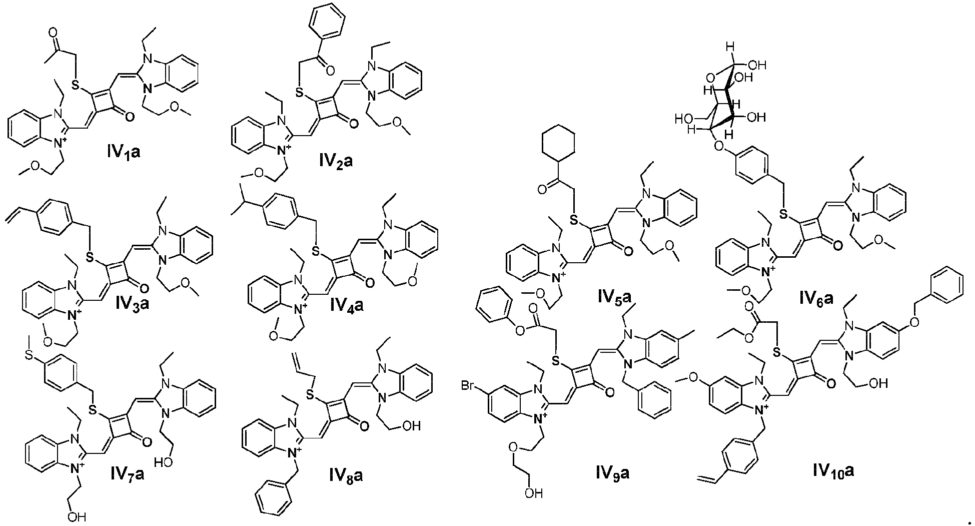
1. Synthetic routes of dyes IV₁a to IV₆a were shown as follows: 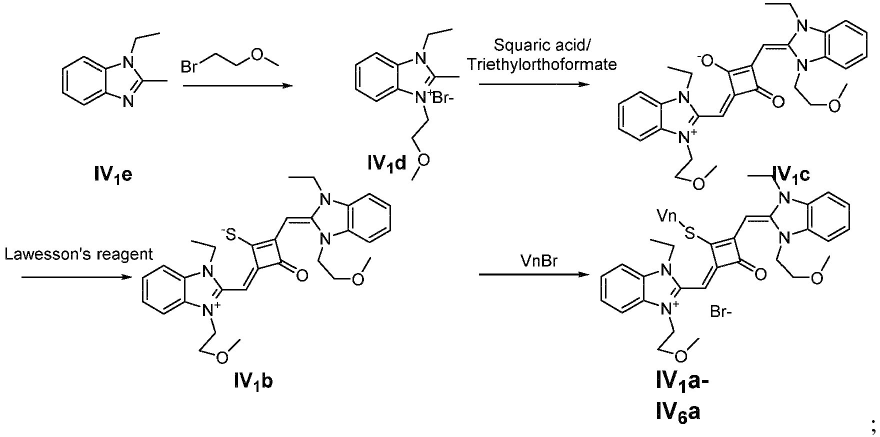 wherein, the structural formulas of Vₙ (n=1 to 6) were respectively shown as follows: 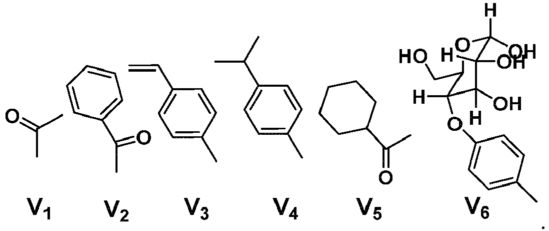
   (1) 1 mol of 2-methyl-3-N-ethylbenzimidazole and 2 mol of 1-bromo-2-methoxyethane were added to 20 mL of dry 1,2-dichlorobenzene. The reaction mixture was heated under nitrogen protection for 35 h. After cooled to room temperature, the reaction mixture was washed with anhydrous diethyl ether and recrystallized with propanone to give a white to pink quaternary ammonium salt solid (IV₁d), which was dried and then stored for use.
   (2) 1 mol of squaric acid was added to 50 mL of absolute ethanol, to which triethyl orthoformate was added as a catalyst. The reaction mixture was heated and refluxed under nitrogen protection until the squaric acid was completely dissolved, and then 2.2 mol of the quaternary ammonium salt prepared above was added. The reaction mixture was continuously heated and refluxed. After the reaction was confirmed to be completed by TLC, dried under reduced pressure to remove the solvent, and purified by column chromatography to give a blue solid dye (IV₁c).
   (3) 1 mol of the dye (IV₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IV₁b).
   (4) 1 mol of the sulfur-substituted squaraine dye (IV₁b) was added to a dry acetonitrile solvent, to which a bromo-substituted intermediate VₙBr (Vₙ was selected from V₁ to V₆) was added. The reaction mixture was stirred at a temperature for a certain period of time, dried under vacuum, and purified by column chromatography to give a corresponding target dye (**IV₁a to IV₆a**).

The compounds **IV₁a to IV₆a** were characterized as follows:
**IV₁a**: ¹H-NMR (400 MHz, CDCl₃): 1.30 (t, 6H, CH₃, *J*=8.0 Hz), 2.28 (s, 3H, CH₃), 3.29 (s, 6H, CH₃), 3.57 (t, 4H, CH₂, *J*=8.0 Hz), 4.01 (m, 6H, CH₂), 4.25 (t, 4H, CH₂, *J*=8.0 Hz), 6.15(s, 2H, CH), 7.11 (d, 2H, ArH, *J*=8.0 Hz), 7.20 (t, 2H, ArH, *J*=8.0 Hz), 7.38 (q, 4H, ArH, *J*=8.0 Hz);
¹³C-NMR (100 MHz, CDCl₃):14.71, 26.80, 44.13, 44.85, 50.35, 59.02, 69.25, 89.87, 110.08, 114.53, 123.16, 124.57, 128.21, 144.24, 158.10, 172.87, 203.65.
HRMS-ESI: *m*/*z* calcd M⁺ for C₃₃H₃₉N₄O₄S⁺, 587.2687; found, 587.2691.
**IV₂a**: HRMS-ESI: *m*/*z* calcd M⁺ for C₃₈H₄₁N₄O₄S⁺, 649.2843; found, 649.2848.
**IV₃a**: ¹H-NMR (400 MHz, CDCl₃): 1.29 (t, 6H, CH₃, *J*=8.0 Hz), 3.26 (s, 6H, CH₃), 3.55 (t, 4H, CH₂, J= 8.0 Hz), 3.98 (t, 4H, CH₂, *J*=8.0 Hz), 4.25 (q, 4H, CH₂, *J*=8.0 Hz), 4.59 (s, 2H, CH₂), 5.15 (m, 1H, =CH₂), 5.58 (m, 1H,=CH₂), 6.34(s, 2H, CH), 6.60 (m, 1H,=CH), 7.13 (m, 4H, ArH), 7.24 (m, 3H, ArH), 7.44 (m, 5H, ArH);
¹³C-NMR (100 MHz, CDCl₃): 14.33, 14.87, 42.10, 44.61, 51.11, 59.80, 70.36, 90.54, 110.17, 114.42, 117.49, 123.16, 124.57, 128.21, 129.32, 144.24, 157.12, 171.98.
HRMS-ESI: *m*/*z* calcd M⁺ for C₃₉H₄₃N₄O₃S⁺, 647.3050; found, 647.3053.
**IV₄a**: HRMS-ESI: *m*/*z* calcd M⁺ for C₄₀H₄₇N₄O₃S⁺, 663.3363; found, 647.3067.
**IV₅a**: ¹H-NMR (400 MHz, CDCl₃): 1.27 (t, 6H, CH₃, *J*=8.0 Hz), 1.53 (m, 8H, CH₂), 1.80 (m, 2H, CH₂), 2.35 (m, 1H, CH), 3.26 (s, 6H, CH₃), 3.58 (t, 4H, CH₂, *J*=8.0 Hz), 3.99 (t, 4H, CH₂, J=8.0 Hz), 4.05 (s, 2H, CH₂), 4.28 (q, 4H, CH₂, *J*=8.0 Hz), 6.31(s, 2H, CH), 7.13 (d, 2H, ArH, *J*=8.0 Hz), 7.25 (m, 2H, ArH), 7.41 (q, 4H, ArH, *J*=8.0 Hz);
¹³C-NMR (100 MHz, CDCl₃): 14.75, 25.35, 28.13, 39.70, 44.89, 50.97, 60.01, 70.25, 91.54, 109.73, 116.49, 122.80, 127.45, 129.52, 145.69, 157.63, 173.40.
HRMS-ESI: *m*/*z* calcd M⁺ for C₃₈H₄₇N₄O₄S⁺, 655.3313; found, 655.3315.
**IV₆a**: HRMS-ESI: *m*/*z* calcd M⁺ for C₄₃H₅₁N₄O₉S⁺, 799.3371; found, 799.3375.

2. The synthetic routes of dye IV₇a was shown as follows: 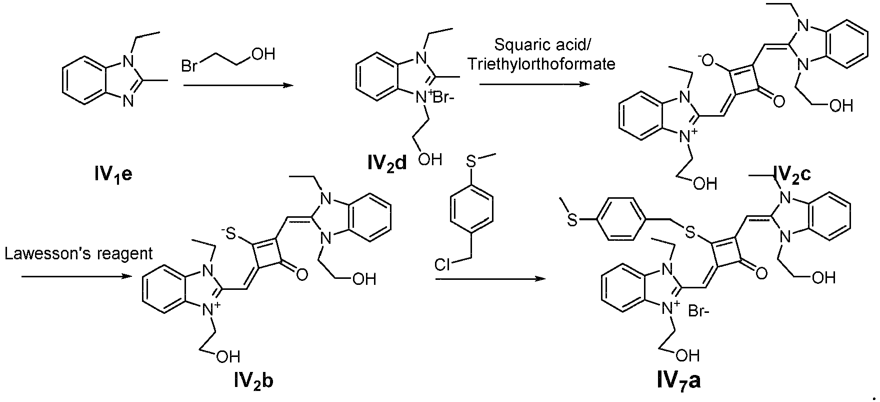
(1) 1mol of 2-methyl-3-N-ethylbenzimidazole and 3 mol of bromoethanol were added to 20 mL of dry toluene. The reaction mixture was heated under nitrogen protection for 12 h. After cooled to room temperature, the reaction mixture was washed with anhydrous diethyl ether and recrystallized with propanone to give a white to pink quaternary ammonium salt solid (IV₂d), which was dried and then stored for use.
(2) 1 mol of squaric acid was added to 50 mL of absolute ethanol, to which triethyl orthoformate was added as a catalyst. The reaction mixture was heated and refluxed under nitrogen protection until the squaric acid was completely dissolved, and then 2.2 mol of the quaternary ammonium salt (IV₂d) prepared above was added. The reaction mixture was continuously heated and refluxed until the reaction was confirmed to be completed by TLC. The reaction mixture was cooled, dried under reduced pressure to remove the solvent, and purified by column chromatography to give a blue solid dye (IV₂c).
(3) 1 mol of the dye (IV₂c) was added to 25 mL of a mixed solvent of dry dichloromethane and tetrahydrofuran to, to which 2 mol of Lawesson's reagent was added. After stirred at 40°C for 3 h, the reaction mixture was dried under vacuum, and purified by column chromatography to give a bluish green centrally sulfur-substituted squaraine dye (IV₂b).
(4) 1 mol of the sulfur-substituted squaraine dye (IV₂b) was added to a dry acetonitrile solvent, to which 2.5 mol of 4-(methylthio)benzyl chloride was added. The reaction mixture was thermally stirred at room temperature for 2 h. After cooled to room temperature, the reaction mixture was dried under vacuum, and purified by column chromatography to give a target dye (**IV₇a**).

The compound **IV₇a** was characterized as follows:
¹H-NMR (400 MHz, CDCl₃): 1.26 (t, 6H, CH₃, *J*=8.0 Hz), 2.53 (s, 3H, CH₃), 3.66 (t, 4H, CH₂, *J*=8.0 Hz), 4.03(t, 4H, CH₂, *J*=8.0 Hz), 4.25(q, 4H, CH₂, *J*=8.0 Hz), 4.69 (s, 2H, CH₂), 6.29(s, 2H, CH), 7.12 (m, 6H, ArH), 7.27 (m, 3H, ArH), 7.47 (m, 3H, ArH);
¹³C-NMR (100 MHz, CDCl₃): 13.79, 42.04, 43.99, 51.58, 63.74, 92.32, 108.45, 117.18, 119.30, 123.06, 127.09, 129.74, 137.70, 146.54, 158.65, 174.04.
HRMS-ESI: m/z calcd M⁺ for C₃₆H₃₉N₄O₃S₂⁺, 639.2458; found, 639.2461.

3. The synthetic route of dye IV₈a was shown as follows: 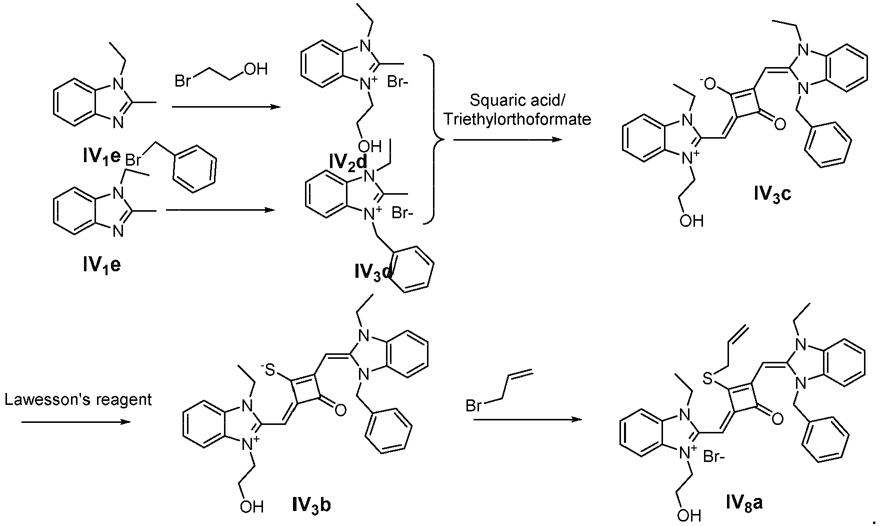
(1) 1mol of 2-methyl-3-N-ethylbenzimidazole and 3 mol of bromoethanol were added to 20 mL of dry toluene. The reaction mixture was heated under nitrogen protection for 12 h. After cooled to room temperature, the reaction mixture was washed with anhydrous diethyl ether and recrystallized with propanone to give a white to pink quaternary ammonium salt solid (IV₂d), which was dried and stored for use.
(2) 1 mol of 2-methyl-3-N-ethylbenzimidazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (IV₃d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (IV₂d) and 1.2 mol of the quaternary ammonium salt (IV₃d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (IV₃c).
(4) 1 mol of the dye (IV₃c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IV₃b).
(5) 1 mol of the sulfur-substituted squaraine dye (IV₃b) was added to a dry acetonitrile solvent, to which 4 mol of allyl bromide was added. The reaction mixture was thermally stirred at room temperature for 2 h. After cooled to room temperature, the reaction mixture was dried under vacuum, and purified by column chromatography to give a target dye (**IV₈a**).

The compound **IV₈a** was characterized as follows:
HRMS-ESI: m/z calcd M⁺ for C₃₆H₃₇N₄O₂S⁺, 589.2632; found, 589.2638.

4. The synthetic route of dye IV₉a was shown as follows: 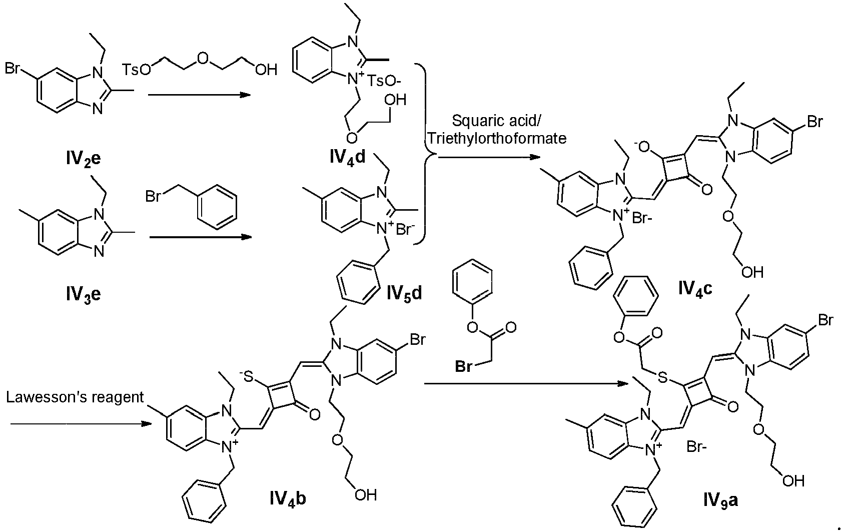
(1) 1 mol of 2-methyl-3-N-ethyl-5-bromobenzimidazole and 3 mol of diethylene glycol tosylate were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (IV₄d), which was dried and stored for use.
(2) 1 mol of 2-methyl-3-N-ethyl-5-methylbenzimidazole and 3 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (IV₅d), which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (IV₄d) and 1.2 mol of the quaternary ammonium salt (IV₅d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (IV₄c).
(4) 1 mol of the dye (IV₄c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IV₄b).
(5) 1 mol of the sulfur-substituted squaraine dye (IV₄b) was added to a dry acetonitrile solvent, to which 2 mol of phenyl 2-bromoacetate was added. After stirred at 40°C for 2 h, the reaction mixture was cooled to room temperature, dried under vacuum, and purified by column chromatography to give a target dye (**IV₉a**).

The compound **IV₉a** was characterized as follows:
¹H-NMR (400 MHz, CDCl₃): 1.28 (m, 6H, CH₃), 2.49 (s, 3H, CH₃), 3.56 (m, 6H, CH₂), 3.98(s, 2H, CH₂), 4.27(m, 6H, CH₂), 4.89 (s, 2H, CH₂), 5.95 (s, 1H, CH), 6.14 (s, 1H, CH), 7.09 (m, 3H, ArH), 7.22 (m, 7H, ArH), 7.36 (m, 4H, ArH), 7.48 (m, 2H, ArH);
¹³C-NMR (100 MHz, CDCl₃): 14.08, 21.93, 36.74, 42.16, 47.51, 53.90, 57.15, 61.78, 69.23, 88.71, 95.44, 107.80,110.72, 114.63, 117.15, 119.03, 121.52, 123.04, 127.71, 129.94, 137.83, 146.40, 158.65, 173.11.
HRMS-ESI: *m*/*z* calcd M⁺ for C₄₄H₄₄BrN₄O₅S⁺, 819.2210; found, 819.2214.

5. The synthetic route of dye IV₁₀a was shown as follows: 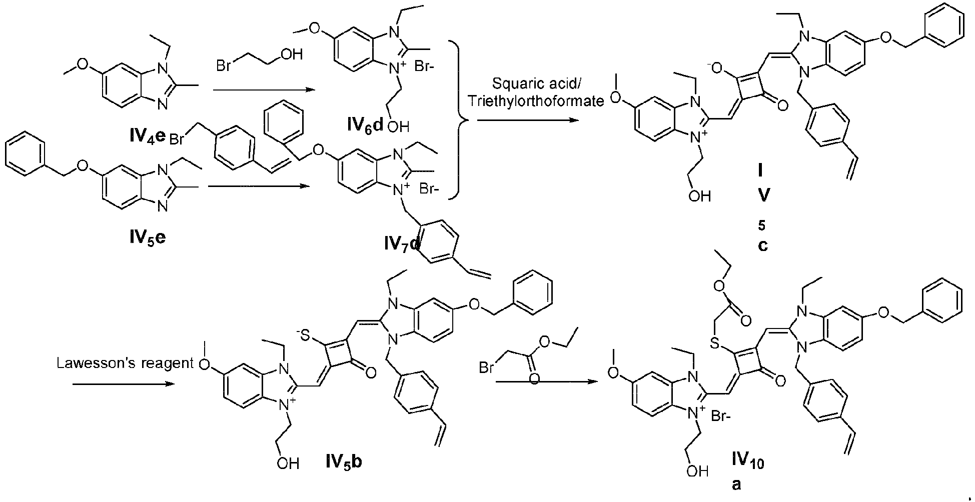
(1) 1 mol of 2-methyl-3-N-ethyl-5-methoxybenzimidazole (IV₄e) and 3 mol of p-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (IV₆d) which was dried and stored for use.
(2) 1 mol of 2-methyl-3-N-ethyl-5-benzyloxybenzimidazole (IV₅e) and 3 mol of 4-vinylbenzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (IV₇d) which was dried and stored for use.
(3) 1 mol of squaric acid was added to 100 mL of absolute ethanol, to which triethyl orthoformate was added as a catalyst. The reaction mixture was heated and refluxed under nitrogen protection until the squaric acid was completely dissolved, and then 0.8 mol of the quaternary ammonium salt (IV₆d) and 1.2 mol of quaternary ammonium salt (IV₇d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (IV₅c).
(4) 1 mol of the dye (IV₅c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IV₅b).
(5) 1 mol of the sulfur-substituted squaraine dye (IV₅b) was added to a dry acetonitrile solvent, to which 2.5 mol of ethyl bromoacetate was added. The reaction mixture was stirred at 40°C for 2 h. After cooled to room temperature, the reaction mixture was dried under vacuum, and purified by column chromatography to give a target dye (**IV₁₀a**).

The compound **IV₁₀a** was characterized as follows:
HRMS-ESI: m/z calcd M⁺ for C₄₇H₄₉N₄O₆S⁺, 797.3367; found, 797.3371.

### Example 5

The asymmetric dyes were further respectively substituted with different groups at the middle position to obtain asymmetric target dyes (V₁a to V₁₀a), which were shown as follows with X=O and Y= C(CH₃)₂ in formula I, wherein formulae V₁a, V₂a, V₃a, V₄a, V₅a, V₆a, V₇a, V₈a, V₉a, V₁₀a are non-inventive embodiments, not falling under the scope of the claims: 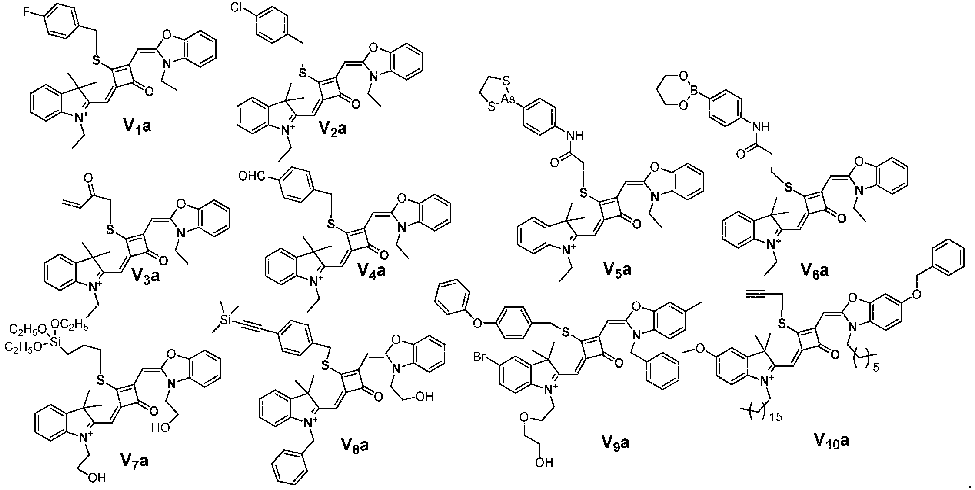
1. The synthetic routes of dyes V₁a to V₆a were shown as follows: 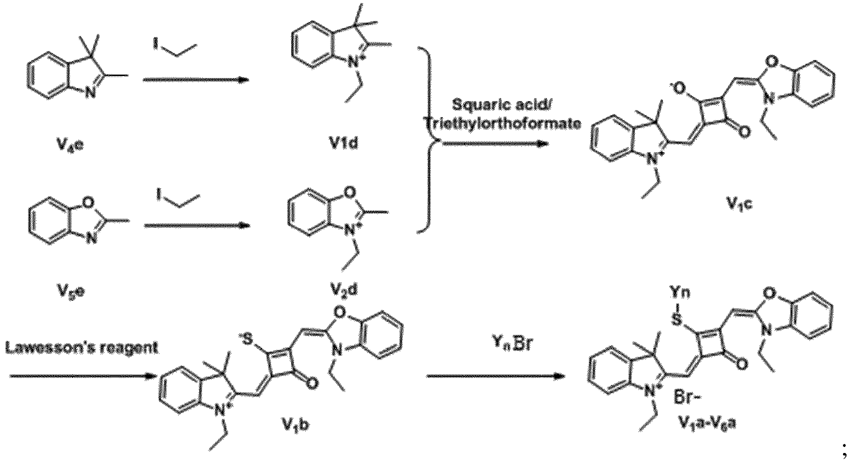 wherein, the structural formulas of Yₙ (n=1 to 6) were respectively shown as follows: 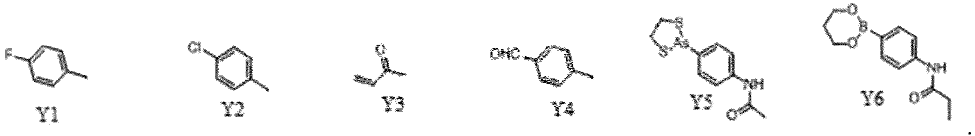
   (1) 1 mol of 2,3,3-trimethyl-3H-indoline (V₄e) and 3 mol of iodoethane were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₁d), which was dried and then stored for use.
   (2) 1 mol of 2-methylbenzoxazole (Vse) and 3 mol of iodoethane were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (V₁d), which was dried and stored for use.
   (3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₁d) and 1 mol of the quaternary ammonium salt (V₁d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (V₁c).
   (4) 1 mol of the dye (V₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (Vib).
   (5) 1 mol of the sulfur-substituted squaraine dye (Vsb) was added to a dry acetonitrile solvent, to which 2.5 mol of a bromo-substituted intermediate YₙBr (Yₙ was selected from Y₁ to Y₆) was added. The reaction mixture was stirred at room temperature or under heating for a certain period of time until the reaction was confirmed to be completed by TLC. Then the reaction mixture was cooled to room temperature, dried under vacuum, and purified by column chromatography to give a corresponding target dye (**V₁a to V₆a**).

The compounds **V₁a to V₆a** were characterized as follows:
**V₁a:** ¹H-NMR (400 MHz, CDCl₃): 1.30 (m, 6H, CH₃), 1.59 (s, 6H, CH₃), 4.36 (m, 4H, CH₂), 4.79(s, 2H, CH₂), 5.98 (s, 1H, CH), 6.22(s, 1H, CH), 7.08 (m, 2H, ArH), 7.23 (m, 4H, ArH), 7.37 (m, 3H, ArH), 7.59 (m, 3H, ArH);
¹³C-NMR (100 MHz, CDCl₃): 13.81, 14.45, 24.74, 42.20, 46.51, 55.09, 89.63, 96.44, 108.70, 111.52, 115.01, 117.85, 120.03, 121.48, 124.90, 127.71, 130.94, 137.13, 141.65, 152.97, 158.72, 173.10, 187.23.
HRMS-ESI: m/z calcd M⁺ for C₃₄H₃₂FN₂O₂S⁺, 819.2210; found, 819.2215.
**V₂a**: HRMS-ESI: m/z calcd M⁺ for C₃₄H₃₂ClN₂O₂S⁺, 567.1868; found, 567.1871.
**V₃a**: ¹H-NMR (400 MHz, CDCl₃): 1.33 (m, 6H, CH₃), 1.57 (s, 6H, CH₃), 4.35 (m, 4H, CH₂), 4.72(s, 2H, CH₂), 5.91(m, 1H,=CH₂), 6.01 (s, 1H, CH), 6.19(m, 1H, =CH₂), 6.23(s, 1H, CH), 6.28(m, 1H,=CH), 7.09 (m, 3H, ArH), 7.23 (m, 3H, ArH), 7.49 (m, 2H, ArH);
¹³C-NMR (100 MHz, CDCl₃): 12.79, 13.88, 25.10, 45.63, 51.90, 55.61, 91.44, 98.28, 109.15, 112.42, 115.11, 118.60, 120.56, 122.41, 126.03, 131.12, 135.84, 142.80, 152.52, 155.90, 174.22.
HRMS-ESI: *m*/*z* calcd M⁺ for C₃₁H₃₁N₂O₃S⁺, 511.2050; found, 511.2054.
**V₄a:** HRMS-ESI: *m*/*z* calcd M⁺ for C₃₅H₃₃N₂O₃S⁺, 561.2206; found, 561.2211.
**V₅a**: ¹H-NMR (400 MHz, CDCl₃): 1.30 (m, 6H, CH₃), 1.58 (s, 6H, CH₃), 3.22 (q, 4H, CH₂, *J*=8.0 Hz), 4.15 (m, 6H, CH₂), 5.99 (s, 1H, CH), 6.18(s, 1H, CH), 7.15 (m, 3H, ArH), 7.28 (m, 6H, ArH), 7.48 (m, 3H, ArH), 8.89 (s, 1H, NH);
¹³C-NMR (100 MHz, CDCl₃): 13.03, 14.17, 24.90, 27.87, 34.75, 45.60, 56.51, 90.24, 99.05, 109.10, 112.89, 114.03, 117.50, 109.84, 121.40, 127.31, 130.48, 134.80, 143.77, 150.92, 156.10, 172.62.
HRMS-ESI: *m*/*z* calcd M⁺ for C₃₇H₃₇AsN₃O₃S₃⁺, 742.1208; found, 742.1213.
**V₆a**: HRMS-ESI: *m*/*z* calcd M⁺ for C₃₉H₄₁BN₃O₅S⁺, 674.2854; found, 674.2858.

2. The synthetic route of dye V₇a was shown as follows: 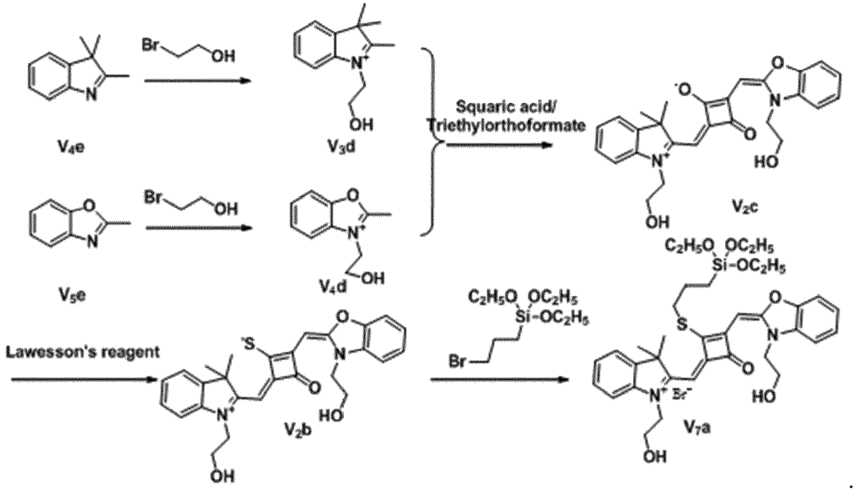
(1) 1mol of 2,3,3-trimethyl-3H-indoline (V₄e) and 3 mol of 2-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₃d), which was dried and stored for use.
(2) 1 mol of 2-methylbenzoxazole (V₅e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (V₄d), which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₂d) and 1 mol of the quaternary ammonium salt (V₂d) were added. The reaction mixture was continuously heated and refluxed until the reaction was confirmed to be completed by TLC. The reaction mixture was cooled, dried under reduced pressure to remove the solvent, and purified by column chromatography to give a blue solid dye (V₂c).
(4) 1 mol of the dye (V₂c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (V₂b).
(5) 1 mol of the sulfur-substituted squaraine dye (V₂b) was added to a dry acetonitrile solvent, to which 2.5 mol of a 3-bromopropyl triethoxysilane intermediate was added. The mixture was stirred under heating for a certain period of time until the reaction was confirmed to be completed by TLC. Then the reaction mixture was cooled to room temperature, dried under vacuum, and purified by column chromatography to give a target dye (**V₇a**).

The compound **V₇a** was characterized as follows:
¹H-NMR (400 MHz, CDCl₃): 0.48 (t, 2H, CH₂, *J*=8.0 Hz), 1.20 (t, 9H, CH₃, *J*=8.0 Hz), 1.51(s, 6H, CH₃), 2.10(m, 2H, CH₂), 3.24 (q, 2H, CH₂, *J*=8.0 Hz), 3.68 (m, 6H, CH₂), 3.88 (m, 8H, CH₂), 6.03 (s, 1H, CH), 6.21(s, 1H, CH), 7.12 (m, 3H, ArH), 7.29 (m, 3H, ArH), 7.46 (m, 2H, ArH);
¹³C-NMR (100 MHz, CDCl₃): 15.70, 17.38, 18.49, 23.35, 35.80, 45.22, 55.07, 58.40, 62.65, 91.53, 99.70, 110.01, 112.60, 114.03, 115.94, 118.20, 121.35, 123.09, 127.40, 130.25, 136.50, 143.91, 149.73, 154.10, 173.30.
HRMS-ESI: *m*/*z* calcd M⁺ for C₃₆H₄₇N₂O₇SSi⁺, 679.2868; found, 679.2872.

3. The synthetic route of dye V₈a was shown as follows: 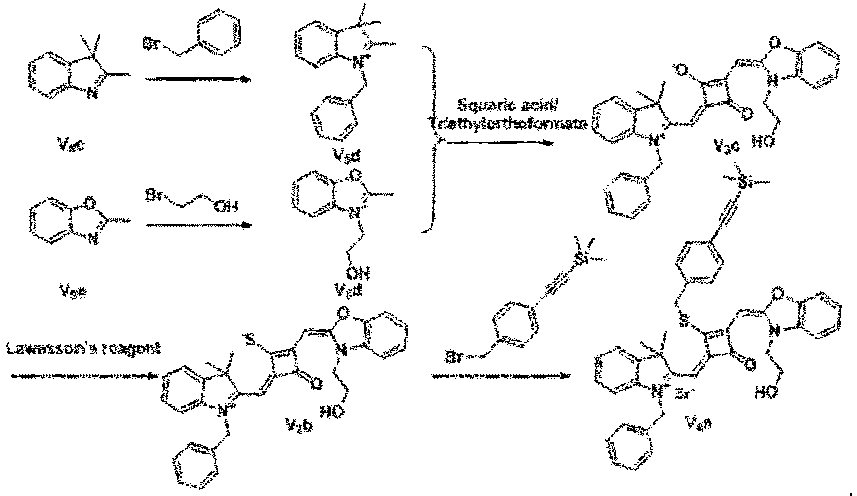
(1) 1mol of 2,3,3-trimethyl-3H-indoline (V₄e) and 3 mol of benzyl bromide were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (Vsd), which was dried and stored for use.
(2) 1 mol of 2-methylbenzoxazole (V₅e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (Vsd), which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₅d) and 1 mol of the quaternary ammonium salt (V₆d) were added. The reaction mixture was continuously heated and refluxed until the reaction was confirmed to be completed by TLC. Then the reaction mixture was cooled, dried under reduced pressure to remove the solvent, and purified by column chromatography to give a blue solid dye (V₃c).
(4) 1 mol of the dye (V₃c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (V₃b).
(5) 1 mol of the sulfur-substituted squaraine dye (V₃b) was added to a dry acetonitrile solvent, to which 2.5 mol of a 4-(2-trimethylsilyl ethynyl benzyl bromide) intermediate was added. The mixture was stirred under heating for a certain period of time until the reaction was confirmed to be completed by TLC. Then the reaction mixture was cooled to room temperature, dried under vacuum, and purified by column chromatography to give a target dye (**V₈a**).

The compound **V₈a** was characterized as follows:
HRMS-ESI: *m*/*z* calcd M⁺ for C₄₄H₄₃N₂O₃SSi⁺, 707.2758; found, 707.2763.

4. The synthetic route of dye V₉a was shown as follows: 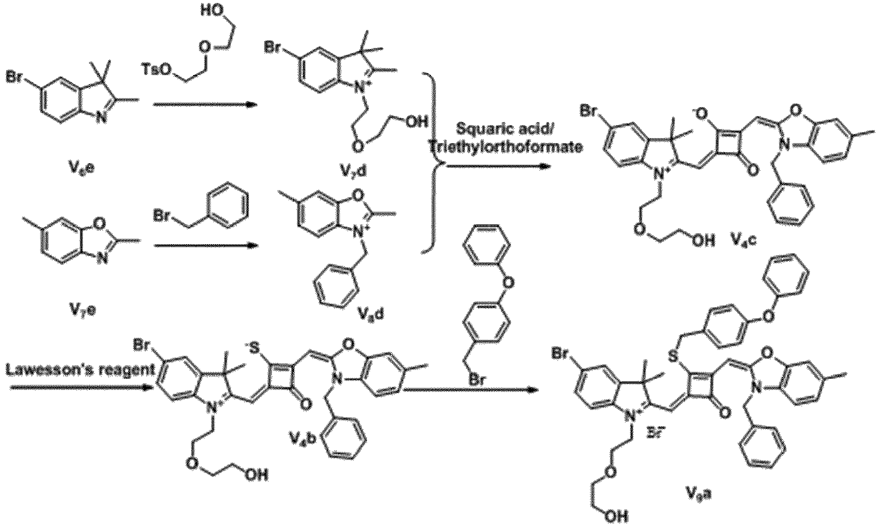
(1) 1 mol of 2,3,3-trimethyl-5-bromo-3H-indoline (V₆e) and 3 mol of ethanol, 2-(2-hydroxyethoxy)-, 1-(4-Methylbenzenesulfonate) were added to 20 mL of dry 1,2-dichlorobenzene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₇d) which was dried and stored for use.
(2) 1 mol of 2-methyl-5-methylbenzoxazole (V₇e) and 3 mol of benzyl bromide were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (V₈d) which was dried and stored use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₇d) and 1 mol of the quaternary ammonium salt (V₈d) were added. The reaction mixture was continuously heated and refluxed until the reaction was confirmed to be completed by TLC. Then the reaction mixture was cooled, dried under reduced pressure to remove the solvent, and purified by column chromatography to give a blue solid dye (V₄c).
(4) 1 mol of the dye (V₄c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (V₄b).
(5) 1 mol of the sulfur-substituted squaraine dye (V₄b) was added to a dry acetonitrile solvent, to which 2.5 mol of a 4-phenoxybenzyl bromide intermediate was added. The reaction mixture was stirred under heating for a certain period of time until the reaction was confirmed to be completed by TLC. Then the reaction mixture was cooled to room temperature, dried under vacuum, and purified by column chromatography to a give target dye (**V₉a**).

The compound **V₉a** was characterized as follows:
¹H-NMR (400 MHz, CDCl₃): 1.50(s, 6H, CH₃), 2.38(s, 3H, CH₃), 3.59 (m, 6H, CH₂), 4.15(t, 2H, CH₂, *J*=8.0 Hz), 4.63 (s, 2H, CH₂), 4.88 (s, 2H, CH₂), 6.04 (s, 1H, CH), 6.28(s, 1H, CH), 7.07 (m, 4H, ArH), 7.21 (m, 8H, ArH), 7.36 (m, 4H, ArH), 7.51 (m, 3H, ArH), 7.79 (m, 1H, ArH);
¹³C-NMR (100 MHz, CDCl₃): 21.17, 25.35, 42.80, 52.42, 55.91, 61.33, 66.80, 70.43, 89.58, 98.04, 110.25, 111.82, 112.51, 114.26, 115.75, 117.60, 119.31, 121.52, 123.14, 124.26, 127.30, 130.65, 132.81, 136.70, 145.22, 149.43, 154.49, 174.20.
HRMS-ESI: *m*/*z* calcd M⁺ for C₄₈H₄₄BrN₂O₅S⁺, 839.2149; found, 839.2153.

5. The synthetic route of dye V₁₀a was shown as follows: 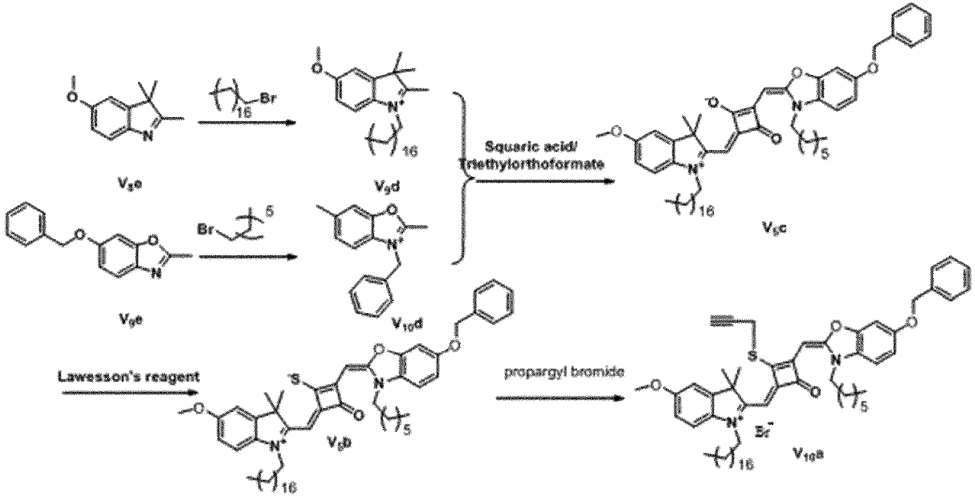
(1) 1 mol of 2,3,3-trimethyl-5-methoxy-3H-indoline (V₈e) and 2 mol of octadecyl bromide were added to 20 mL of dry 1,2-dichlorobenzene, to which potassium iodide was added as a catalyst. Then the reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₉d) which was dried and stored for use.
(2) 1 mol of 2-methyl-5-benzyloxybenzoxazole (V₉e) and 3 mol of bromoheptane were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (V₁₀d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (Vgd) and 1 mol of quaternary ammonium salt (V₁₀d) were added. The reaction mixture was continuously heated and refluxed until the reaction was confirmed to be completed by TLC. Then the reaction mixture was cooled, dried under reduced pressure to remove the solvent, and purified by column chromatography to give a blue solid dye (V₅c).
(4) 1 mol of the dye (V₅c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (V₅b).
(5) 1 mol of the sulfur-substituted squaraine dye (V₅b) was added to a dry acetonitrile solvent, to which 2.5 mol of a propargyl bromide intermediate was added. The reaction mixture was stirred under heating for a certain period of time until the reaction was confirmed to be completed by TLC. Then the reaction mixture was cooled to room temperature, dried under vacuum, and purified by column chromatography to give a target dye (**V₁₀a**).

The compound **V₁₀a** was characterized as follows:
HRMS-ESI: *m*/*z* calcd M⁺ for C₅₈H₇₇N₂O₄S⁺, 897.5599; found, 897.5603.

### Example 6

The asymmetric dyes were further respectively substituted with different groups at the middle position to obtain asymmetric target dyes (**VI₁a to VI₁₀a**) which were shown as follows with X= S and Y= C(CH₃)₂ in formula (I), wherein formulae VI₁a, VI₂a, VI₃a, VI₄a, VI₅a, VI₆a, VI₇a, VI₈a, VI₉a, VI₁₀a are non-inventive embodiments, not falling under the scope of the claims: 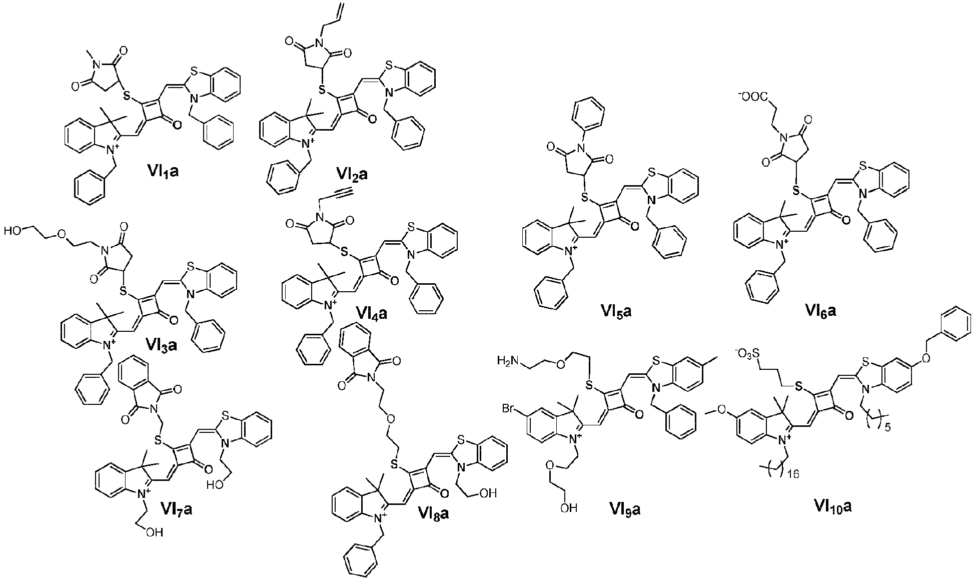
1. Synthetic routes of dyes VI₁a to VI₆a were shown as follows: 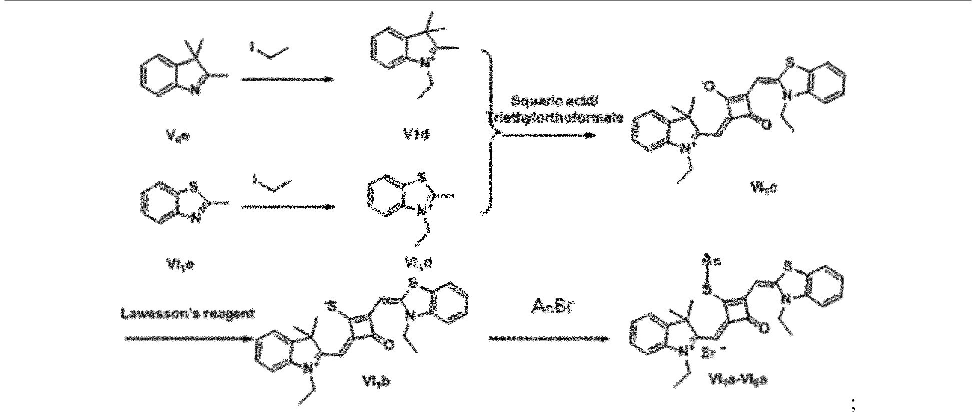 wherein, the structural formulas of Aₙ (n=1 to 6) were respectively shown as follows:

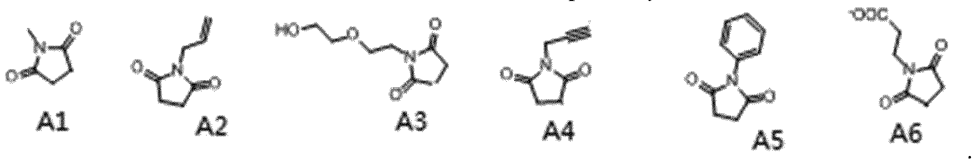

The dyes VI₁a-VI₆a were specifically prepared as follows.
(1) 1 mol of 2,3,3-trimethyl-3H-indoline (V₄e), 3 mol of benzyl bromide and 0.1 mol of potassium iodide were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₁d) which was dried and stored for use.
(2) 1mol of 2-methylbenzothiazole (VI₁e), 3 mol of benzyl bromide and 0.1 mol of potassium iodide were added to 20 mL of dry toluene. The reaction mixture was heated under nitrogen protection for 48 h. After cooled to room temperature, the reaction mixture was washed with anhydrous diethyl ether and recrystallized with propanone to give a white to pink quaternary ammonium salt solid (VI₁d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₁d) and 1 mol of the quaternary ammonium salt (VI₁d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VI₁c).
(4) 1 mol of the dye (VI₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VI₁b).
(5) 1 mol of the sulfur-substituted squaraine dye (VI₁b) was added to dry acetonitrile, to which 2.5 mol of a bromo-substituted intermediate AₙBr (Aₙ was selected from A₁ to A₆) was added. The reaction mixture was stirred at room temperature or under heating for a certain period of time until the reaction was confirmed to be completed by TLC. Then the reaction mixture was cooled to room temperature, dried under vacuum, and purified by column chromatography to give a corresponding target dye (**VI₁a to VI₆a**).

The compounds **VI₁a to VI₆a** were characterized as follows:
**VI₁a**: ¹H-NMR (400 MHz, CDCl₃): 1.48(s, 6H, CH₃), 3.49(m, 2H, CH₂), 3.76 (s, 6H, CH₂, CH&CH₃), 5.42 (s, 2H, CH₂), 6.06 (s, 1H, CH), 6.31(s, 1H, CH), 7.11 (m, 4H, ArH), 7.28 (m, 9H, ArH), 7.49 (m, 5H, ArH).
¹³C-NMR (100 MHz, CDCl₃): 24.90, 33.41, 41.34, 52.26, 55.05, 58.13, 90.06, 99.53, 108.70, 111.42, 113.95, 116.07, 117.94, 120.15, 123.81, 126.34, 130.40, 133.18, 136.60, 143.13, 148.35, 155.02, 173.73, 205.40;
HRMS-ESI: *m*/*z* calcd M⁺ for C₄₂H₃₆N₃O₃S₂⁺, 694.2193; found, 694.2196.
**VI₂a**: HRMS-ESI: *m*/*z* calcd M⁺ for C₄₄H₃₈N₃O₃S₂⁺, 720.2349; found, 720.2353.
**VI₃a**: ¹H-NMR (400 MHz, CDCl₃): 1.58(s, 6H, CH₃), 3.46(m, 8H, CH₂), 3.78 (m, 5H, CH2&CH), 5.44 (s, 2H, CH₂), 6.13 (s, 1H, CH), 6.37(s, 1H, CH), 7.10 (m, 4H, ArH), 7.24 (m, 4H, ArH), 7.33 (m, 5H, ArH), 7.52 (m, 5H, ArH).
¹³C-NMR (100 MHz, CDCl₃): 24.90, 33.41, 41.34, 52.26, 55.05, 58.13, 90.06, 99.53, 108.70, 111.42, 113.95, 116.07, 117.94, 120.15, 123.81, 126.34, 130.40, 133.18, 136.60, 143.13, 148.35, 155.02, 173.73, 205.40.
HRMS-ESI: *m*/*z* calcd M⁺ for C₄₅H₄₂N₃O₅S₂⁺, 768.2560; found, 768.2565.
**VI₄a**: HRMS-ESI: *m*/*z* calcd M⁺ for C₄₄H₃₆N₃O₃S₂⁺, 718.2193; found, 768.2597.
**VI₅a**: ¹H-NMR (400 MHz, CDCl₃): 1.57(s, 6H, CH₃), 3.50(m, 2H, CH₂), 3.79 (m, 3H, CH&CH₂), 5.42 (s, 2H, CH₂), 6.16 (s, 1H, CH), 6.39(s, 1H, CH), 7.08 (m, 4H, ArH), 7.23 (m, 9H, ArH), 7.35 (m, 5H, ArH), 7.50 (m, 5H, ArH).
¹³C-NMR (100 MHz, CDCl₃): 25.75, 33.90, 42.46, 53.02, 54.11, 91.39, 98.80, 108.61, 110.20, 111.87, 113.52, 116.10, 117.33, 118.92, 120.25, 122.19, 123.50, 126.81, 131.04, 133.50, 136.93, 143.10, 148.42, 155.08, 174.11;
HRMS-ESI: *m*/*z* calcd M⁺ for C₄₇H₃₈N₃O₃S₂⁺, 756.2349; found, 756.2353.
**VI₆a**: HRMS-ESI: *m*/*z* calcd M⁺ for C₄₄H₃₇N₃O₅S₂, 751.2175; found, 752.2179.

2. The synthetic route of dye VI₇a was shown as follows: 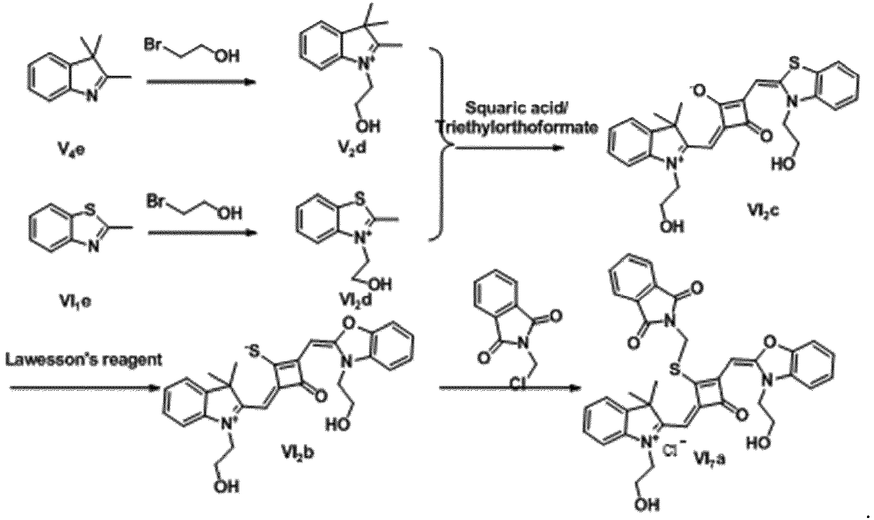
(1) 1 mol of 2,3,3-trimethyl-3H-indoline (V₄e) and 3 mol of 2-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₂d) which was dried and stored for use.
(2) 1 mol of 2-methylbenzoxazole (VI₁e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₂d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₂d) and 1 mol of the quaternary ammonium salt (VI₂d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VI₂c).
(4) 1 mol of the dye (VI₂c) was added to 25 mL of a mixed solvent of dry dichloromethane and tetrahydrofuran, to which 2 mol of Lawesson' s reagent was added. After stirred at 40°C for 3 h, the reaction mixture was dried under vacuum, and purified by column chromatography to give a bluish green centrally sulfur-substituted squaraine dye (VI₂b).
(5) 1 mol of the sulfur-substituted squaraine dye (VI₂b) was added to dry acetonitrile, to which 2.5 mol of an N-chloromethyl phthalimide intermediate was added. The reaction mixture was stirred under heating for a certain period of time until the reaction was confirmed to be completed by TLC. Then the reaction mixture was cooled to room temperature, dried under vacuum, and purified by column chromatography to give a target dye (**VI₇a**).

The compound **VI₇a** was characterized as follows:
¹H-NMR (400 MHz, CDCl₃): 1.49(s, 6H, CH₃), 3.66(m, 6H, CH₂), 3.95 (m, 2H, CH₂), 5.33 (s, 2H, CH₂), 6.15 (s, 1H, CH), 6.38(s, 1H, CH), 7.10 (m, 3H, ArH), 7.18 (m, 2H, ArH), 7.37 (m, 5H, ArH), 7.49 (m, 2H, ArH).
¹³C-NMR (100 MHz, CDCl₃): 24.75, 44.80, 54.25, 55.83, 61.62, 63.30, 88.90, 98.11, 109.15, 111.09, 114.41, 115.20, 118.73, 121.65, 124.29, 126.30, 130.04, 133.81, 136.60, 145.89, 147.55, 153.90, 173.07.
HRMS-ESI: *m*/*z* calcd M⁺ for C₃₆H₃₂N₃O₅S2⁺, 650.1778; found, 650.1783.

3. The synthetic route of dye VI₈a was shown as follows: 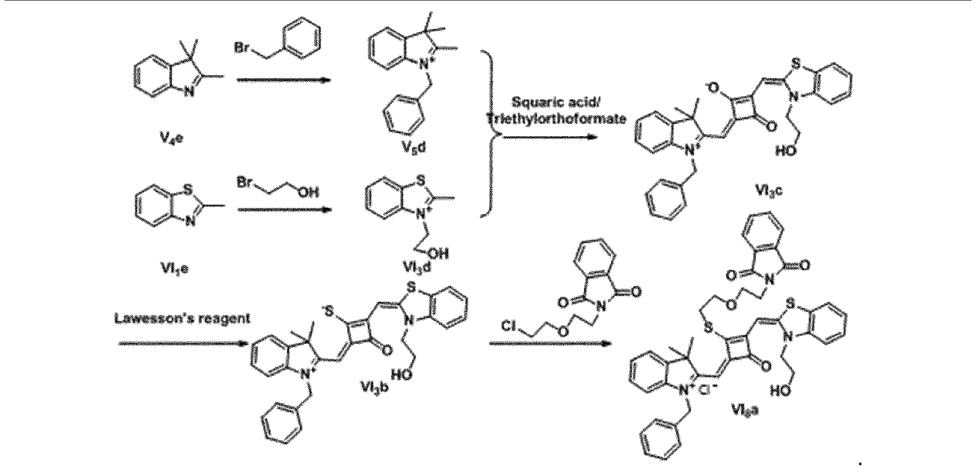
(1) 1 mol of 2,3,3-trimethyl-3H-indoline (V₄e) and 3 mol of benzyl bromide were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₅d) which was dried and stored for use.
(2) 1 mol of 2-methylbenzothiazole (VI₁e) and 2 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₃d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₅d) and 1 mol of the quaternary ammonium salt (VI₃d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VI₄c).
(4) 1 mol of the dye (VI₄c) was added to 25 mL of a mixed solvent of dry dichloromethane and tetrahydrofuran, to which 2 mol of Lawesson' s reagent was added. After stirred at 40°C for 3 h, the reaction mixture was dried under vacuum, and purified by column chromatography to give a bluish green centrally sulfur-substituted squaraine dye (VI₃b).
(5) 1 mol of the sulfur-substituted squaraine dye (VI₃b) was added to dry acetonitrile, to which 2.5 mol of a 2-chloroethoxyethyl phthalimide intermediate was added. The reaction mixture was stirred at 60°C for a certain period of time until the reaction was confirmed to be completed by TLC. Then the reaction mixture was cooled to room temperature, dried under vacuum, and purified by column chromatography to give a target dye (**VI₈a**).

The compound **VI₈a** was characterized as follows:
HRMS-ESI: *m*/*z* calcd M⁺ for C₄₄H₄₀N₃O₅S₂⁺, 754.2404; found, 754.2408.

4. The synthetic route of dye VI₉a was shown as follows: 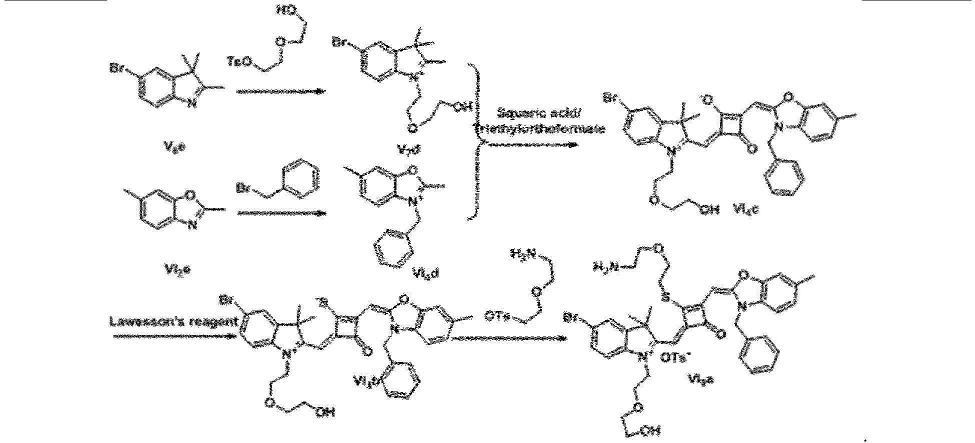
(1) 1 mol of 2,3,3-trimethyl-5-bromo-3H-indoline (V₆e) and 3 mol of ethanol, 2-(2-hydroxyethoxy)-, 1-(4-Methylbenzenesulfonate) were added to 20 mL of dry 1,2-dichlorobenzene. The reaction was heated and refluxed under nitrogen protection for 24 h. After cooled to room temperature, the reaction mixture was washed with anhydrous diethyl ether and recrystallized with propanone to give a white to pink quaternary ammonium salt solid (V₇d) which was dried and stored for use.
(2) 1 mol of 2, 5-dimethylbenzothiazole (VI₂e) and 3 mol of benzyl bromide were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₄d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₇d) and 1 mol of the quaternary ammonium salt (VI₄d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VI₄c).
(4) 1 mol of the dye (VI₄c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VI₄b).
(5) 1 mol of the sulfur-substituted squaraine dye (VI₄b) was added to dry acetonitrile, to which 2.5 mol of 2-aminoethoxyethyl *p*-toluenesulfonate was added. The reaction mixture was stirred at 35°C for a certain period of time until the reaction was confirmed to be completed by TLC. Then the reaction mixture was cooled to room temperature, dried under vacuum, and purified by column chromatography to give a target dye (**VI₉a**).

The compound **VI₉a** was characterized as follows:
¹H-NMR (400 MHz, CDCl₃): 1.52(s, 6H, CH₃), 2.43(s, 3H, CH₃), 3.03 (m, 4H, CH₂), 3.73 (m, 10H, CH₂), 4.09 (t, 2H, CH₂, *J*=8.0 Hz), 5.13 (s, 2H, CH₂, *J*=8.0 Hz), 6.14 (s, 1H, CH), 6.39(s, 1H, CH), 7.06 (m, 2H, ArH), 7.19 (m, 6H, ArH), 7.44 (m, 3H, ArH).
¹³C-NMR (100 MHz, CDCl₃): 20.89, 24.70, 33.81, 41.60, 50.33, 54.60, 61.52, 69.04, 73.11, 89.45, 100.16, 108.04, 111.35, 113.26, 114.90, 118.37, 121.50, 125.18, 126.90, 131.50, 134.96, 136.82, 144.03, 147.78, 152.60, 173.15.
HRMS-ESI: *m*/*z* calcd M⁺ for C₃₉H₄₃BrN₃O₄S₂⁺, 760.1873; found, 760.1878.

5. The synthetic route of dye VI₁₀a was shown as follows: 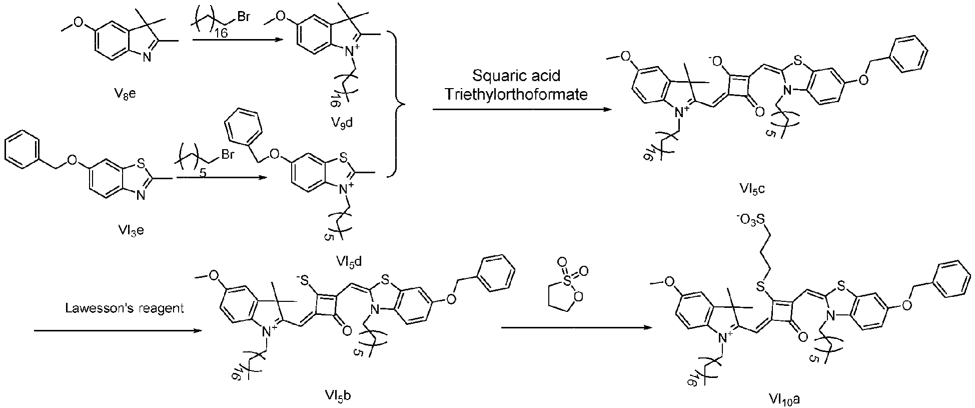
(1) 1 mol of 2,3,3-trimethyl-5-methoxy-3H-indoline (V₈e) and 2 mol of octadecyl bromide were added to 20 mL of dry 1,2-dichlorobenzene, to which potassium iodide was added as a catalyst. Then the reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₉d) which was dried and stored for use.
(2) 1 mol of 2-methyl-5-benzyloxybenzoxazole (VI₃e) and 3 mol of bromoheptane were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₅d) which was dried and stored for use.
(3) 2 mol of squaric acid was added to 100 mL of absolute ethanol, to which triethyl orthoformate was added as a catalyst. The mixture was heated and refluxed under nitrogen protection until the squaric acid was completely dissolved, and then 1 mol of the quaternary ammonium salt (V₉d) and 1 mol of the quaternary ammonium salt (VI₅d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VI₅c).
(4) 1 mol of the dye (VI₅c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VI₅b).
(5) 1 mol of the sulfur-substituted squaraine dye (VI₅b) was added to dry acetonitrile, to which 2 mol of a butane sultone intermediate was added. The reaction mixture was stirred at 35°C for a certain period of time until the reaction was confirmed to be completed by TLC. Then the reaction mixture was cooled to room temperature, dried under vacuum, and purified by column chromatography to give a target dye (**VI₁₀a**).

The compound **VI₁₀a** was characterized as follows:
HRMS-ESI: *m*/*z* calcd [M+H]⁺ for C₅₈H₈₁N₂O₆S₃, 997.5179; found, 997.5185.

### Example 7

The asymmetric dyes were further respectively substituted with different groups at the middle position to obtain asymmetric dyes (**VII₁a to VII₁₀a**) which were shown as follows with X= NC₂H₅ and Y= C(CH₃)₂ in formula (I), wherein formulae VII₁a, VII₂a, VII₃a, VII₄a, VII₅a, VII₆a, VII₇a, VII₈a, VII₉a, VII₁₀a are non-inventive embodiments, not falling under the scope of the claims: 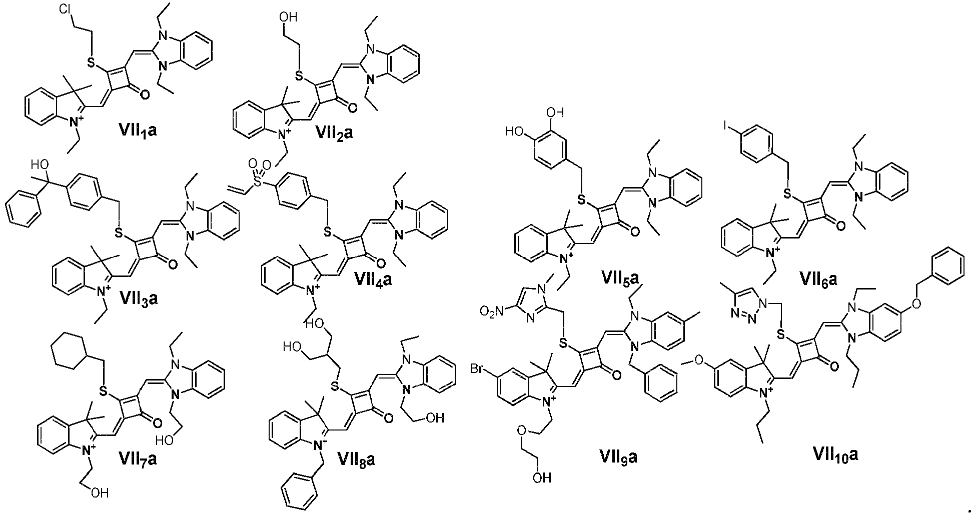
1. The synthetic routes of dyes VII₁a to VII₆a were shown as follows: 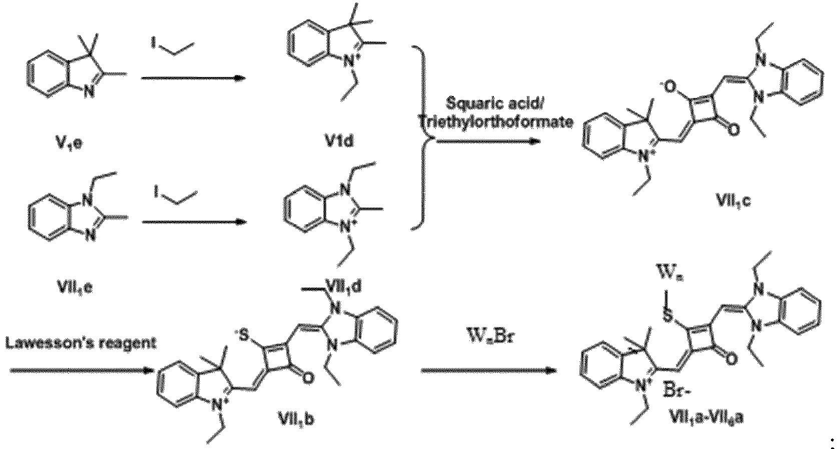 wherein, the structural formulas of Wₙ (n=1 to 6) were respectively shown as follows:

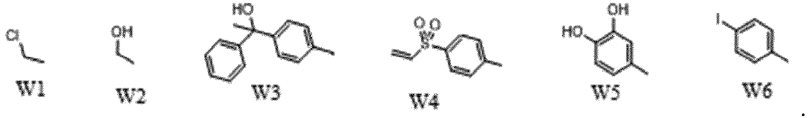

The dyes VII₁a-VII₆a were specifically prepared as follows.
(1) 1 mol of 2,3,3-trimethyl-3H-indoline (V₁e) and 3 mol of iodoethane were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₁d) which was dried and stored for use.
(2) 1 mol of 2-methyl-1-N-ethylbenzimidazole (VII₁e) and 3 mol of iodoethane were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (VII₁d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₁d) and 1 mol of the quaternary ammonium salt (VII₁d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VII₁c).
(4) 1 mol of the dye (VII₁c) was added to 25 mL of a mixed solvent of dry dichloromethane and tetrahydrofuran, to which 2 mol of Lawesson's reagent was added. After stirred at 40°C for 3 h, the mixture was dried under vacuum, and purified by column chromatography to give a bluish green centrally sulfur-substituted squaraine dye (VII₁b).
(5) 1 mol of the sulfur-substituted squaraine dye (VII₁b) was added to dry acetonitrile, to which 2.5 mol of a bromo-substituted intermediate WₙBr (Wₙ was selected from W₁ to W₆) was added. The reaction mixture was stirred at room temperature or under heating for a certain period of time until the reaction was confirmed to be completed by TLC. Then the reaction mixture was cooled to room temperature, dried under vacuum, and purified by column chromatography to give a corresponding target dye (**VII₁a to VII₆a**).

The compounds **VII₁a to VII₆a** were characterized as follows:
VII₁a: ¹H-NMR (400 MHz, CDCl₃): 1.33 (m, 6H, CH₃), 1.44 (t, 3H, CH₃, *J*=8.0 Hz), 1.55 (s, 6H, CH₃), 3.16 (t, 2H, CH₂, *J*=8.0 Hz), 4.12 (m, 6H, CH₂), 4.59 (q, 2H, CH₂, *J*=8.0 Hz), 6.17 (s, 1H, CH), 6.39(s, 1H, CH), 7.11 (m, 3H, ArH), 7.24 (m, 3H, ArH), 7.47 (m, 2H, ArH).
¹³C-NMR (100 MHz, CDCl₃): 14.13, 25.70, 33.85, 43.40, 44.36, 47.90, 56.02, 89.90, 99.25, 108.50, 111.71, 114.15, 117.08, 120.46, 123.21, 124.45, 128.75, 143.56, 158.18, 172.70, 203.50.
HRMS-ESI: *m*/*z* calcd M⁺ for C₃₁H₃₅ClN₃OS⁺, 532.2184; found, 532.2188.
VII₂a: HRMS-ESI: *m*/*z* calcd M⁺ for C₃₁H₃₆N3O2S⁺, 514.2523; found, 514.2527.
VII₃a: HRMS-ESI: *m*/*z* calcd M⁺ for C₄₄H₄₆N₃O₂S⁺, 680.3305; found, 680.3308.
VII₄a: HRMS-ESI: *m*/*z* calcd M⁺ for C₃₈H₄₀N₃O₃S₂⁺, 650.2506; found, 650.2510.
VII5a: HRMS-ESI: *m*/*z* calcd M⁺ for C₃₆H₃₈N₃O₃S₂⁺, 592.2628; found, 592.2632.
VII₆a: HRMS-ESI: *m*/*z* calcd M⁺ for C₃₆H₃₇IN₃OS⁺, 686.1697; found, 686.1701.

2. The synthetic route of dye VII₇a was shown as follows: 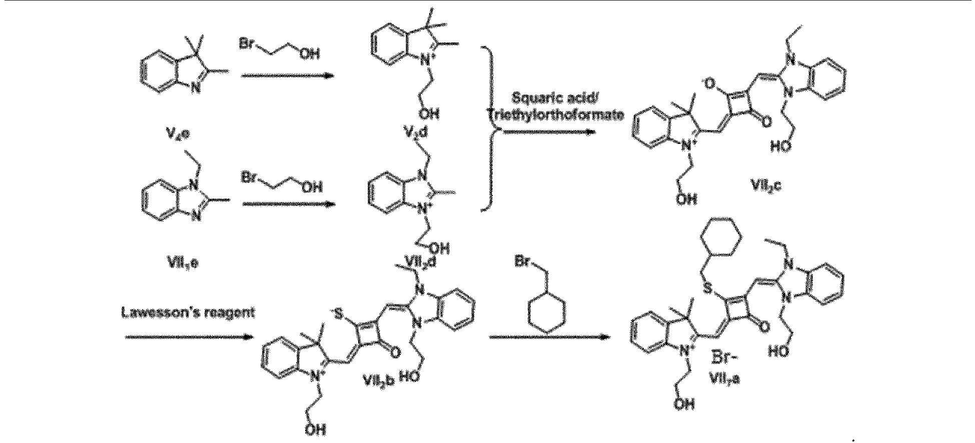
(1) 1mol of 2,3,3-trimethyl-3H-indoline (V₁e) and 3 mol of 2-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₂d) which was dried and stored for use.
(2) 1 mol of 1-N-ethyl-2-methylbenzimidazole (VII₁e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (VII₂d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₂d) and 1 mol of the quaternary ammonium salt (VII₂d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VII₂c).
(4) 1 mol of the dye (VII₂c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VII₂b).
(5) 1 mol of the sulfur-substituted squaraine dye (VII₂b) was added to dry acetonitrile, to which 2.5 mol of cyclohexylmethyl bromide was added. The reaction mixture was stirred under heating until the reaction was confirmed to be completed by TLC. Then the reaction mixture was cooled to room temperature, dried under vacuum, and purified by column chromatography to give a target dye (**VII₇a**).

The compound **VII₇a** was characterized as follows:
¹H-NMR (400 MHz, CDCl₃): 1.29 (m, 5H, CH₃&CH₂), 1.49 (m, 8H, CH₂), 1.65 (m, 7H, CH₃&CH), 2.83 (d, 2H, CH₂, *J*=8.0 Hz), 3.68 (m, 4H, CH₂), 3.92 (m, 6H, CH₂), 6.15 (s, 1H, CH), 6.38(s, 1H, CH), 7.10 (m, 3H, ArH), 7.27 (m, 3H, ArH), 7.49 (m, 2H, ArH).
¹³C-NMR (100 MHz, CDCl₃): 14.37, 25.80, 26.92, 32.64, 34.85, 42.40, 52.85, 63.30, 90.05, 100.61, 109.44, 112.17, 113.30, 115.90, 117.35, 120.82, 123.08, 124.19, 127.40, 143.31, 158.25, 173.90.
HRMS-ESI: *m*/*z* calcd M⁺ for C₃₆H₄₄N₃O₃S⁺, 598.3098; found, 598.3103.

3. The synthetic route of dye VII₈a was shown as follows: 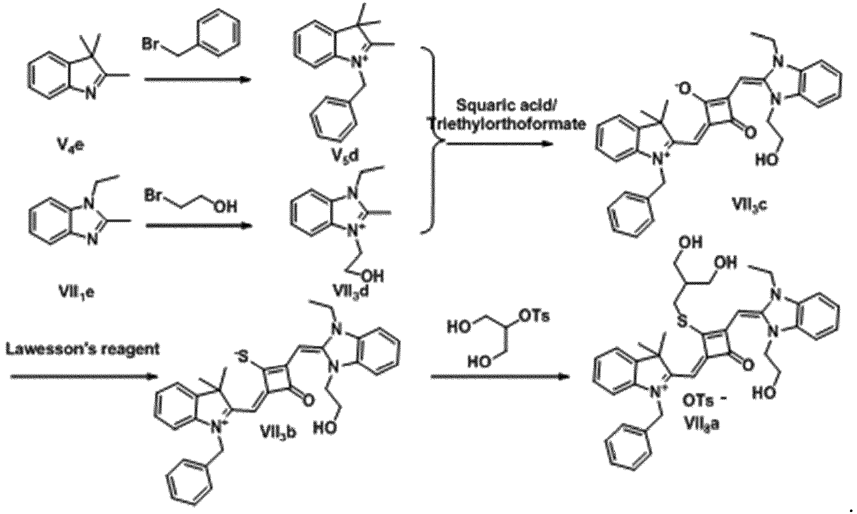
(1) 1 mol of 2,3,3-trimethyl-3H-indoline (V₁e) and 3 mol of benzyl bromide were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (V₅d) which was dried and stored for use.
(2) 1 mol of 1-N-ethyl-2-methylbenzimidazole (VII₁e) and 2 mol of 2-bromoethanol were added to 20 mL of dry toluene. The reaction was heated under nitrogen protection for 24 h. After cooled to room temperature, the reaction mixture was washed with anhydrous diethyl ether and recrystallized with propanone to give a white to pink quaternary ammonium salt solid (VII₃d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₅d) and 1 mol of the quaternary ammonium salt (VII₃d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VII₃c).
(4) 1 mol of the dye (VII₃c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VII₃b).
(5) 1 mol of the sulfur-substituted squaraine dye (VII₃b) was added to dry acetonitrile, to which 2.5 mol of 2-*p*-toluenesulfonate glyceryl was added. The reaction mixture was stirred under heating until the reaction was confirmed to be completed by TLC. Then the reaction mixture was cooled to room temperature, dried under vacuum, and purified by column chromatography to give a target dye (**VII₈a**).

The compound **VII₈a** was characterized as follows:
HRMS-ESI: *m*/*z* calcd M⁺ for C₃₈H₄₂N₃O₄S⁺, 636.2891; found, 636.2896.

4. The synthetic route of dye VII₉a was shown as follows:

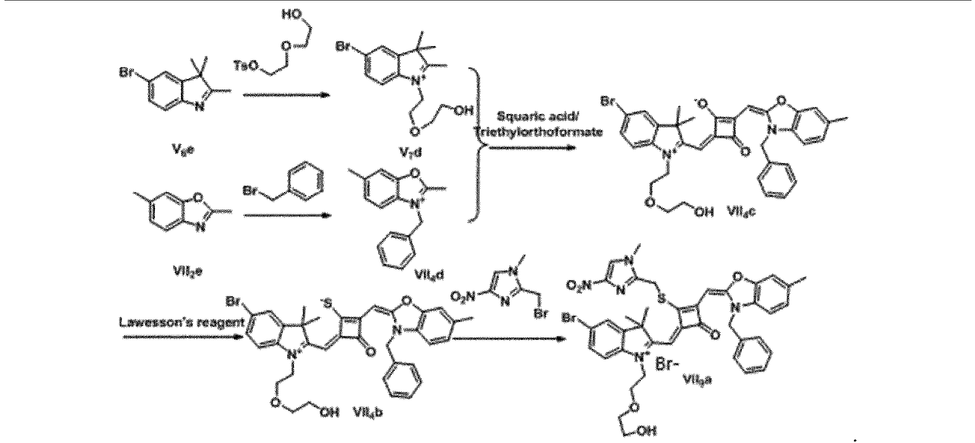

The dye VII₉a was specifically prepared as follows.
(1) 1 mol of 2,3,3-trimethyl-5-bromo-3H-indoline(V₆e) and 3 mol of ethanol, 2-(2-hydroxyethoxy)-, 1-(4-Methylbenzenesulfonate) were added to 20 mL of dry 1,2-dichlorobenzene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₇d) which was dried and stored for use.
(2) 1 mol of 2,5-dimethylbenzothiazole (VII₂e) and 3 mol of benzyl bromide were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VII₄d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₇d) and 1 mol of the quaternary ammonium salt (VII₄d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VII₄c).
(4) 1 mol of the dye (VII₄c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VII₄b).
(5) 1 mol of the sulfur-substituted squaraine dye (VII₄b) was added to dry acetonitrile, to which 2.5 mol of N-methyl-nitroimidazole methyl bromide was added. The reaction mixture was stirred under heating until the reaction was confirmed to be completed by TLC. Then the reaction mixture was cooled to room temperature, dried under vacuum, and purified by column chromatography to a give target dye (**VII₉a**).

The compound **VII₉a** was characterized as follows:
¹H-NMR (400 MHz, CDCl₃): 1.31 (t, 3H, CH₃, *J*=8.0 Hz), 1.58 (s, 6H, CH₃), 2.45 (s, 3H, CH₃), 3.72 (m, 11H, CH₃&CH₂), 4.12 (m, 4H, CH₂), 6.19 (s, 1H, CH), 6.42(s, 1H, CH), 7.12 (m, 4H, ArH), 7.33 (m, 5H, ArH), 7.48 (m, 3H, ArH).
¹³C-NMR (100 MHz, CDCl₃): 15.35, 21.60, 26.93, 32.08, 44.10, 50.42, 53.95, 61.30, 67.05, 70.61, 89.10, 99.53, 109.60, 112.45, 113.03, 113.95, 115.50, 117.81, 118.30, 120.26, 123.70, 124.18, 129.40, 143.38, 158.72, 174.60.
HRMS-ESI: *m*/*z* calcd M⁺ for C₄₂H₄₄BrN₆O₅S₊, 823.2272; found, 823.2275.

5. The synthetic route of dye VII₁₀a was shown as follows: 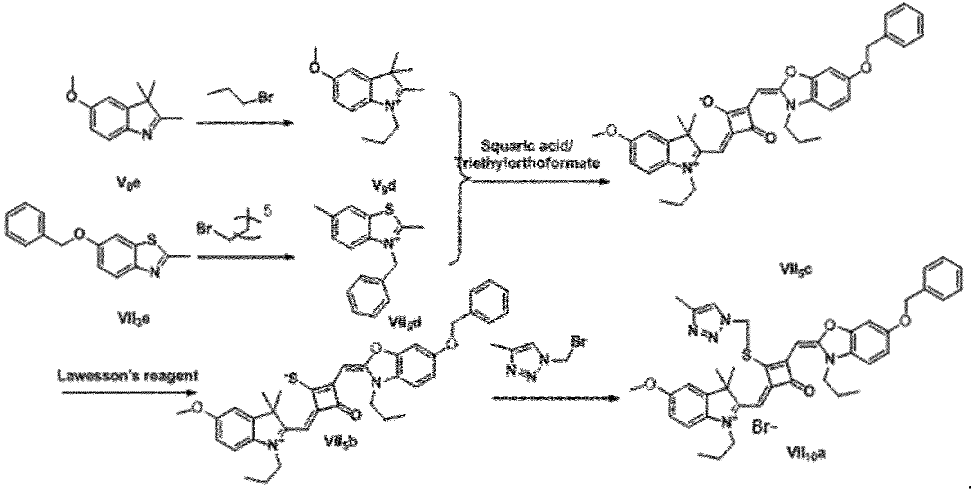
(1) 1 mol of 2,3,3-trimethyl-5-methoxy-3H-indoline (V₈e) and 2 mol of bromopropane were added to 20 mL of dry 1,2-dichlorobenzene, to which potassium iodide was added as a catalyst. Then the reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (V₉d) which was dried and stored for use.
(2) 1 mol of 2-methyl-5-benzyloxybenzoxazole (VII₃e) and 3 mol of bromopropane were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VII₅d) which was dried and stored for use.
(3) 2 mol of squaric acid was added to 100 mL of absolute ethanol, to which triethyl orthoformate was added as a catalyst. The mixture was heated and refluxed under nitrogen protection until the squaric acid was completely dissolved, and then 1 mol of the quaternary ammonium salt (Vgd) and 1 mol of the quaternary ammonium salt (VII₅d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VII₅c).
(4) 1 mol of the dye (VII₅c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VII₅b).
(5) 1 mol of the sulfur-substituted squaraine dye (VI₅b) was added to dry acetonitrile, to which 2.5 mol of methyltriazole methyl bromide was added. The reaction mixture was stirred under heating until the reaction was confirmed by TLC to be completed. Then the reaction mixture was cooled to room temperature, dried under vacuum, and purified by column chromatography to give a target dye (**VII₁₀a**).

The compound **VII₁₀a** was characterized as follows:
HRMS-ESI: *m*/*z* calcd M⁺ for C₄₃H₄₉N₆O₃S⁺, 729.3581; found, 729.3585.

### Example 8

The asymmetric dyes were further respectively substituted with different groups at the middle position to obtain exemplary asymmetric target dyes (**VIII₁a to VIII₁₀a**) which were shown as follows with X= O and Y= S in formula (I), wherein formulae VIII₁a, VIII₂a, VIII₃a, VIII₅a, VIII₆a, VIII₇a, VIII₈a, VIII₉a, VIII₁₀a are non-inventive embodiments, not falling under the scope of the claims: 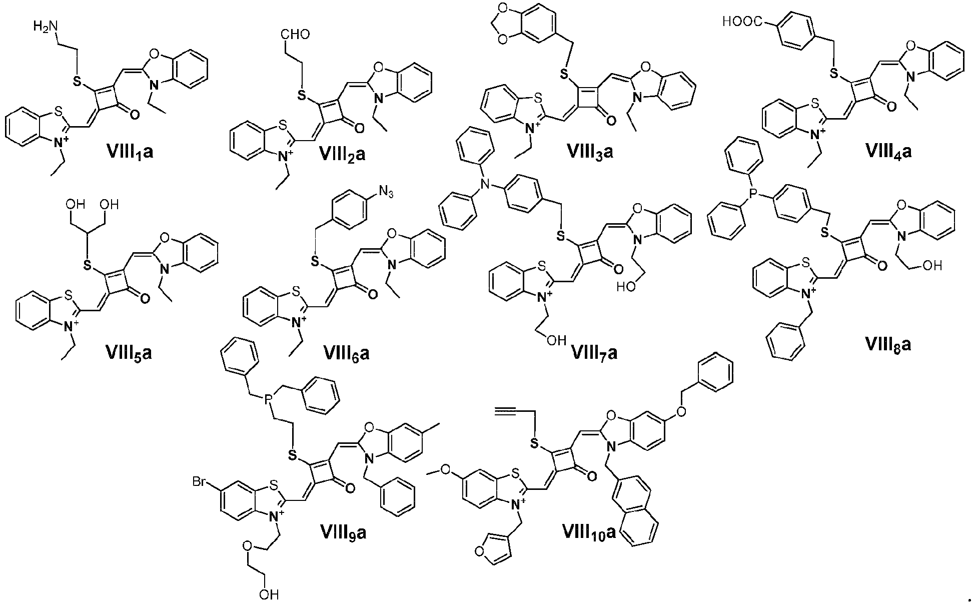
1. The synthetic routes of dyes VIII₁a to VIII₆a were shown as follows: 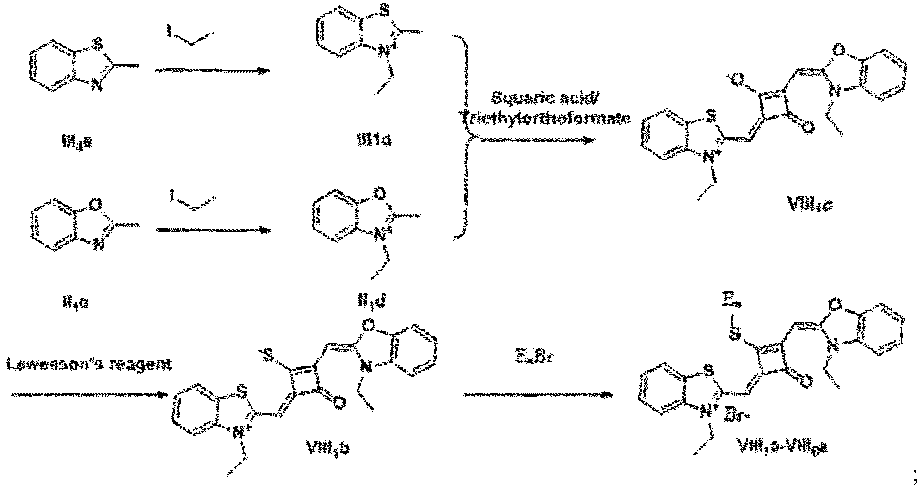 wherein, the structural formulas of Eₙ (n=1 to 6) were respectively shown as follows: 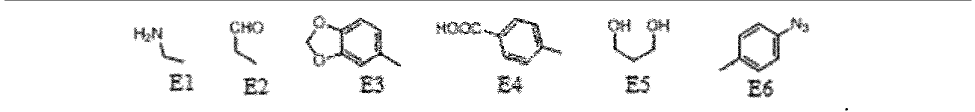
   (1) 1 mol of 2-methylbenzothiazole (III₄e) and 3 mol of iodoethane were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (III₁d) which was dried and stored for use.
   (2) 1 mol of 2-methylbenzoxazole (II₄e) and 3 mol of iodoethane were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₁d) which was dried and stored for use.
   (3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₁d) and 1 mol of the quaternary ammonium salt (II₁d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VIII₁c).
   (4) 1 mol of the dye (VIII₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VIII₁b).
   (5) 1 mol of the sulfur-substituted squaraine dye (VIII₁b) was added to dry acetonitrile, to which 2.5 mol of a bromo-substituted intermediate EₙBr (Eₙ was selected from E₁ to E₆) was added. The reaction mixture was stirred at room temperature or under heating until the reaction was confirmed by TLC to be completed. Then the reaction mixture was cooled to room temperature, dried under vacuum, and purified by column chromatography to give a corresponding target dye (**VIII₁a to VIII₆a**).

The compounds **VIII₁a to VIII₆a** were characterized as follows:
**VIII₁a**: HRMS-ESI: *m*/*z* calcd M⁺ for C₂₆H₂₆N₃O₂S₂⁺, 476.1461; found, 476.1464.
**VIII₂a**: ¹H-NMR (400 MHz, CDCl₃): 1.44 (m, 6H, CH₃), 3.15 (m, 4H, CH₂), 4.45 (m, 4H, CH₂), 6.18 (s, 1H, CH), 6.45(s, 1H, CH), 7.10 (m, 4H, ArH), 7.35 (m, 5H, ArH), 7.47 (m, 3H, ArH), 9.72 (t, 1H, CHO, *J*=8.0 Hz).
¹³C-NMR (100 MHz, CDCl₃): 14.39, 15.60, 29.93, 43.08, 50.49, 53.10, 90.30, 99.05, 108.61, 111.35, 112.20, 113.09, 115.87, 117.12, 118.75, 120.40, 123.16, 124.90, 127.04, 143.49, 158.45, 175.07, 203.50.
HRMS-ESI: *m*/*z* calcd M⁺ for C₂₇H₂₅N₂O₇S₂⁺, 489.1301; found, 489.1304.
**VIII₃a**: HRMS-ESI: *m*/*z* calcd M⁺ for C₃₂H₂₇N₂O₄S₂⁺, 567.1407; found, 567.1411.
**VIII₄a**: HRMS-ESI: *m*/*z* calcd M⁺ for C₃₂H₂₇N₂O₄S₂⁺, 567.1407; found, 567.1415.
**VIII₅a**: HRMS-ESI: *m*/*z* calcd M⁺ for C₂₇H₂₇N₂O₄S₂⁺, 507.1407; found, 507.1412.
**VIII₆a**: HRMS-ESI: *m*/*z* calcd M⁺ for C₃₁H₂₆N₅O₂S₂⁺, 564.1522; found, 564.1526.

2. The synthetic route of dye VIII₇a was shown as follows: 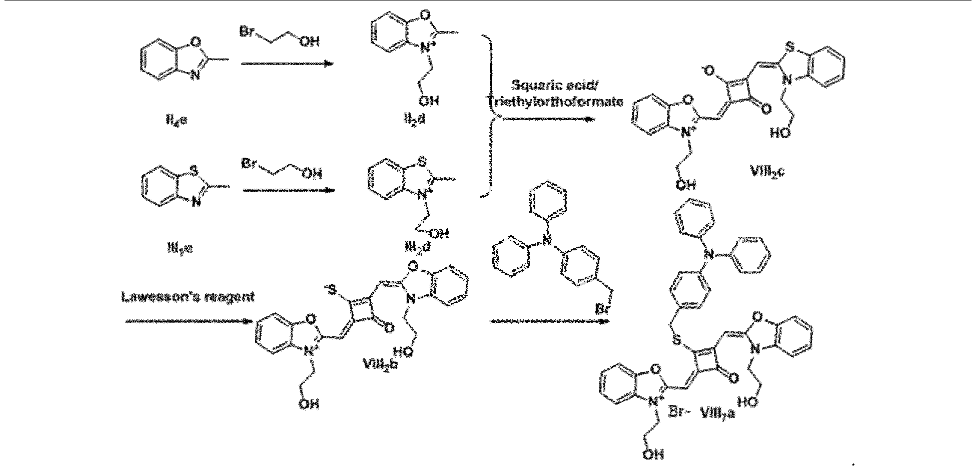
(1) 1 mol of 2-methylbenzothiazole (III₄e) and 3 mol of 2-bromoethanol were added to 20 mL of dry toluene. The reaction mixture was heated and refluxed under nitrogen protection for 12 h. After cooled to room temperature, the reaction mixture was washed with anhydrous diethyl ether and recrystallized with propanone to give a white to pink quaternary ammonium salt solid (III₂d) which was dried and stored for use.
(2) 1 mol of 2-methylbenzoxazoic (II₄e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₂d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₂d) and 1 mol of the quaternary ammonium salt (II₂d) were added. The reaction mixture was continuously refluxed until the reaction was confirmed to be completed by TLC. Then the reaction mixture was cooled, dried under reduced pressure to remove the solvent, and purified by column chromatography to give a blue solid dye (VIII₂c).
(4) 1 mol of the dye (VIII₂c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VIII₂b).
(5) 1 mol of the sulfur-substituted squaraine dye (VIII₂b) was added to dry acetonitrile, to which 2.5 mol of a bromomethyltriphenylamine intermediate was added. The reaction mixture was stirred under heating until the reaction was confirmed by TLC to be completed. Then the reaction mixture was cooled to room temperature, dried under vacuum, and purified by column chromatography to give a target dye (**VIII₇a**).

The compound **VIII₇a** was characterized as follows:
¹H-NMR (400 MHz, CDCl₃): 3.64 (m, 5H, CH₂&OH), 3.72 (m, 2H, CH₂), 3.99 (m, 2H, CH₂), 4.49 (s, 2H, CH₂), 6.13 (s, 1H, CH), 6.37(s, 1H, CH), 7.10 (m, 2H, ArH), 7.15 (m, 4H, ArH), 7.29 (m, 5H, ArH), 7.34 (m, 6H, ArH), 7.47 (m, 5H, ArH).
¹³C-NMR (100 MHz, CDCl₃): 42.70, 51.82, 54.65, 63.05, 64.40, 88.12, 98.63, 109.90, 112.07, 112.99, 113.65, 115.18, 117.78, 118.40, 120.26, 123.50, 124.61, 126.93, 127.42, 130.03, 138.17, 143.45, 156.90, 173.87.
HRMS-ESI: *m*/*z* calcd M⁺ for C₄₃H₃₆N₃O₄S₂⁺, 722.2142; found, 722.2147.

3. The synthetic route of dye VIII₈a was shown as follows: 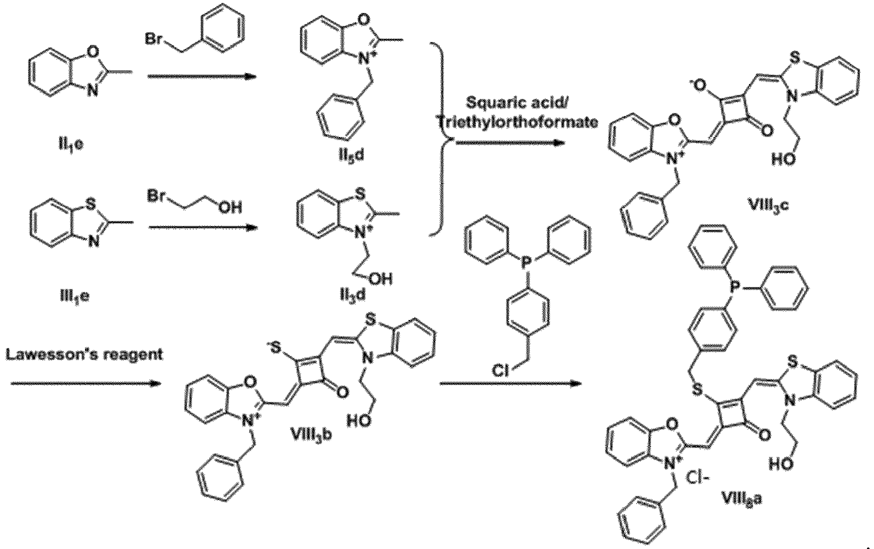
(1) 1 mol of 2-methylbenzoxazole (II₁e) and 3 mol of 2-bromoethanol were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to white quaternary ammonium salt solid (II₅d)which was dried and stored for use.
(2) 1mol of 2-methylbenzothiazole (III₁e) and 2 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₃d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (II₅d) and 1 mol of the quaternary ammonium salt (II₃d) were added. The reaction mixture was continuously refluxed until the reaction was confirmed by TLC to be completed. Then the reaction mixture was cooled, dried under reduced pressure to remove the solvent, and purified by column chromatography to give a blue solid dye (VIII₃c).
(4) 1 mol of the dye (VIII₃c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VIII₃b).
(5) 1 mol of the sulfur-substituted squaraine dye (VIII₃b) was added to dry acetonitrile, to which 2.5 mol of a chloromethyl triphenylphosphine intermediate was added. The reaction mixture was stirred under heating for a certain period of time until the reaction was confirmed by TLC to be completed. Then the reaction mixture was cooled to room temperature, dried under vacuum, and purified by column chromatography to give a target dye (**VIII₈a**).

The compound **VIII8a** was characterized as follows:
HRMS-ESI: *m*/*z* calcd M⁺ for C₄₈H₃₈N₂O₃PS₂⁺, 785.2056; found, 785.2062.

4. The synthetic route of dye VIII₉a was shown as follows: 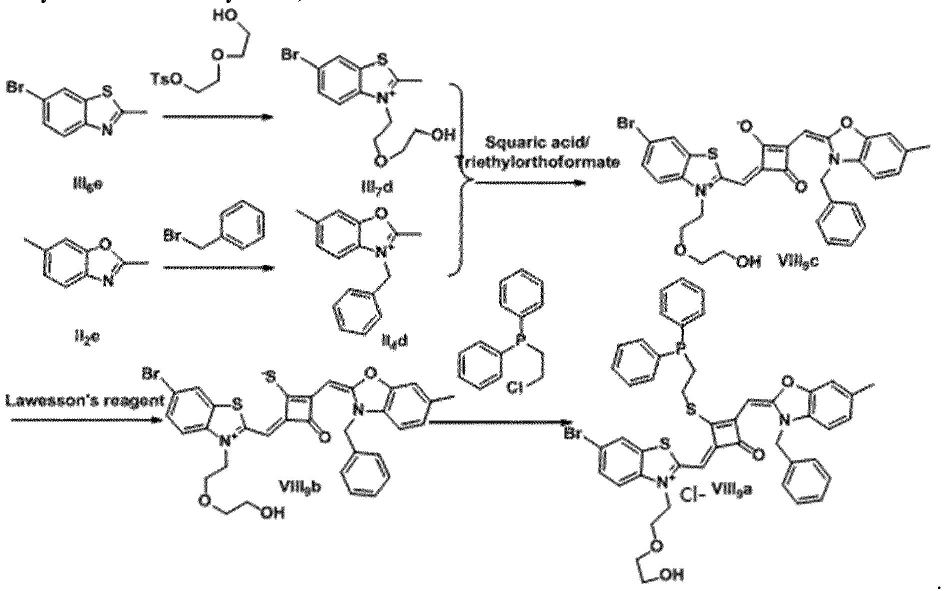
(1) 1 mol of 2-methyl-5-bromobenzothiazole (III₆e) and 3 mol of ethanol, 2-(2-hydroxyethoxy)-, 1-(4-Methylbenzenesulfonate) were added to 20 mL of dry 1,2-dichlorobenzene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (III₇d) which was dried and stored for use.
(2) 1 mol of 2,5-dimethylbenzoxazole (II₂e) and 3 mol of benzyl bromide were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₄d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₇d) and 1 mol of the quaternary ammonium salt (II₄d) were added. The reaction mixture was continuously refluxed until the reaction was confirmed by TLC to be completed. Then the reaction mixture was cooled, dried under reduced pressure to remove the solvent, and purified by column chromatography to give a blue solid dye (VIII₉c).
(4) 1 mol of the dye (VIII₉c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VIII₉b).
(5) 1 mol of the sulfur-substituted squaraine dye (VIII₉b) was added to dry acetonitrile, to which 2.5 mol of a 2-chloroethyl diphenylphosphine intermediate was added. The reaction mixture was stirred under heating until the reaction was confirmed by TLC to be completed. Then the reaction mixture was cooled to room temperature, dried under vacuum, and purified by column chromatography to a give target dye (**VIII₉a**).

The compound **VIII9a** was characterized as follows:
HRMS-ESI: *m*/*z* calcd M⁺ for C₄₈H₄₅BrN₂O₄PS₂⁺, 887.1736; found, 887.1741.

5. The synthetic route of dye VIII₁₀a was shown as follows: 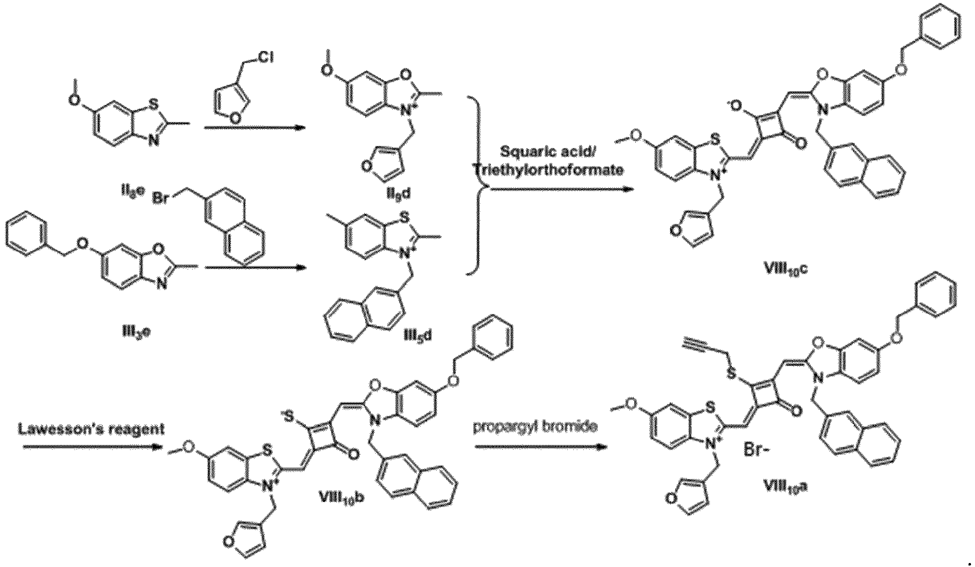
(1) 1 mol of 2-methyl-5-benzyloxy-benzothiazole (II₈e) and 2 mol of 3-furanmethyl chloride were added to 20 mL of dry 1,2-dichlorobenzene, to which potassium iodide was added as a catalyst. Then the reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (II₉d) which was dried and stored for use.
(2) 1 mol of 2-methyl-5-methoxybenzoxazole (II₃e) and 3 mol of naphthylmethyl bromide were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (III₅d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (II₉d) and 1 mol of quaternary ammonium salt (III₅d) were added. The reaction mixture was continuously refluxed until the reaction mixture was confirmed by TLC to be completed. Then the reaction mixture was cooled, dried under reduced pressure to remove the solvent, and purified by column chromatography to give a blue solid dye (VIII₁₀c).
(4) 1 mol of the dye (VIII₁₀c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (VIII₁₀b).
(5) 1 mol of the sulfur-substituted squaraine dye (VIII₁₀b) was added to dry acetonitrile, to which 2.5 mol of a propargyl bromide intermediate was added. The reaction mixture was stirred under heating until the reaction was confirmed by TLC to be completed. Then the reaction mixture was cooled to room temperature, dried under vacuum, and purified by column chromatography to give a target dye (**VIII₁₀a**).

The compound **VIII₁₀a** was characterized as follows:
¹H-NMR (400 MHz, CDCl₃): 2.83 (t, 1H, CH, *J*=8.0 Hz), 3.82 (s, 3H, CH₃), 4.33 (d, 2H, CH₂, *J*=8.0 Hz), 5.16 (s, 2H, CH₂), 5.40 (s, 2H, CH₂), 5.53 (s, 2H, CH₂), 6.16 (s, 1H, CH), 6.38(s, 1H, CH), 6.73(d, 1H, ArH, *J*=4.0 Hz), 7.08 (m, 4H, ArH), 7.17 (m, 3H, ArH), 7.29 (m, 10H, ArH), 7.58 (m, 3H, ArH).
¹³C-NMR (100 MHz, CDCl₃): 22.70, 45.02, 53.65, 55.80, 70.87, 73.05, 76.40, 89.21, 98.33, 108.41, 111.07, 113.60, 116.55, 117.01, 120.29, 124.42, 125.93, 127.70, 130.51, 133.72, 134.10, 138.29, 141.16, 143.05, 156.50, 173.31.
HRMS-ESI: *m*/*z* calcd M⁺ for C₄₇H₃₅N₂O₅S₂⁺, 771.1982; found, 771.1987.

### Example 9

The asymmetric dyes were further respectively substituted with different groups at the middle position to obtain exemplary asymmetric target dyes (**IX₁a to IX₁₀a**) which were shown as follows with X= NCH₂CH₃ and Y= S in formula (I), wherein formulae IX₂a, IX₄a, IX₅a, IX₆a, IX₇a, IX₈a, IX₉a, IX₁₀a are non-inventive embodiments, not falling under the scope of the claims: 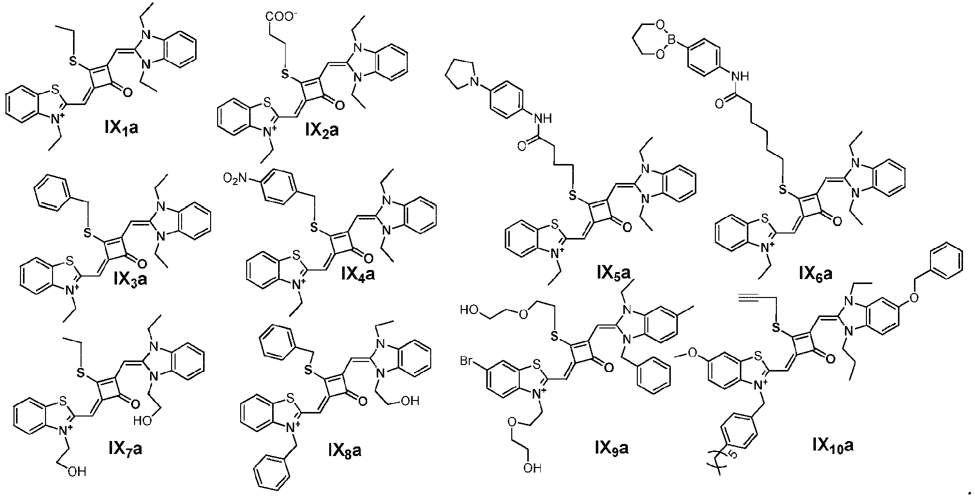
1. The synthetic route of dyes IX₁a to IX₆a was shown as follows: 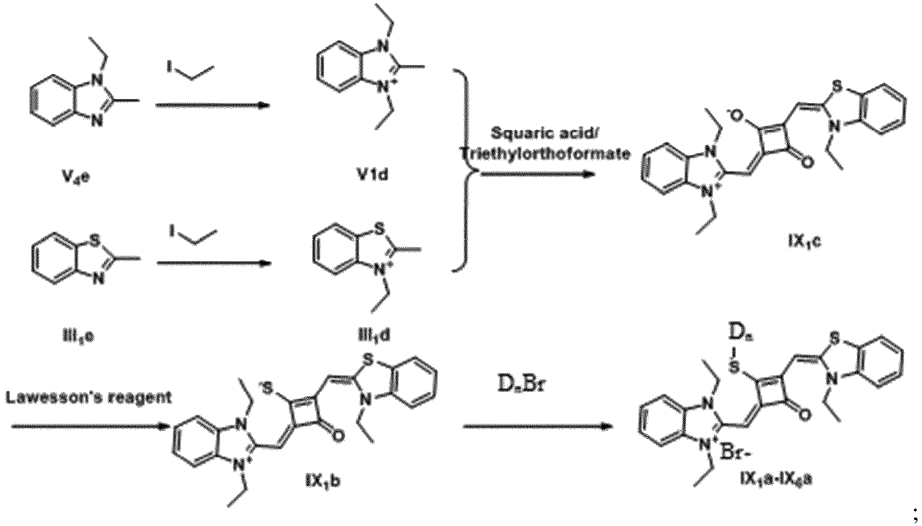 wherein, the structural formulas of Dₙ (n=1-6) were respectively shown as follows: 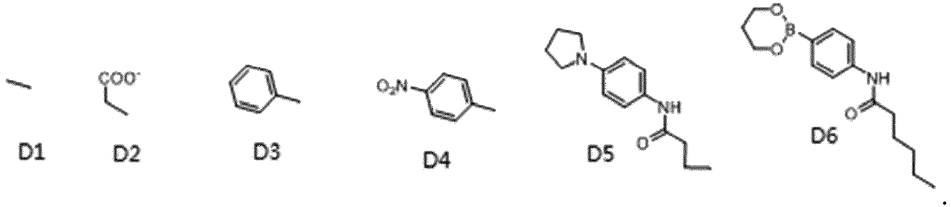
   (1) 1 mol of 2-methyl-N-ethylbenzimidazole (V₄e) and 4 mol of iodoethane were added to 20 mL of dry 1,2-dichlorobenzene. The reaction mixture was heated and refluxed under nitrogen protection for 36 h. After cooled to room temperature, the reaction mixture was washed with anhydrous diethyl ether and recrystallized with propanone to give a white to dark gray quaternary ammonium salt solid (V₁d) which was dried and stored for use.
   (2) 1mol of 2-methylbenzothiazole (II₁e) and 3 mol of iodoethane were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (II₁d) which was dried and stored for use.
   (3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (IV₁d) and 1 mol of the quaternary ammonium salt (II₁d) were added. The reaction mixture was continuously refluxed until the reaction was confirmed by TLC to be completed. Then the reaction mixture was cooled, dried under reduced pressure to remove the solvent, and purified by column chromatography to give a blue solid dye (IX₁c).
   (4) 1 mol of the dye (IX₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IX₁b).
   (5) 1 mol of the sulfur-substituted squaraine dye (IX₁b) was added to dry acetonitrile, to which 2.5 mol of a bromo-substituted intermediate DₙBr (Dₙ was selected from D₁ to D₆) was added. The reaction mixture was stirred at room temperature or under heating until the reaction was confirmed by TLC to be completed. Then the reaction mixture was cooled to room temperature, dried under vacuum, and purified by column chromatography to give a corresponding target dye (**IX₁a to IX₆a**).

The compounds **IX₁a to IX₆a** were characterized as follows:
**IX₁a**: ¹H-NMR (400 MHz, CDCl₃): 1.35 (m, 6H, CH₃), 1.49 (m, 6H, CH₃), 3.17 (t, 2H, CH₂, *J*=8.0 Hz), 4.26 (m, 2H, CH₂), 4.51 (m, 4H, CH₂), 6.13 (s, 1H, CH), 6.44(s, 1H, CH), 7.06 (m, 3H, ArH), 7.19 (m, 2H, ArH), 7.38 (m, 3H, ArH).
¹³C-NMR (100 MHz, CDCl₃): 14.30, 15.49, 16.60, 29.52, 42.27, 48.05, 55.30, 91.40, 101.51, 110.41, 112.70, 115.53, 117.02, 120.91, 125.37, 131.15, 138.80, 141.64, 143.03, 156.15, 173.70.
HRMS-ESI: *m*/*z* calcd M⁺ for C₂₈H₃₀N₃OS₂⁺, 488.1825; found, 488.1828.
**IX₂a**: HRMS-ESI: *m*/*z* calcd [M+Na]⁺ for C₂₉H₂₉N₃NaO₃S₂⁺, 554.1543; found, 554.1547.
**IX₃a**: HRMS-ESI: *m*/*z* calcd M⁺ for C₃₃H₃₂N₃OS₂⁺, 550.1981; found, 550.1985.
**IX₄a**: HRMS-ESI: *m*/*z* calcd M⁺ for C₃₃H₃₁N₄O₃S₂⁺, 595.1832; found, 595.1838.
**IX₅a**: HRMS-ESI: *m*/*z* calcd M⁺ for C₄₀H₄₄N₅O₂S₂⁺, 690.2931; found, 690.2936.
**IX₆a**: HRMS-ESI: *m*/*z* calcd M⁺ for C₄₁H₄₆BN₄O₄S₂⁺, 733.3048; found, 733.3053.

2. The synthetic route of dye IX₇a was shown as follows: 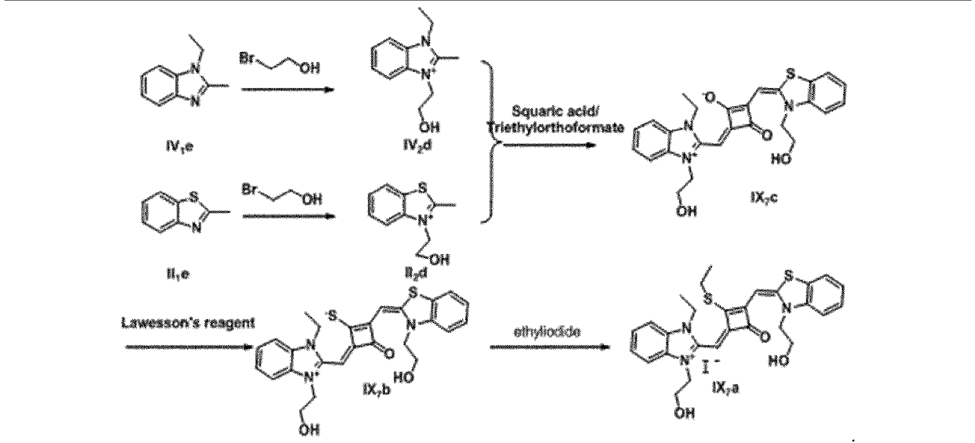
(1) 1 mol of 2-methyl-N-ethylbenzimidazole (IV₁e) and 4 mol of 2-bromoethanol were added to 20 mL of dry 1,2-dichlorobenzene. The reaction mixture was heated and refluxed under nitrogen protection for 36 h. After cooled to room temperature, the reaction mixture was washed with anhydrous diethyl ether and recrystallized with propanone to give a white to dark gray quaternary ammonium salt solid (IV₂d) which was dried and stored for use.
(2) 1 mol of 2-methylbenzothiazole (II₁e) and 4 mol of 2-bromoethanol were added to 20 mL of dry 1,2-dichlorobenzene. The reaction mixture was heated under nitrogen protection for 36 h. After cooled to room temperature, the reaction mixture was washed with anhydrous diethyl ether and recrystallized with propanone to give a white to gray quaternary ammonium salt solid (II₂d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (IV₂d) and 1 mol of the quaternary ammonium salt (II₂d) were added. The reaction mixture was continuously refluxed until the reaction was confirmed by TLC to be completed. Then the reaction mixture was cooled, dried under reduced pressure to remove the solvent, and purified by column chromatography to give a blue solid dye (IX₇c).
(4) 1 mol of the dye (IX₇c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IX₇b).
(5) 1 mol of the sulfur-substituted squaraine dye (IX₇b) was added to dry acetonitrile, to which 2.5 mol of an iodoethane intermediate was added. The reaction mixture was stirred at room temperature for 1 h. After the reaction was confirmed by TLC to be completed, the reaction mixture was cooled to room temperature, dried under vacuum, and purified by column chromatography to give a target dye (**IX₇a**).

The compound **IX₇a** was characterized as follows:
HRMS-ESI: *m*/*z* calcd M⁺ for C₂₈H₃₀N₃O₃S₂⁺, 520.1723; found, 520.1727.

3. The synthetic route of dye IX₈a was shown as follows: 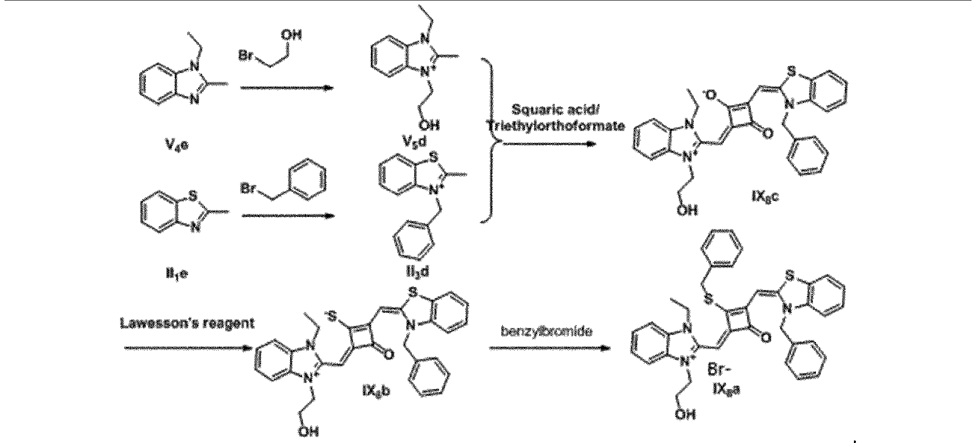
(1) 1 mol of 2-methyl-N-ethylbenzimidazole (V₄e) and 3 mol of 2-bromoethanol were added to 20 mL of dry 1,2-dichlorobenzene. The reaction mixture was heated and refluxed under nitrogen protection for 36 h. After cooled to room temperature, the reaction mixture was washed with anhydrous diethyl ether and recrystallized with propanone to give a gray quaternary ammonium salt solid (V₅d) which was dried and stored for use.
(2) 1 mol of 2-methylbenzothiazole (II₁e) and 2 mol of benzyl bromide were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white quaternary ammonium salt solid (II₃d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₅d) and 1 mol of the quaternary ammonium salt (II₃d) were added. The reaction mixture was continuously refluxed until the reaction was confirmed by TLC to be completed. Then the reaction mixture was cooled, dried under reduced pressure to remove the solvent, and purified by column chromatography to give a blue solid dye (IX₈c).
(4) 1 mol of the dye (IX₈c) was added to 25 mL of a mixed solvent of dry dichloromethane and tetrahydrofuran. After the solid dye was completely dissolved, 2 mol of Lawesson's reagent was added. After stirred at 40°C for 3 h, the reaction mixture was dried under vacuum, and purified by column chromatography to give a bluish green centrally sulfur-substituted squaraine dye (IX₈b).
(5) 1 mol of the sulfur-substituted squaraine dye (IX₈b) was added to dry acetonitrile, to which 2.5 mol of an iodoethane intermediate was added. The reaction mixture was stirred under heating until the reaction was confirmed by TLC to be completed. Then the reaction mixture was cooled to room temperature, dried under vacuum, and purified by column chromatography to give a corresponding target dye (**IX₈a**).

The compound **IX₈a** was characterized as follows:
HRMS-ESI: *m*/*z* calcd M⁺ for C₂₈H₃₀N₃O₃S₂⁺, 520.1723; found, 520.1725.

4. The synthetic route of dye IX₉a was shown as follows: 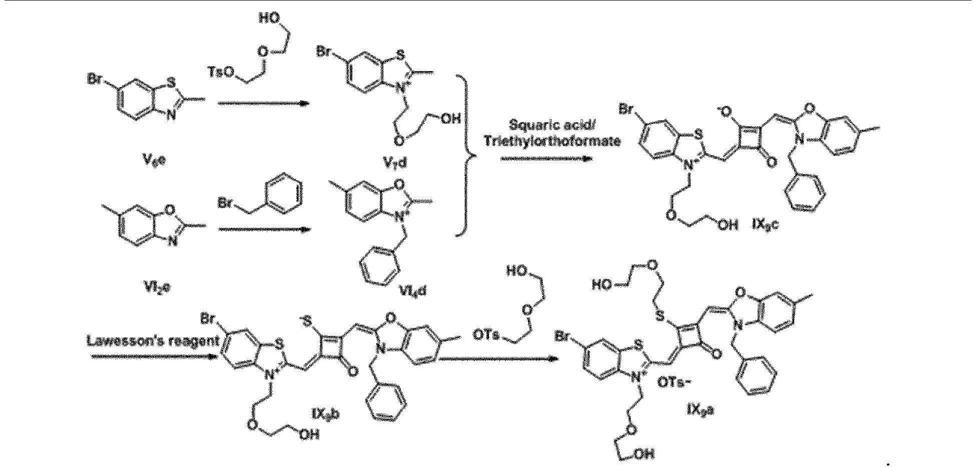
(1) 1 mol of 2,5-dimethyl-N-ethylbenzimidazole (V₆e) and 3 mol of benzyl bromide were added to 20 mL of dry 1,2-dichlorobenzene. The reaction mixture was heated and refluxed under nitrogen protection for 30 h. After cooled to room temperature, the reaction mixture was washed with anhydrous diethyl ether and recrystallized with propanone to give a white quaternary ammonium salt solid (V₇d) which was dried and stored for use.
(2) 1 mol of 2-methyl-5-bromobenzothiazole (VI₂e) and 3 mol of ethanol, 2-(2-hydroxyethoxy)-, 1-(4-Methylbenzenesulfonate) were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₄d) which was dried and stored for use.
(3) 2 mol of squaric acid was added to 100 mL of absolute ethanol, to which triethyl orthoformate was added as a catalyst. The reaction mixture was refluxed under nitrogen protection until the squaric acid was completely dissolved, and then 1 mol of the quaternary ammonium salt (V₇d) and 1 mol of the quaternary ammonium salt (II₄d) were added. The reaction mixture was continuously refluxed until the reaction was confirmed by TLC to be completed. Then the reaction mixture was cooled, dried under reduced pressure to remove the solvent, and purified by column chromatography to give a blue solid dye (IX₉c).
(4) 1 mol of the dye (IX₉c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IX₉b).
(5) 1 mol of the sulfur-substituted squaraine dye (IX₉b) was added to dry acetonitrile, to which 2.5 mol of 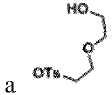 intermediate was added. The reaction mixture was stirred under heating until the reaction was confirmed by TLC to be completed. Then the reaction mixture was cooled to room temperature, dried under vacuum, and purified by column chromatography to give a corresponding target dye (**IX₉a**).

The compound **IX₉a** was characterized as follows:
¹H-NMR (400 MHz, CDCl₃): 1.31 (t, 3H, CH₃, *J*=8.0 Hz), 2.36 (s, 3H, CH₃), 3.01(t, 2H, CH₂, *J*=8.0 Hz), 3.55 (m, 14H, CH₂&OH), 4.08 (m, 4H, CH₂), 5.51 (s, 2H, CH₂), 6.15 (s, 1H, CH), 6.41(s, 1H, CH), 7.08 (m, 3H, ArH), 7.21 (m, 5H, ArH), 7.50 (m, 3H, ArH).
¹³C-NMR (100 MHz, CDCl₃): 14.80, 21.52, 33.27, 44.05, 53.30, 52.02, 61.33, 66.14, 70.71, 70.45, 89.83, 100.14, 109.73, 112.80, 113.65, 115.12, 117.74, 118.60, 120.59, 122.81, 125.53, 128.81, 131.37, 133.31, 138.08, 139.75, 141.64, 143.90, 153.05, 154.72, 174.49.
HRMS-ESI: *m*/*z* calcd M⁺ for C₃₈H₄₁BrN₃O₅S₂⁺, 762.1666; found, 762.1671.

5. The synthetic route of dye IX₁₀a was shown as follows: 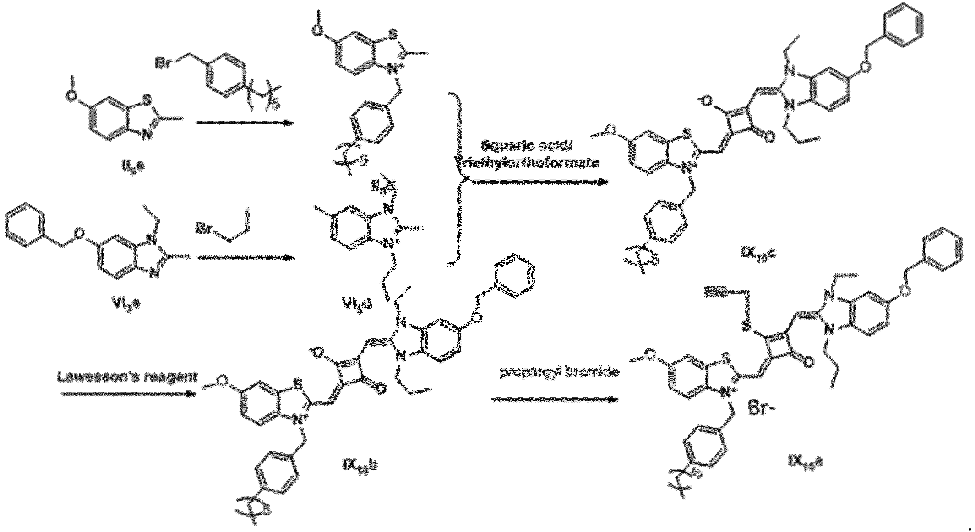
(1) 1 mol of 2-methyl-5-methoxybenzothiazole (II₈e) and 2 mol of *p*-hexylbenzyl bromide were added to 20 mL of dry 1,2-dichlorobenzene, to which potassium iodide was added as a catalyst. Then the reaction mixture was heated and refluxed under nitrogen protection for 36 h. After cooled to room temperature, the reaction mixture was washed with anhydrous diethyl ether and recrystallized with propanone to give a dark gray quaternary ammonium salt solid (II₉d) which was dried and stored for use.
(2) 1 mol of 2-methyl-5-benzyloxybenzoxazole (VI₃e) and 3 mol of bromopropane were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white to pink quaternary ammonium salt solid (VI₅d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (II₉d) and 1 mol of the quaternary ammonium salt (VI₅d) were added. The reaction mixture was continuously refluxed until the reaction was confirmed by TLC to be completed. Then the reaction mixture was cooled, dried under reduced pressure to remove the solvent, and purified by column chromatography to give a blue solid dye (IX₁₀c), which was dried and stored for use.
(4) 1 mol of the dye (IX₁₀c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (IX₁₀b).
(5) 1 mol of the sulfur-substituted squaraine dye (IX₁₀b) was added to dry acetonitrile, to which 2.5 mol of a propargyl bromide intermediate was added. The reaction mixture was stirred under heating until the reaction was confirmed by TLC to be completed. Then the reaction mixture was cooled to room temperature, dried under vacuum, and purified by column chromatography to give a target dye (**IX₁₀a**).

The compound **IX₁₀a** was characterized as follows:
HRMS-ESI: *m*/*z* calcd M⁺ for C₄₉H₅₂N₃O₃S₂⁺, 794.3445; found, 794.3449.

### Example 10

The asymmetric dyes **X₁a to X₁₀a** with X= NCH₂CH₃ and Y= O in formula (I) were shown as follows, wherein formulae X₂a, X₄a, X₅a, X₆a, X₇a, X₈a, X₉a, X₁₀a are non-inventive embodiments, not falling under the scope of the claims: 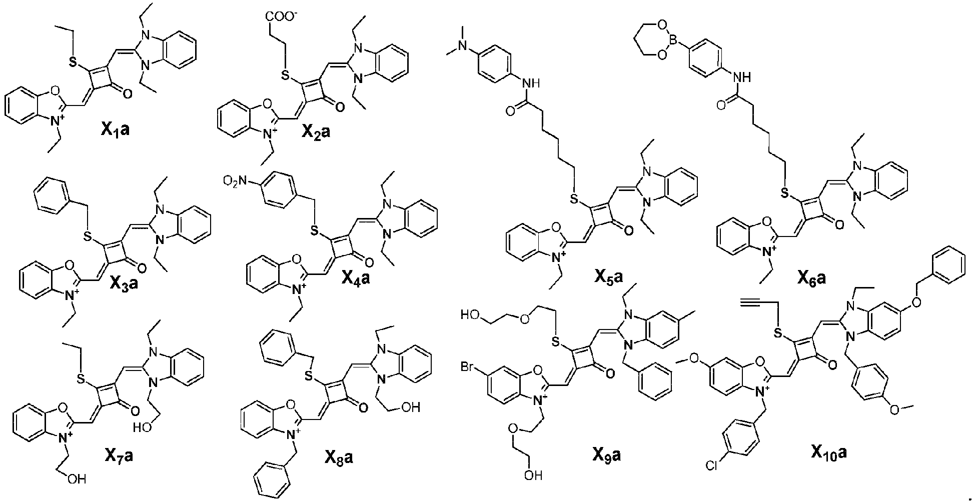
1. The synthetic route of dyes X₁a to X₆a was shown as follows: 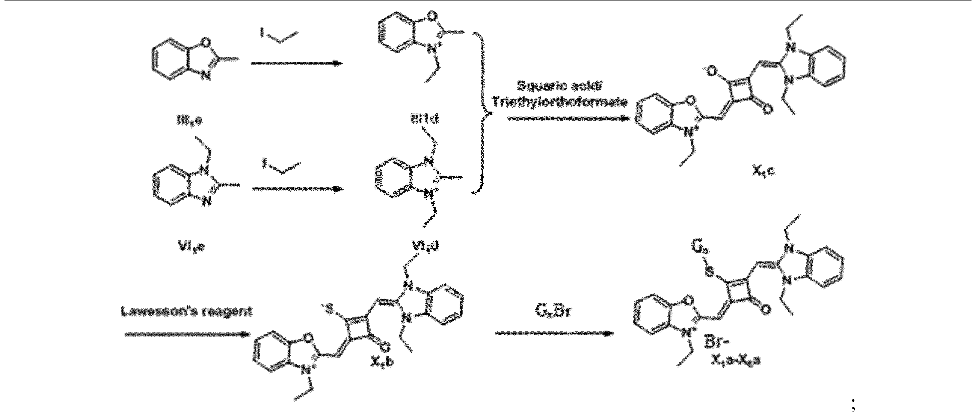 wherein, the structural formulas of Gₙ (n=1-6) were respectively shown as follows: 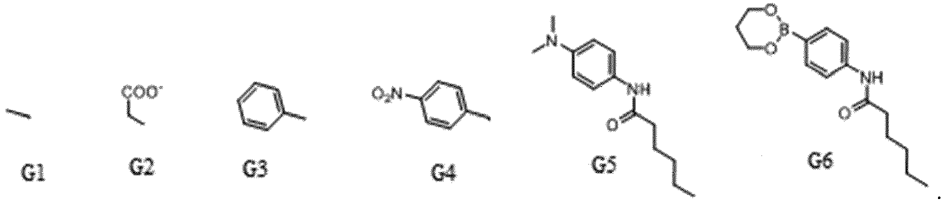
   (1) 1 mol of 2-methyl-N-ethylbenzimidazole (VI₁e) and 3 mol of iodoethane were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (VI₁d) which was dried and stored for use.
   (2) 1 mol of 2-methylbenzoxazole (III₁e) and 3 mol of iodoethane were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (III₁d) which was dried and stored for use.
   (3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₁d) and 1 mol of the quaternary ammonium salt (VI₁d) were added. The reaction mixture was continuously refluxed until the reaction was confirmed by TLC to be completed. Then the reaction mixture was cooled, dried under reduced pressure to remove the solvent, and purified by column chromatography to give a blue solid dye (X₁c).
   (4) 1 mol of the dye (X₁c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (X₁b).
   (5) 1 mol of the sulfur-substituted squaraine dye (X₁b) was added to dry acetonitrile, to which 2.5 mol of a bromo-substituted intermediate GₙBr (Gₙ was selected from G₁ to G₆) was added. The reaction mixture was stirred at room temperature or under heating until the reaction was confirmed by TLC to be completed. Then the reaction mixture was cooled to room temperature, dried under vacuum, and purified by column chromatography to give a corresponding target dye (**X₁a to X₆a**).

The compounds **X₁a to X₆a** were characterized as follows:
**X₁a**: ¹H-NMR (400 MHz, CDCl₃): 1.32 (m, 6H, CH₃), 1.46 (m, 6H, CH₃), 3.16(q, 2H, CH₂, *J*=8.0 Hz), 4.25(q, 2H, CH₂, *J*=8.0 Hz), 4.55 (m, 4H, CH₂), 6.11 (s, 1H, CH), 6.41(s, 1H, CH), 7.11 (m, 2H, ArH), 7.24 (m, 3H, ArH), 7.47 (m, 3H, ArH).
¹³C-NMR (100 MHz, CDCl₃): 14.17, 15.20, 16.35, 29.52, 44.27, 50.19, 51.08, 88.50, 100.37, 109.01, 113.80, 114.49, 116.60, 117.93, 121.24, 122.80, 128.17, 133.05, 137.74, 142.28, 153.30, 173.91.
HRMS-ESI: *m*/*z* calcd M⁺ for C₂₈H₃₀N₃O₂S⁺, 472.2053; found, 472.2057.
**X₂a**: HRMS-ESI: *m*/*z* calcd [M+Na]⁺ for C₂₉H29N3NaO4S, 538.1771; found, 538.1775.
**X₃a**: HRMS-ESI: *m*/*z* calcd M⁺ for C₃₃H₃₂N₃O₂S⁺, 534.2210; found, 534.2215.
**X₄a**: HRMS-ESI: *m*/*zcalcd* M⁺ for C₃₃H₃₁N₄O₄S⁺, 579.2061; found, 579.2066.
**X₅a**: HRMS-ESI: *m*/*zcalcd* M⁺ for C₄₀H₄₆N₅O₃S⁺, 676.3316; found, 676.3320.
**X₆a**: HRMS-ESI: *m*/*zcalcd* M⁺ for C₄₁H₄₆BN₄O₅S⁺, 717.3276; found, 717.3281.

2. The synthetic route of dye X₇a was shown as follows: 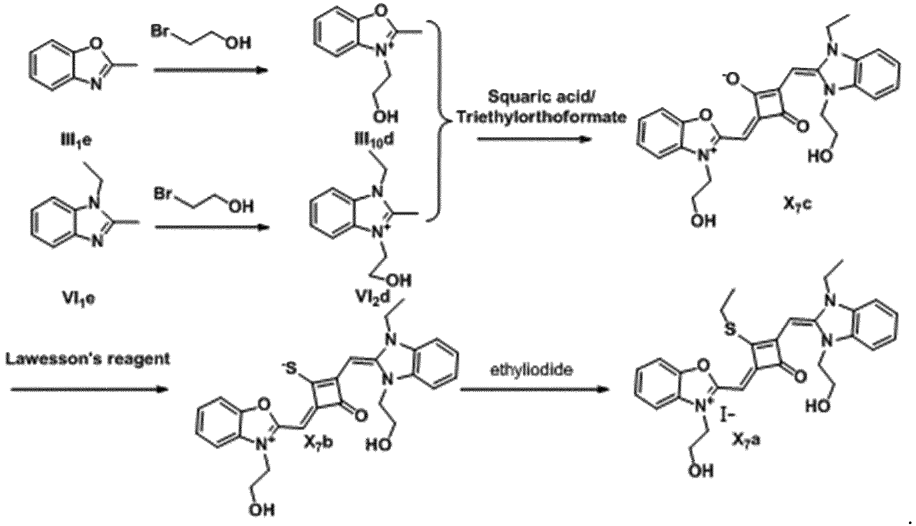
(1) 1 mol of 2-methyl-N-ethylbenzimidazole (VI₁e) and 3 mol of 2-bromoethanol were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (VI₂d) which was dried and stored for use.
(2) 1 mol of 2-methylbenzoxazoic (III₁e) and 3 mol of 2-bromoethanol were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (III₁₀d) which was dried and stored for use.
(3) 2 mol of squaric acid was added to 100 mL of absolute ethanol, to which triethyl orthoformat was added as a catalyst. The reaction mixture was heated and refluxed under nitrogen protection until the squaric acid was completely dissolved, and then 1 mol of the quaternary ammonium salt (III₁₀d) and 1 mol of the quaternary ammonium salt (VI₂d) were added. The reaction mixture was continuously refluxed until the reaction was confirmed by TLC to be completed. Then the reaction mixture was cooled, dried under reduced pressure to remove the solvent, and purified by column chromatography to give a blue solid dye (X₇c).
(4) 1 mol of the dye (X₇c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (X₇b).
(5) 1 mol of the sulfur-substituted squaraine dye (X₇b) was added to dry acetonitrile, to which 2.5 mol of an iodoethane intermediate was added. The reaction mixture was stirred under heating until the reaction was confirmed by TLC to be completed. Then the reaction mixture was cooled to room temperature, dried under vacuum, and purified by column chromatography to give a target dye (**X₇a**).

The compound **X₇a** was characterized as follows:
HRMS-ESI: *m*/*zcalcd* M⁺ for C₂₈H₃₀N₃O₄S⁺, 504.1952; found, 504.1956.

3. The synthetic route of dye X₈a was shown as follows: 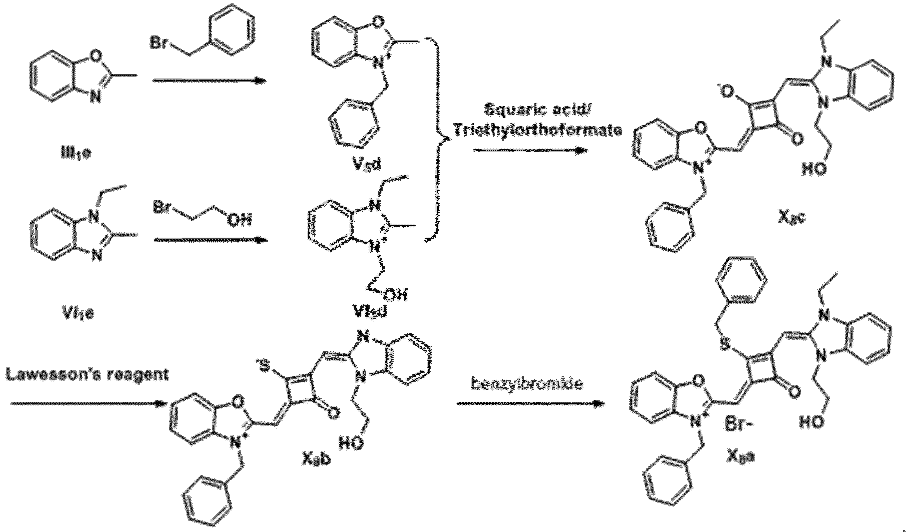
(1) 1 mol of 2-methyl-N-ethylbenzimidazole (VI₁e) and 3 mol of 2-bromoethanol were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (VI₂d) which was dried and stored for use.
(2) 1 mol of 2-methylbenzothiazole (III₁e) and 2 mol of benzyl bromide were added to 20 mL of dry toluene. The reaction mixture was heated under nitrogen protection for 24 h. After cooled to room temperature, the reaction mixture was washed with anhydrous diethyl ether and recrystallized with propanone to give a white to pink quaternary ammonium salt solid (V₅d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (V₅d) and 1 mol of the quaternary ammonium salt (VI₃d) were added. The reaction mixture was continuously refluxed until the reaction was confirmed by TLC to be completed. Then the reaction mixture was cooled, dried under reduced pressure to remove the solvent, and purified by column chromatography to give a blue solid dye (X₈c).
(4) 1 mol of the dye (X₈c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (X₈b).
(5) 1 mol of the sulfur-substituted squaraine dye (X₈b) was added to dry acetonitrile, to which 2.5 mol of a benzyl bromide intermediate was added. The reaction mixture was stirred under heating until the reaction was confirmed by TLC to be completed. Then the reaction mixture was cooled to room temperature, dried under vacuum, and purified by column chromatography to give a target dye (X₈a).

The compound X₈a was characterized as follows:
HRMS-ESI: *m*/*zcalc*d M⁺ for C₃₈H₃₄N₃O₃S⁺, 612.2315; found, 612.2320.

4. The synthetic route of dye X₉a was shown as follows: 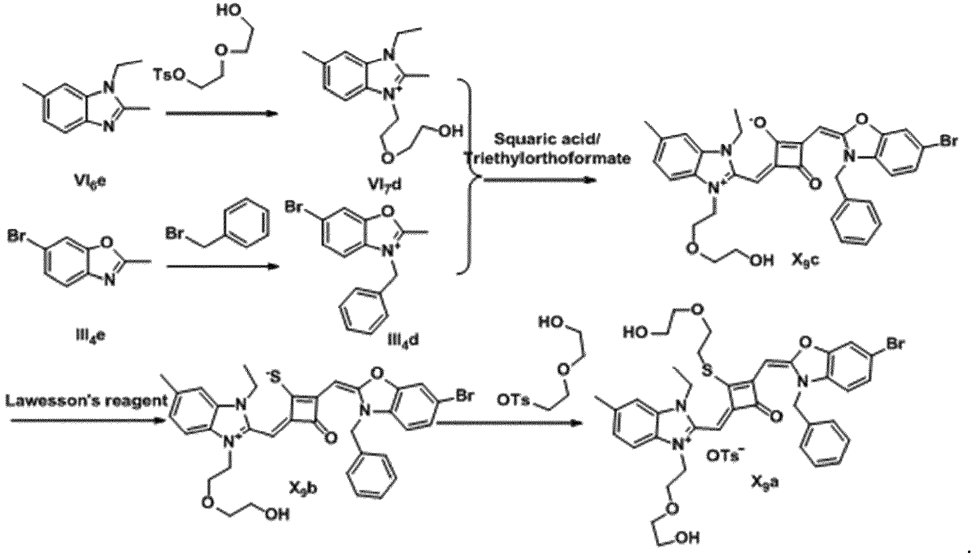
(1) 1 mol of 2,5-dimethyl-N-ethylbenzimidazole (VI₆e) and 3 mol of benzyl bromide were added to 20 mL of dry toluene. The reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (VI₇d) which was dried and stored for use.
(2) 1 mol of 2-methyl-5-bromobenzoxazole (III₄e) and 3 mol of ethanol, 2-(2-hydroxyethoxy)-, 1-(4-Methylbenzenesulfonate) were added to 20 mL of dry 1,2-dichlorobenzene. The reaction mixture was heated under nitrogen protection for 12 h. After cooled to room temperature, the reaction mixture was washed with anhydrous diethyl ether and recrystallized with propanone to give a white to pink quaternary ammonium salt solid (III₄d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₄d) and 1 mol of the quaternary ammonium salt (VI₇d) were added. The reaction mixture was continuously refluxed until the reaction was confirmed by TLC to be completed. Then the reaction mixture was cooled, dried under reduced pressure to remove the solvent, and purified by column chromatography to give a blue solid dye (Xgc).
(4) 1 mol of the dye (X₉c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (Xgb).
(5) 1 mol of the sulfur-substituted squaraine dye (Xgb) was added to dry acetonitrile, to which 2.5 mol of a diethylene glycol monobenzenesulfonate intermediate was added. The reaction mixture was stirred under heating until the reaction was confirmed by TLC to be completed. Then the reaction mixture was cooled to room temperature, dried under vacuum, and purified by column chromatography to a give target dye (X₉a).

The compound X₉a was characterized as follows:
¹H-NMR (400 MHz, CDCl₃): 1.31 (t, 3H, CH₃, 1=8.0 Hz), 2.36 (s, 3H, CH₃), 3.03(t, 2H, CH₂, *J*=8.0 Hz), 3.70(m, 12H, CH₂&OH), 4.01 (t, 2H, CH₂, *J*=8.0 Hz), 4.22 (q, 2H, CH₂, *J*=8.0 Hz), 6.09 (s, 1H, CH), 6.42(s, 1H, CH), 7.12 (m, 3H, ArH), 7.29 (m, 5H, ArH), 7.50 (m, 3H, ArH).
¹³C-NMR (100 MHz, CDCl₃): 14.37, 21.20, 33.35, 44.03, 49.75, 53.29, 61.13, 67.02, 70.50, 73.07, 89.63, 99.40, 110.05, 113.43, 114.69, 116.51, 117.07, 121.25, 122.91, 125.12, 128.06, 129.77, 133.53, 135.08, 138.47, 142.48, 153.35, 174.90.
HRMS-ESI: *m*/*zcalcd* M⁺ for C₃₈H₄₁BrN₃O₆S⁺, 746.1894; found, 746.1898.

5. The synthetic route of dye X₁₀a was shown as follows: 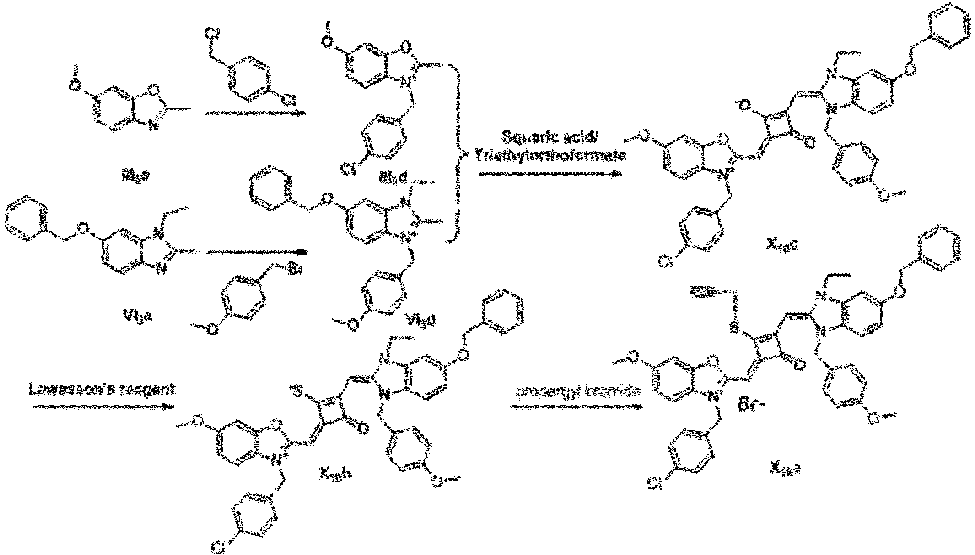
(1) 1 mol of 2-methyl-5-methoxybenzoxazole (III₆e) and 3 mol of 4-chlorobenzyl chloride were added to 20 mL of dry 1,2-dichlorobenzene. The reaction mixture was heated under nitrogen protection for 24 h. After cooled to room temperature, the reaction mixture was washed with anhydrous diethyl ether and recrystallized with propanone to give a white quaternary ammonium salt solid (III₉d) which was dried and stored for use.
(2) 1 mol of N-ethyl-2-methyl-5-benzyloxy-3H-indoline (VI₃e) and 2 mol of p-methoxybenzyl bromide were added to 20 mL of dry 1,2-dichlorobenzene, to which potassium iodide was added as a catalyst. Then the reaction mixture was heated and refluxed under nitrogen protection for 24 h. After cooled to room temperature, the reaction mixture was washed with anhydrous diethyl ether and recrystallized with propanone to give a white to pink quaternary ammonium salt solid (VI₅d) which was dried and stored for use.
(3) 2 mol of squaric acid was added to 100 mL of absolute ethanol, to which triethyl orthoformate was added as a catalyst. The reaction mixture was heated and refluxed under nitrogen protection until the squaric acid was completely dissolved, and then 1 mol of the quaternary ammonium salt (III₉d) and 1 mol of the quaternary ammonium salt (VI₅d) were added. The reaction mixture was continuously refluxed until the reaction mixture was confirmed by TLC to be completed. Then the reaction mixture was cooled, dried under reduced pressure to remove the solvent, and purified by column chromatography to give a blue solid dye (X₁₀c).
(4) 1 mol of the dye (X₁₀c) was added to 25 mL of a mixed solvent of dry dichloromethane and tetrahydrofuran, to which 2 mol of Lawesson's reagent was added. After stirred at 40°C for 3 h, the reaction mixture was dried under vacuum, and purified by column chromatography to give a bluish green centrally sulfur-substituted squaraine dye (X₁₀b).
(5) 1 mol of the sulfur-substituted squaraine dye (X₁₀b) was added to dry acetonitrile, to which 2.5 mol of a propargyl bromide intermediate was added. The reaction mixture was stirred under heating until the reaction was confirmed by TLC to be completed. Then the reaction mixture was cooled to room temperature, dried under vacuum, and purified by column chromatography to give a target dye (X₁₀a).

The compound **X₁₀a** was characterized as follows:
HRMS-ESI: *m*/*zcalcd* M⁺ for C₄₈H₄₁ClN₃O₅S⁺, 806.2450; found, 806.2454.

### Example 11

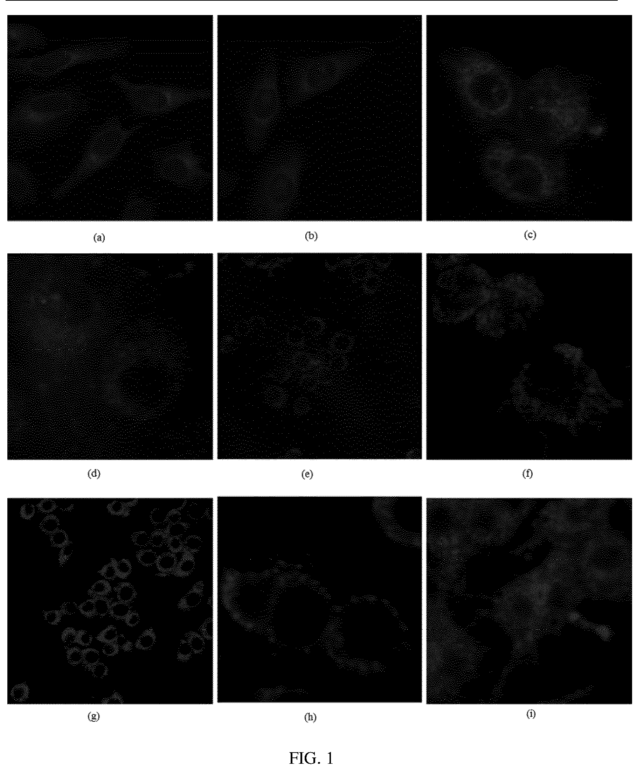

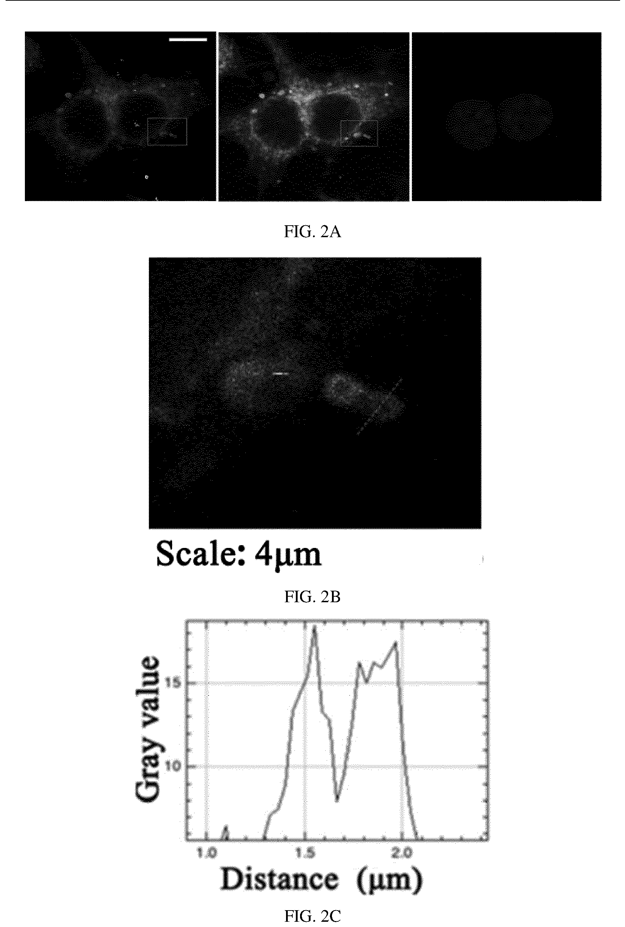

Ten representative dyes (shown as the following formulas I₀a to X₀a) respectively from the above Examples 1-10 were tested for the living cell membrane permeability, intracellular localization ability, fluorescence imaging and super-resolution imaging effect. The experiment was specifically described as follows, wherein formulae I₀a, II₀a, III₀a, IV₀a, V₀a, VI₀a, VII₀a, VIII₀a, IX₀a, X₀a are non-inventive embodiments, not falling under the scope of the claims.
1. The ten dyes shown as I₀a to X₀a were accurately weighed separately, and then dissolved in DMSO solvent to prepare a corresponding 1 mM dye solution (mother liquor), which was stored in a refrigerator for
2. After macrophages were resuscitated and passaged according to standard experimental methods, the previously prepared dye solutions were respectively added to the cell suspension to a final concentration of 1 µM. After cultured at 37°C and 5% carbon dioxide for 30 min, the cells were observed under a laser confocal microscope, where the excitation wavelength was selected to 633 nm, and the fluorescence generated under 645 nm or higher was collected. The results of the living cell confocal laser scanning imaging involving the above ten representative dyes were shown in Fig. 1, wherein, (a)-I₀a; (b)-II₀a; (c)-III₀a; (d)-IV₀a; (e)-V₀a; (f)-VI₀a; (g)-VII₀a; (h)-VIII₀a; (i)-IX₀a.
3. The dyes IX₀a and X₀a were simultaneously used for the STED super-resolution imaging of macrophages, and the results were shown in Figs. 2A-2E, where Fig.2A:IX₀a; and Fig.2B: X₀a.

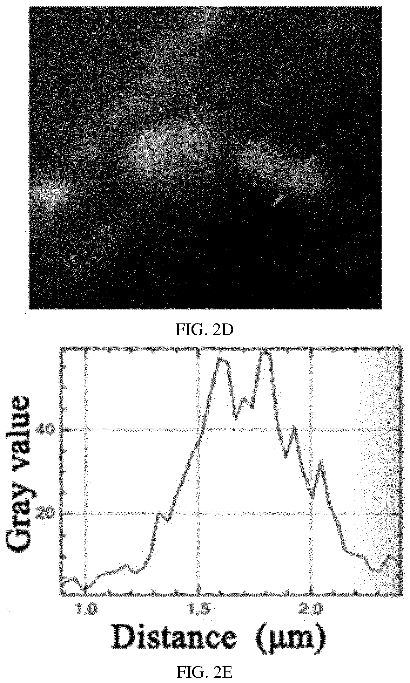

It can be seen from Figs. 1 and 2 that the dyes of the invention had good membrane permeability and localization ability in living cells, and thus they are suitable for the positioning imaging and STED super-resolution imaging in living cells.

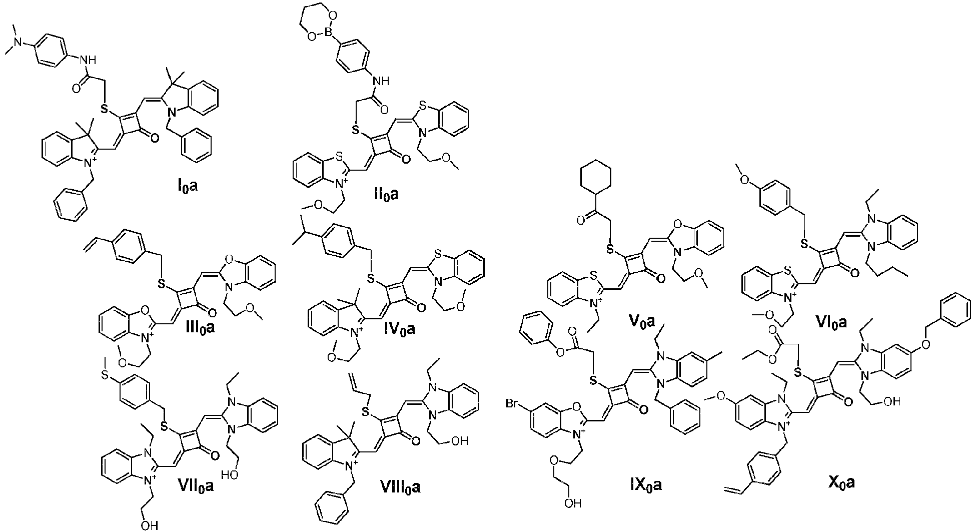

## Claims

1. A fluorescent dye of formula (I): 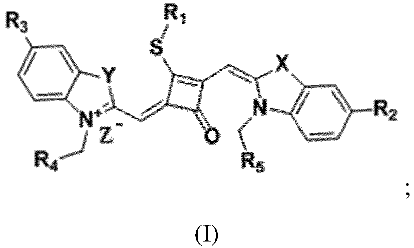
wherein X and Y are independently selected from O, S, C(CH₃)₂ or NR₆;
R₁ is (CH₂)ₙ₋₁CH₃, (CH₂)ₙR₇, (CH₂)ₘC₆H₅ or (CH₂)ₘC₆H₄R₇;
R₂ and R₃ are independently selected from H, F, Cl, Br, I, (CH₂)ₙ₋₁CH₃, (CH₂)ₙR₈, CH₂C₆H₅, CH₂C₆H₄R₈, O(CH₂)ₙ₋₁CH₃, O(CH₂)ₙR₈, OCH₂C₆H₅, OCH₂C₆H₄R₈ and CN,
R₄, R₅ and R₆ are independently selected from (CH₂)ₙ₋₁CH₃, (CH₂)ₙR₈, CH₂C₆H₅ and CH₂C₆H₄R₈; wherein R₇ is C₆H₅, C₆H₄R₉, SO₃R₁₀ or COOR₁₁;
R₈ is SO₃R₁₀ or COOR₁₁; n and m are integers respectively selected from 1-18 and 0-18;
R₉ is arsonic acid, boric acid, carboxylic acid, sulfonic acid, amino, hydroxyl, sulfhydryl or C₁-C₁₈ alkyl;
R₁₀ is N(R₁₂R₁₃R₁₄R₁₅); R₁₁ is a C₁-C₁₈ alkyl; R₁₂, R₁₃, R₁₄ and R₁₅ are independently selected from H, C₁-C₁₈ alkyl and (CH₂)ₚOH, and p is an integer selected from 0-18, and
Z⁻ is a halide anion or OTs⁻.

2. A method of preparing the fluorescent dye of claim 1, comprising:
preparing a compound of formula (III) and Lawesson's reagent;
dissolving the compound of formula (III) and the Lawesson's reagent in a first solvent; and subjecting the reaction mixture to substitution reaction under heating in an inert gas to produce a compound of formula (II); and
subjecting the compound of formula (II) and a nucleophilic reagent R₁Z to addition reaction to produce the fluorescent dye of formula (I); 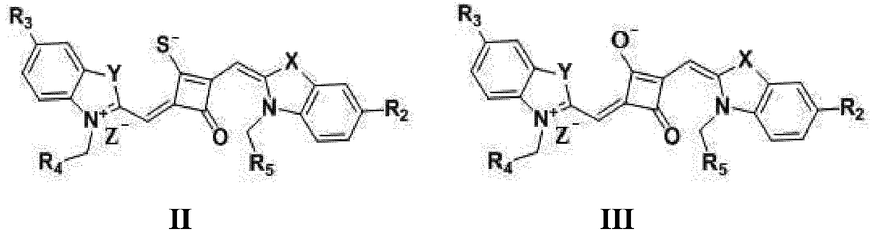
wherein X and Y are independently selected from O, S, C(CH₃)₂ and NR₆;
R₁ is (CH₂)ₙ₋₁CH₃, (CH₂)ₙR₇, (CH₂)ₘC₆H₅ or (CH₂)ₘC₆H₄R₇;
R₂ and R₃ are independently selected from H, F, Cl, Br, I, (CH₂)ₙ₋₁CH₃, (CH₂)nR8, CH₂C₆H₅, CH₂C₆H₄R₈, O(CH₂)ₙ₋₁CH₃, O(CH₂)ₙR₈, OCH₂C₆H₅, OCH₂C₆H₄R₈ and CN;
R₄, R₅ and R₆ are independently selected from (CH₂)ₙ₋₁CH₃, (CH₂)ₙR₈, CH₂C₆H₅ and CH₂C₆H₄R₈; wherein R₇ is C₆H₅, C₆H₄R₉, SO₃R₁₀ or COOR₁₁; R₈ is SO₃R₁₀ or COOR₁₁; n and m are integers respectively selected from 1-18 and 0-18;
R₉ is arsonic acid, boric acid, carboxylic acid, sulfonic acid, amino, hydroxyl, sulfhydryl or C₁-C₁₈ alkyl;
R₁₀ is N(R₁₂R₁₃R₁₄R₁₅);
R₁₁ is a C₁-C₁₈ alkyl;
R₁₂, R₁₃, R₁₄ and R₁₅ are independently selected from H, C₁-C₁₈ alkyl and (CH₂)ₚOH, and p is an integer selected from 0-18; and
Z- is a halide anion or OTs⁻.

3. The method of claim 2, wherein the first solvent is a mixed solution of dichloromethane and anhydrous tetrahydrofuran; and/or
a temperature of the substitution reaction is in a range of from 40°C to 50°C; and/or
a temperature of the addition reaction is in a range of from 25°C to 60°C; and/or
a molar ratio of the compound of formula (III) to the Lawesson's reagent is 1:1; and/or
a molar ratio of the compound of formula (II) to the nucleophilic reagent R₁Z is 1: 2 to 5.

4. The method of claim 2, wherein the compound of formula (III) is prepared by the steps of:
preparing a compound of formula (VI) and a compound of formula (VII);
subjecting the compound of formula (VI) and a nucleophilic reagent R₄Z to addition reaction to produce a compound of formula (V);
subjecting the compound of formula (VII) and a nucleophilic reagent of R₅Z to addition reaction to produce a compound of formula (IV);
mixing the compound of formula (V) and the compound of formula (IV) with a squaric acid solution, and reacting the reaction mixture in the presence of a catalyst to obtain the compound of formula (III);

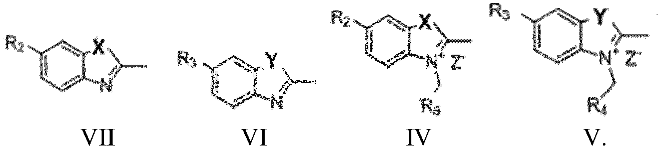

5. The method of claim 4, wherein a solvent in the squaric acid solution is ethanol; and/or
the catalyst is triethyl orthoformate; and/or
a molar ratio of the compound of formula (IV) to the compound of formula (V) to the squaric acid is 1: 0.8 to 1.2 : 0.8 to1.2.

6. An application of the fluorescent dye of claim 1, comprising:
applying the fluorescent dye in the fluorescence imaging of living cells and the fluorescent labeling of intracellular microstructure.

## Patentansprüche

1. Fluoreszenzfarbstoff der Formel (I): 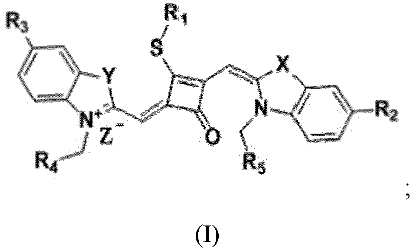
wobei X und Y unabhängig ausgewählt sind aus O, S, C(CH₃)₂ oder NR₆;
R₁ (CH₂)ₙ₋₁CH₃, (CH₂)ₙR₇, (CH₂)ₘC₆H₅ oder (CH₂)ₘC₆H₄R₇ ist;
R₂ und R₃ unabhängig ausgewählt sind aus H, F, Cl, Br, I, (CH₂)ₙ₋₁CH₃, (CH₂)ₙR₈, CH₂C₆H₅, CH₂C₆H₄R₈, O(CH₂)ₙ₋₁CH₃, O(CH₂)ₙR₈, OCH₂C₆H₅, OCH₂C₆H₄R₈ und CN;
R₄, R₅ und R₆ unabhängig ausgewählt sind aus (CH₂)ₙ₋₁CH₃, (CH₂)ₙR₈, CH₂C₆H₅ und CH₂C₆H₄R₈; wobei R₇ C₆H₅, C₆H₄R₉, SO₃R₁₀ oder COOR₁₁ ist;
R₈ SO₃R₁₀ oder COOR₁₁ ist; n und m Ganzzahlen sind, ausgewählt aus 1-18 bzw. 0-18;
R₉ Arsonsäure, Borsäure, Carbonsäure, Sulfonsäure, Amino, Hydroxyl, Sulfhydryl oder C₁-C₁₈-Alkyl ist;
R₁₀ N(R₁₂R₁₃R₁₄R₁₅) ist;
R₁₁ ein C₁-C₁₈-Alkyl ist;
R₁₂, R₁₃, R₁₄ und R₁₅ unabhängig ausgewählt sind aus H, C₁-C₁₈-Alkyl und (CH₂)ₚOH, und p eine Ganzzahl ausgewählt aus 0-18 ist; und
Z⁻ ein Halogenidanion oder OTs⁻ ist.

2. Verfahren zur Herstellung des Fluoreszenzfarbstoffs nach Anspruch 1, umfassend:
Herstellen einer Verbindung der Formel (III) und eines Lawessons Reagenz;
Lösen der Verbindung der Formel (III) und des Lawessons Reagenz in einem ersten Lösungsmittel; und Unterziehen des Reaktionsgemischs einer Substitutionsreaktion unter Erhitzen in einem Inertgas zur Herstellung einer Verbindung der Formel (II); und
Unterziehen der Verbindung der Formel (II) und eines nucleophilen Reagenz R₁Z einer Additionsreaktion zur Herstellung des Fluoreszenzfarbstoff der Formel (I); 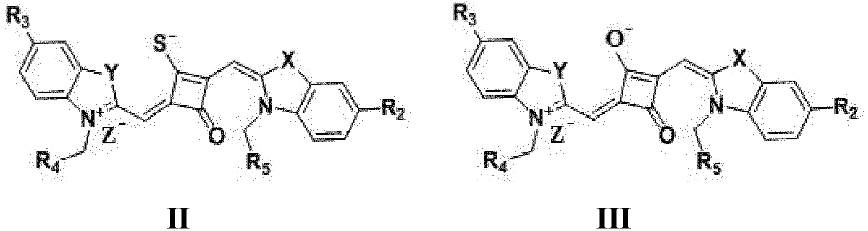
wobei X und Y unabhängig ausgewählt sind aus O, S, C(CH₃)₂ oder NR₆;
R₁ (CH₂)ₙ₋₁CH₃, (CH₂)ₙR₇, (CH₂)ₘC₆H₅ oder (CH₂)ₘC₆H₄R₇ ist;
R₂ und R₃ unabhängig ausgewählt sind aus H, F, Cl, Br, I, (CH₂)ₙ₋₁CH₃, (CH₂)ₙR₈, CH₂C₆H₅, CH₂C₆H₄R₈, O(CH₂)ₙ₋₁CH₃, O(CH₂)ₙR₈, OCH₂C₆H₅, OCH₂C₆H₄R₈ und CN;
R₄, R₅ und R₆ unabhängig ausgewählt sind aus (CH₂)ₙ₋₁CH₃, (CH₂)ₙR₈, CH₂C₆H₅ und CH₂C₆H₄R₈, wobei R₇ C₆H₅, C₆H₄R₉, SO₃R₁₀ oder COOR₁₁ ist;
R₈ SO₃R₁₀ oder COOR₁₁ ist; n und m Ganzzahlen sind, ausgewählt aus 1-18 bzw. 0-18;
R₉ Arsonsäure, Borsäure, Carbonsäure, Sulfonsäure, Amino, Hydroxyl, Sulfhydryl oder C₁-C₁₈-Alkyl ist;
R₁₀ N(R₁₂R₁₃R₁₄R₁₅) ist;
R₁₁ ein C₁-C₁₈-Alkyl ist;
R₁₂, R₁₃, R₁₄ und R₁₅ unabhängig ausgewählt sind aus H, C₁-C₁₈-Alkyl und (CH₂)ₚOH, und p eine Ganzzahl ausgewählt aus 0-18 ist; und
Z⁻ ein Halogenidanion oder OTs⁻ ist.

3. Verfahren nach Anspruch 2, wobei das erste Lösungsmittel eine gemischte Lösung von Dichlormethan und wasserfreiem Tetrahydrofuran ist; und/oder
eine Temperatur der Substitutionsreaktion in einem Bereich von 40 °C bis 50 °C liegt; und/oder
eine Temperatur der Additionsreaktion in einem Bereich von 25 °C bis 60 °C liegt; und/oder
ein Molverhältnis der Verbindung der Formel (III) zum Lawessons Reagenz 1: 1 beträgt; und/oder
ein Molverhältnis der Verbindung der Formel (II) zum nucleophilen Reagenz R₁Z 1:2 bis 5 beträgt.

4. Verfahren nach Anspruch 2, wobei die Verbindung der Formel (III) durch die folgenden Schritte hergestellt wird:
Herstellen einer Verbindung der Formel (VI) und einer Verbindung der Formel (VII) ;
Unterziehen der Verbindung der Formel (VI) und eines nucleophilen Reagenz R₄Z einer Additionsreaktion zur Herstellung einer Verbindung der Formel (V);
Unterziehen der Verbindung der Formel (VII) und eines nucleophilen Reagenz R₅Z einer Additionsreaktion zur Herstellung einer Verbindung der Formel (IV);
Mischen der Verbindung der Formel (V) und der Verbindung der Formel (IV) mit einer Quadratsäurelösung, und Umsetzen des Reaktionsgemischs in Gegenwart eines Katalysators, um die Verbindung der Formel (III) zu erhalten;

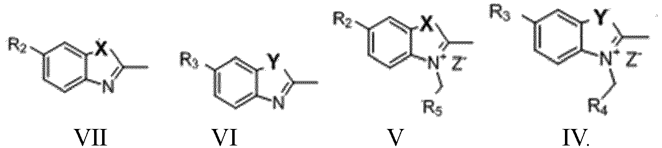

5. Verfahren nach Anspruch 4, wobei das Lösungsmittel in der Quadratsäurelösung Ethanol ist; und/oder
der Katalysator Triethylorthoformiat ist; und/oder
ein Molverhältnis der Verbindung der Formel (IV) zur Verbindung der Formel (V) zur Quadratsäure 1:0,8 bis 1,2:0,8 bis 1,2 beträgt.

6. Anwendung des Fluoreszenzfarbstoffs nach Anspruch 1, umfassend:
Anwenden des Fluoreszenzfarbstoffs in der Fluoreszenzbildgebung von lebenden Zellen und der Fluoreszenzmarkierung der intrazellulären Mikrostruktur.

## Revendications

1. Un colorant fluorescent de formule (I) : 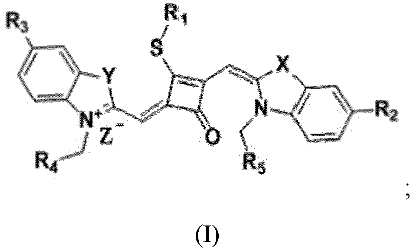
dans lequel X et Y sont choisis indépendamment parmi O, S, C(CH₃)₂ ou NR₆ ;
R₁ est (CH₂)ₙ₋₁CH₃, (CH₂)ₙR₇, (CH₂)ₘC₆H₅ ou (CH₂)ₘC₆H₄R₇ ;
R₂ et R₃ sont choisis indépendamment parmi H, F, Cl, Br, I, (CH₂)ₙ₋₁CH₃, (CH₂)ₙR₈, CH₂C₆H₅, CH₂C₆H₄R₈, O(CH₂)ₙ₋₁CH₃, O(CH₂)ₙR₈, OCH₂C₆H₅, OCH₂C₆H₄R₈ et CN ;
R₄, R₅ et R₆ sont choisis indépendamment parmi (CH₂)ₙ₋₁CH₃, (CH₂)ₙR₈, CH₂C₆H₅ et CH₂C₆H₄R₈; dans lequel R₇ est C₆H₅, C₆H₄R₉, SO₃R₁₀ ou COOR₁₁ ;
R₈ est SO₃R₁₀ ou COOR₁₁ ; n et m sont des nombres entiers choisis parmi 1-18 et 0-18, respectivement ;
R₉ est l'acide arsonique, l'acide borique, l'acide carboxylique, l'acide sulfonique, l'amino, l'hydroxyle, le sulfhydryle ou un alkyle en C₁-C₁₈ ;
R₁₀ est N(R₁₂R₁₃R₁₄R₁₅) ;
R₁₁ est un alkyle en C₁-C₁₈ ;
R₁₂, R₁₃, R₁₄ et R₁₅ sont choisis indépendamment parmi H, un alkyle en C₁-C₁₈ et (CH₂)ₚOH, et p est un nombre entier choisi parmi 0-18 ; et
Z⁻ est un anion halogénure ou OTs⁻.

2. Un procédé de préparation du colorant fluorescent selon la revendication 1, comprenant :
préparer un composé de formule (III) et un réactif de Lawesson ;
dissoudre le composé de formule (III) et le réactif de Lawesson dans un premier solvant ; et soumettre le mélange réactionnel à une réaction de substitution tout en le chauffant dans un gaz inerte pour produire un composé de formule (II) ; et
soumettre le composé de formule (II) et un réactif nucléophile R₁Z à une réaction d'addition pour produire le colorant fluorescent de formule (I) ; 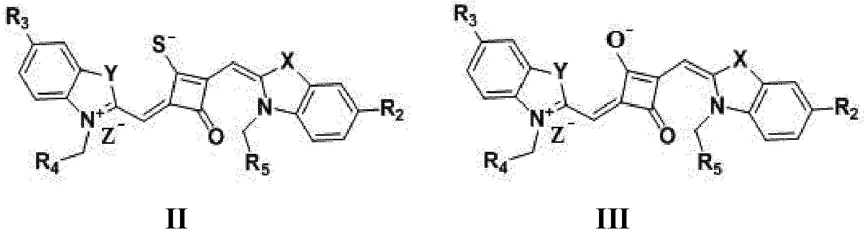
dans lequel X et Y sont choisis indépendamment parmi O, S, C(CH₃)₂ ou NR₆ ;
R₁ est (CH₂)ₙ₋₁CH₃, (CH₂)ₙR₇, (CH₂)ₘC₆H₅ ou (CH₂)ₘC₆H₄R₇ ;
R₂ et R₃ sont choisis indépendamment parmi H, F, Cl, Br, I, (CH₂)ₙ₋₁CH₃, (CH₂)ₙR₈, CH₂C₆H₅, CH₂C₆H₄R₈, O(CH₂)ₙ₋₁CH₃, O(CH₂)ₙR₈, OCH₂C₆H₅, OCH₂C₆H₄R₈ et CN ;
R₄, R₅ et R₆ sont choisis indépendamment parmi (CH₂)ₙ₋₁CH₃, (CH₂)ₙR₈, CH₂C₆H₅ et CH₂C₆H₄R₈; dans lequel R₇ est C₆H₅, C₆H₄R₉, SO₃R₁₀ ou COOR₁₁ ;
R₈ est SO₃R₁₀ ou COOR₁₁ ; n et m sont des nombres entiers choisis parmi 1-18 et 0-18, respectivement ;
R₉ est l'acide arsonique, l'acide borique, l'acide carboxylique, l'acide sulfonique, l'amino, l'hydroxyle, le sulfhydryle ou un alkyle en C₁-C₁₈ ;
R₁₀ est N(R₁₂R₁₃R₁₄R₁₅);
R₁₁ est un alkyle en C₁-C₁₈ ;
R₁₂, R₁₃, R₁₄ et R₁₅ sont choisis indépendamment parmi H, un alkyle en C₁-C₁₈ et (CH₂)ₚOH, et p est un nombre entier choisi parmi 0-18 ; et
Z⁻ est un anion halogénure ou OTs⁻.

3. Le procédé selon la revendication 2, dans lequel le premier solvent est une solution mixte de dichlorométhane et tetrahydrofurane anhydre; et/ou
une température de la réaction de substitution se situe dans une plage de 40 °C à 50 °C ; et/ou
une température de la réaction d'addition se situe dans une plage de 25 °C à 60 °C ; et/ou
un rapport molaire du composé de formule (III) au réactif de Lawesson est de 1:1 ; et/ou
un rapport molaire du composé de formule (II) au réactif nucléophile R₁Z est de 1:2 à 5.

4. Le procédé selon la revendication 2, dans lequel le composé de formule (III) est préparé par les étapes suivantes :
préparer un composé de formule (VI) et un composé de formule (VII) ;
soumettre le composé de formule (VI) et un réactif nucléophile R₄Z à une réaction d'addition pour produire un composé de formule (V) ;
soumettre le composé de formule (VII) et un réactif nucléophile R₅Z à une réaction d'addition pour produire un composé de formule (IV) ;
mélanger le composé de formule (V) et le composé de formule (IV) avec une solution d'acide quadratique, et faire réagir le mélange réactionnel en présence d'un catalyseur pour obtenir le composé de formule (III) ;

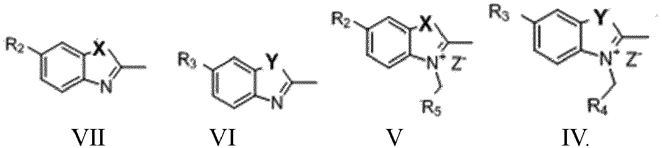

5. Le procédé selon la revendication 4, dans lequel le solvent dans la solution d'acide quadratique est l'éthanol ; et/ou
le catalyseur est l'orthoformiate de triéthyle ; et/ou
un rapport molaire du composé de formule (IV) au composé de formule (V) à la solution d'acide quadratique est de 1:0.8 à 1.2:0.8 à 1.2.

6. Une application du colorant fluorescent selon la revendication 1, comprenant :
appliquer le colorant fluorescent à l'imagerie de fluorescence des cellules vivantes et au marquage fluorescent de la microstructure intracellulaire.
